# EUROPEAN PATENT APPLICATION

(11) **EP 4 606 385 A1**
(43) Date of publication of application: **27.08.2025**
(21) Application number: 23879825.0
(22) Date of filing: 18.10.2023
(51) Int. Cl.: A61K 39/395, A61P 35/00, A61P 35/02, A61P 43/00, C07K 16/28, C07K 16/30, C12N 15/13

(54) **THERAPEUTIC AGENT FOR T CELL TUMOR**

(30) Priority: 18.10.2022 JP 2022167027
(71) Applicant: Meiji Seika Pharma Co., Ltd., Tokyo 104-8002 (JP)
(72) Inventor: ISHIDA Yoji, Tokyo 104-8002 (JP); FUKUSHIMA Takayoshi, Odawara-shi, Kanagawa 250-0852 (JP); CHIKADA Tsubasa, Tokyo 104-8002 (JP); ASANO Hikari, Tokyo 104-8002 (JP); TSUCHIYA Toshiyuki, Tokyo 104-8002 (JP)
(74) Representative: Godemeyer Blum Lenze Patentanwälte Partnerschaft mbB - werkpatent
(86) International application number: PCT/JP2023/037683
(87) International publication number: WO 2024/085182

(57) **Abstract**

An object of the present invention is to provide a novel method for treating a T cell malignancy, which can induce cytotoxicity against tumor cells and is unlikely to cause immunodeficiency. According to the present invention, there is provided a therapeutic agent for a T cell malignancy comprising a bispecific antigen-binding molecule, wherein the bispecific antigen-binding molecule comprises (1) at least one portion that specifically binds to a target tumor antigen expressed on T cell tumor cells, and (2) at least one portion that specifically binds to a normal T cell-side target antigen having subtypes, provided that the target tumor antigen expressed on the T cell tumor cells is either not present on normal T cells, or if present, the normal T cells are not substantially activated when the bispecific antigen-binding molecule binds to the same antigen as the target tumor antigen present on the normal T cells, but binding of the bispecific antigen-binding molecule to the normal T cell-side target antigen activates the normal T cells, and a sufficient proportion of a subtype of the normal T cell-side target antigen is present to provide a sufficient number of activated T cells for the treatment of the T cell tumor.

## Description

### Technical Field

### Cross-reference to related application

The present application claims conventional priority based on Japanese Patent Application No. 2022-167027, filed October 18, 2022, the entire description of which is incorporated herein by reference.

The present invention relates to a therapeutic agent for a T cell malignancy comprising a bispecific antigen-binding molecule, wherein the bispecific antigen-binding molecule comprises at least one portion that specifically binds to a target tumor antigen expressed on T cell tumor cells and at least one portion that specifically binds to a normal T cell-side target antigen having subtypes. The present invention relates to such a bispecific antigen-binding molecule and use thereof, as well as a method for treating a T cell malignancy comprising administering the bispecific antigen-binding molecule to a subject.

### Background Art

Bispecific antibodies that can induce T cell activity in target cells and selectively kill the target cells *in vivo* have been developed. The bispecific antibody that can simultaneously recognize human CD19 and CD3 antigens (blinatumomab) has been approved for marketing as a therapeutic agent for relapsed/refractory B cell acute lymphoblastic leukemia (ALL) (Goebeler, M.E. and R. Bargou, Blinatumomab: a CD19/CD3 bispecific T cell engager (BiTE) with unique anti-tumor efficacy. Leuk Lymphoma, 2016. 57(5): p.1021-32; the entire description of which is incorporated herein by reference). Blinatumomab is a bispecific T cell-inducing antibody (BiTE^{™}), which has a structure of a single-chain antibody consisting of two single-chain variable region fragments (scFv) connected by a short linker. In addition to blinatumomab, many other bispecific antibodies have been developed in recent years that target CD3 antigen and tumor cell antigens.

Lymphoid malignancies are broadly classified into B cell malignancies and T cell malignancies, and most bispecific antibodies have been developed to treat B cell malignancies. Acute myeloid leukemia cells have the same cell surface antigens as normal bone marrow progenitor cells. Therefore, bispecific antibodies against tumor cell antigens of acute myeloid leukemia attack not only leukemic cells but also normal bone marrow progenitor cells, causing neutropenia, which leads to development of febrile neutropenia, and thus invites a situation difficult to treat. Also in immunotherapies used for B cell malignancies, the target antigens are also expressed on normal B cells. Bispecific antibodies against tumor cell antigens of B cell malignancies attack not only tumor cells but also normal B cell progenitor cells, resulting in B cell immunodeficiency and inability to produce antibodies. However, B cell immunodeficiency can be prevented by regular administration of immunoglobulin preparations (Maciocia PM, et al., Targeting the T cell receptor β-chain constant region for immunotherapy of T cell malignancies. Nat Med. 2017 Dec;23(12):1416-1423, the entire description of which is incorporated herein by reference)

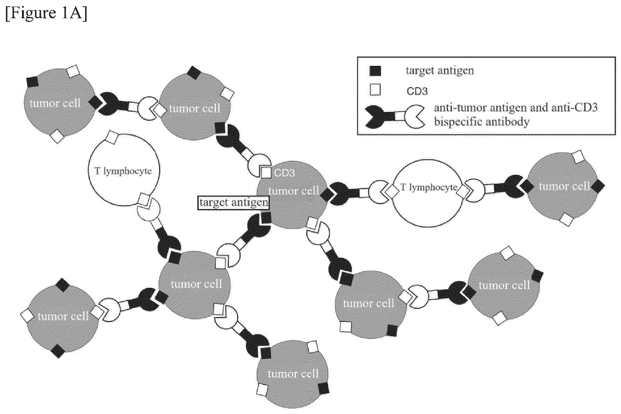

On the other hand, immunotherapies for T cell malignant tumors have been limited, because CD3 is expressed by both normal T cells and tumor T cells alike, and bispecific antibodies targeting CD3 and a tumor antigen bind simultaneously to the CD3 antigen expressed on the T cell malignant tumor and the target antigen expressed on the T cell malignant tumor to link the malignant tumor cells (Fig. 1A), which highly likely results in inadequate therapeutic effect of the bispecific antibodies. An additional problem is that there are no known target antigens for discriminating normal T cells and malignant cells. The T cell dysplasia that results from targeting pan-T cell antigens can lead to serious and unacceptable immunodeficiency (Maciocia PM, et al., supra).

As for the target on the effector cell (T cell) side, there is a bispecific antibody that utilizes TRGV9 expressed on γδ T cells (WO2021/173896A1, the entire description of which is incorporated herein by reference), aside from the CD3 antigen. However, since γδ T cells comprise less than a few percent of all T cells, they cannot be mobilized in sufficient numbers to injure tumor cells to achieve a therapeutic efficacy.

### Summary of the Invention

### Object to be achieved by the invention

There is a need for a novel treatment method for T cell malignancies, which is less likely to cause problems of immunodeficiency and can achieve high therapeutic efficacy even when used to treat T cell malignancies.

Therefore, an object of the present invention is to provide a novel method for treating a T cell malignancy, which can induce cytotoxicity against tumor cells and is unlikely to cause immunodeficiency.

### Means for achieving the object

The inventors of the present invention diligently conducted researches to achieve the above object, and as a result, found that a bispecific antigen-binding molecule that specifically binds to a target tumor antigen expressed on T cell tumor cells and to a target antigen having subtypes on the side of normal T cells mobilizes effector cells (normal T cells) and thereby induces cytotoxicity against tumor cells. The inventors of the present invention further found that by utilizing, among antigens having subtypes, an antigen of which different subtypes are expressed on T cell tumor cells and normal T cells as a target antigen on the side of normal T cells to which the bispecific antigen-binding molecule binds, tumor cells of T cell malignant tumors can be injured while sparing sufficient normal T cells. The present invention was accomplished on the basis of the above findings.

The present invention provides the following inventions.
[1] A therapeutic agent for a T cell malignancy comprising a bispecific antigen-binding molecule, wherein the bispecific antigen-binding molecule comprises
   (1) at least one portion that specifically binds to a target tumor antigen expressed on T cell tumor cells, and
   (2) at least one portion that specifically binds to a normal T cell-side target antigen having subtypes,
   provided that
   the target tumor antigen expressed on the T cell tumor cells is either not present on normal T cells, or if present, the normal T cells are not substantially activated when the bispecific antigen-binding molecule binds to the same antigen as the target tumor antigen present on the normal T cells, but
   binding of the bispecific antigen-binding molecule to the normal T cell-side target antigen activates the normal T cells, and
   a sufficient proportion of a subtype of the normal T cell-side target antigen is present to provide a sufficient number of activated T cells for the treatment of the T cell tumor.
[2] The therapeutic agent according to [1], wherein the subtype of the normal T cell-side target antigen is a subtype selected from the subtypes of the antigen that are not a subtype of the antigen expressed on the T cell tumor cells.
[3] The therapeutic agent according to [1] or [2], wherein the normal T cell-side target antigen having subtypes is a TRBC (T cell receptor beta-chain constant region),
   the subtype expressed on the T cell tumor cells is TRBC1, and
   the subtype of the normal T cell-side target antigen is TRBC2.
[4] The therapeutic agent according to [1] or [2], wherein the normal T cell-side target antigen having subtypes is a TRBC,
   the subtype expressed on the T cell tumor cells is TRBC2, and
   the subtype of the normal T cell-side target antigen is TRBC1.
[5] The therapeutic agent according to [1] or [2], wherein the normal T cell-side target antigen having subtypes is a TRBC,
   the T cell tumor cells are TRBC1-negative and TRBC2-negative, and
   the subtype of the normal T cell-side target antigen is TRBC1 or TRBC2.
[6] The therapeutic agent according to any one of [1] to [5], wherein the target tumor antigen expressed on the T cell tumor cells is any one selected from the group consisting of CCR1, CCR4, CCR7, CCR8, CCR10, CXCR4, CXCR7, TIGIT, CADM1, GPR15, CXCR5, CXCL13, SLAM, ICOS, CD134, CXCR3, anaplastic lymphoma kinase, CD30, ST2(L), CCR5, Notchl, CD38, CD1a, CCR9 (CD199), CD47, IL-7Rα (CD127), and CD40L (CD154).
[7] The therapeutic agent according to any one of [1] to [6], wherein the subtype of the normal T cell-side target antigen is TRBC1,
   the bispecific antigen-binding molecule comprises at least one portion that specifically binds to TRBC1 of the normal T cells, and the at least one portion comprises
   a VH domain comprising
   a heavy chain CDR1 comprising the amino acid sequence of SEQ ID NO: 10;
   a heavy chain CDR2 comprising the amino acid sequence of SEQ ID NO: 11; and
   a heavy chain CDR3 comprising the amino acid sequence of SEQ ID NO: 12; and
   a VL domain comprising
   a light chain CDR1 comprising the amino acid sequence of SEQ ID NO: 13;
   a light chain CDR2 comprising the amino acid sequence of SEQ ID NO: 14; and
   a light chain CDR3 comprising the amino acid sequence of SEQ ID NO: 15.
[8] The therapeutic agent according to [7], wherein the at least one portion that specifically binds to TRBC1 of the normal T cells comprises
   a heavy chain variable region VH that is at least 90% identical to the amino acid sequence of SEQ ID NO: 4, and
   a light chain variable region VL that is at least 90% identical to the amino acid sequence of SEQ ID NO: 5.
[9] The therapeutic agent according to any one of [1] to [6], wherein the subtype of the normal T cell-side target antigen is TRBC2,
   the bispecific antigen-binding molecule comprises at least one portion that specifically binds to TRBC2 of the normal T cells, and the at least one portion comprises
   a VH domain comprising
   a heavy chain CDR1 comprising the amino acid sequence of SEQ ID NO: 16;
   a heavy chain CDR2 comprising the amino acid sequence of SEQ ID NO: 17; and
   a heavy chain CDR3 comprising the amino acid sequence of SEQ ID NO: 18; and
   a VL domain comprising
   a light chain CDR1 comprising the amino acid sequence of SEQ ID NO: 19;
   a light chain CDR2 comprising the amino acid sequence of SEQ ID NO: 20; and
   a light chain CDR3 comprising the amino acid sequence of SEQ ID NO: 21.
[10] The therapeutic agent according to [9], wherein the at least one portion that specifically binds to TRBC2 of the normal T cells comprises
   a heavy chain variable region VH that is at least 90% identical to the amino acid sequence of SEQ ID NO: 6, and
   a light chain variable region VL that is at least 90% identical to the amino acid sequence of SEQ ID NO: 7.
[11] The therapeutic agent according to any one of [1] to [10], wherein the target tumor antigen expressed on the T cell tumor cells is CCR4,
   the bispecific antigen-binding molecule comprises at least one portion that specifically binds to CCR4, and the at least one portion comprises
   a VH domain comprising
   a heavy chain CDR1 comprising the amino acid sequence of SEQ ID NO: 22,
   a heavy chain CDR2 comprising the amino acid sequence of SEQ ID NO: 23, and
   a heavy chain CDR3 comprising the amino acid sequence of SEQ ID NO: 24, and
   a VL domain comprising
   a light chain CDR1 comprising the amino acid sequence of SEQ ID NO: 25,
   a light chain CDR2 comprising the amino acid sequence of SEQ ID NO: 26, and
   a light chain CDR3 comprising the amino acid sequence of SEQ ID NO: 27.
[12] The therapeutic agent according to [11], wherein the at least one portion that specifically binds to CCR4 comprises
   a heavy chain variable region VH that is at least 90% identical to the amino acid sequence of SEQ ID NO: 8, and
   a light chain variable region VL that is at least 90% identical to the amino acid sequence of SEQ ID NO: 9.
[13] The therapeutic agent according to any one of [1] to [10], wherein the target tumor antigen expressed on the T cell tumor cells is CD1a,
   the bispecific antigen-binding molecule comprises at least one portion that specifically binds to CD1a, and the at least one portion comprises
   a VH domain comprising
   a heavy chain CDR1 comprising the amino acid sequence of SEQ ID NO: 34,
   a heavy chain CDR2 comprising the amino acid sequence of SEQ ID NO: 35, and
   a heavy chain CDR3 comprising the amino acid sequence of SEQ ID NO: 36, and
   a VL domain comprising
   a light chain CDR1 comprising the amino acid sequence of SEQ ID NO: 37,
   a light chain CDR2 comprising the amino acid sequence of SEQ ID NO: 38, and
   a light chain CDR3 comprising the amino acid sequence of SEQ ID NO: 39.
[14] The therapeutic agent according to [13], wherein the at least one portion that specifically binds to CD1a comprises
   a heavy chain variable region VH that is at least 90% identical to the amino acid sequence of SEQ ID NO: 40, and
   a light chain variable region VL that is at least 90% identical to the amino acid sequence of SEQ ID NO: 41.
[15] The therapeutic agent according to any one of [1] to [10], wherein the target tumor antigen expressed on the T cell tumor cells is CCR9,
   the bispecific antigen-binding molecule comprises at least one portion that specifically binds to CCR9, and the at least one portion comprises
   a VH domain comprising
   a heavy chain CDR1 comprising the amino acid sequence of SEQ ID NO: 101,
   a heavy chain CDR2 comprising the amino acid sequence of SEQ ID NO: 102, and
   a heavy chain CDR3 comprising the amino acid sequence of SEQ ID NO: 103, and
   a VL domain comprising
   a light chain CDR1 comprising the amino acid sequence of SEQ ID NO: 104,
   a light chain CDR2 comprising the amino acid sequence of SEQ ID NO: 105, and
   a light chain CDR3 comprising the amino acid sequence of SEQ ID NO: 106.
[16] The therapeutic agent according to [15], wherein the at least one portion that specifically binds to CCR9 comprises
   a heavy chain variable region VH that is at least 90% identical to the amino acid sequence of SEQ ID NO: 131, and
   a light chain variable region VL that is at least 90% identical to the amino acid sequence of SEQ ID NO: 132.
[17] The therapeutic agent according to any one of [1] to [10], wherein the target tumor antigen expressed on the T cell tumor cells is CXCR4,
   the bispecific antigen-binding molecule comprises at least one portion that specifically binds to CXCR4, and the at least one portion comprises
   a VH domain comprising
   a heavy chain CDR1 comprising the amino acid sequence of SEQ ID NO: 107,
   a heavy chain CDR2 comprising the amino acid sequence of SEQ ID NO: 108, and
   a heavy chain CDR3 comprising the amino acid sequence of SEQ ID NO: 109, and
   a VL domain comprising
   a light chain CDR1 comprising the amino acid sequence of SEQ ID NO: 110,
   a light chain CDR2 comprising the amino acid sequence of SEQ ID NO: 111, and a light chain CDR3 comprising the amino acid sequence of SEQ ID NO: 112.
[18] The therapeutic agent according to [17], wherein the at least one portion that specifically binds to CXCR4 comprises
   a heavy chain variable region VH that is at least 90% identical to the amino acid sequence of SEQ ID NO: 133, and
   a light chain variable region VL that is at least 90% identical to the amino acid sequence of SEQ ID NO: 134.
[19] The therapeutic agent according to any one of [1] to [10], wherein the target tumor antigen expressed on the T cell tumor cells is TIGIT,
   the bispecific antigen-binding molecule comprises at least one portion that specifically binds to TIGIT, and the at least one portion comprises
   a VH domain comprising
   a heavy chain CDR1 comprising the amino acid sequence of SEQ ID NO: 89,
   a heavy chain CDR2 comprising the amino acid sequence of SEQ ID NO: 90, and
   a heavy chain CDR3 comprising the amino acid sequence of SEQ ID NO: 91, and
   a VL domain comprising
   a light chain CDR1 comprising the amino acid sequence of SEQ ID NO: 92,
   a light chain CDR2 comprising the amino acid sequence of SEQ ID NO: 93, and
   a light chain CDR3 comprising the amino acid sequence of SEQ ID NO: 94.
[20] The therapeutic agent according to [19], wherein the at least one portion that specifically binds to TIGIT comprises
   a heavy chain variable region VH that is at least 90% identical to the amino acid sequence of SEQ ID NO: 135, and
   a light chain variable region VL that is at least 90% identical to the amino acid sequence of SEQ ID NO: 136.
[21] The therapeutic agent according to any one of [1] to [10], wherein the target tumor antigen expressed on the T cell tumor cells is CCR8,
   the bispecific antigen-binding molecule comprises at least one portion that specifically binds to CCR8, and the at least one portion comprises
   a VH domain comprising
   a heavy chain CDR1 comprising the amino acid sequence of SEQ ID NO: 95,
   a heavy chain CDR2 comprising the amino acid sequence of SEQ ID NO: 96, and
   a heavy chain CDR3 comprising the amino acid sequence of SEQ ID NO: 97, and
   a VL domain comprising
   a light chain CDR1 comprising the amino acid sequence of SEQ ID NO: 98,
   a light chain CDR2 comprising the amino acid sequence of SEQ ID NO: 99, and
   a light chain CDR3 comprising the amino acid sequence of SEQ ID NO: 100.
[22] The therapeutic agent according to [21], wherein the at least one portion that specifically binds to CCR8 comprises
   a heavy chain variable region VH that is at least 90% identical to the amino acid sequence of SEQ ID NO: 137, and
   a light chain variable region VL that is at least 90% identical to the amino acid sequence of SEQ ID NO: 138.
[23] The therapeutic agent according to any one of [1] to [10], wherein the target tumor antigen expressed on the T cell tumor cells is CD30,
   the bispecific antigen-binding molecule comprises at least one portion that specifically binds to CD30, and the at least one portion comprises
   a VH domain comprising
   a heavy chain CDR1 comprising the amino acid sequence of SEQ ID NO: 113,
   a heavy chain CDR2 comprising the amino acid sequence of SEQ ID NO: 114, and
   a heavy chain CDR3 comprising the amino acid sequence of SEQ ID NO: 115, and
   a VL domain comprising
   a light chain CDR1 comprising the amino acid sequence of SEQ ID NO: 116,
   a light chain CDR2 comprising the amino acid sequence of SEQ ID NO: 117, and
   a light chain CDR3 comprising the amino acid sequence of SEQ ID NO: 118.
[24] The therapeutic agent according to [23], wherein the at least one portion that specifically binds to CD30 comprises
   a heavy chain variable region VH that is at least 90% identical to the amino acid sequence of SEQ ID NO: 139, and
   a light chain variable region VL that is at least 90% identical to the amino acid sequence of SEQ ID NO: 140.
[25] The therapeutic agent according to any one of [1] to [10], wherein the target tumor antigen expressed on the T cell tumor cells is CD40L (CD154),
   the bispecific antigen-binding molecule comprises at least one portion that specifically binds to CD40L (CD154), and the at least one portion comprises
   a VH domain comprising
   a heavy chain CDR1 comprising the amino acid sequence of SEQ ID NO: 119,
   a heavy chain CDR2 comprising the amino acid sequence of SEQ ID NO: 120, and
   a heavy chain CDR3 comprising the amino acid sequence of SEQ ID NO: 121, and
   a VL domain comprising
   a light chain CDR1 comprising the amino acid sequence of SEQ ID NO: 122,
   a light chain CDR2 comprising the amino acid sequence of SEQ ID NO: 123, and
   a light chain CDR3 comprising the amino acid sequence of SEQ ID NO: 124.
[26] The therapeutic agent according to [25], wherein the at least one portion that specifically binds to CD40L (CD154) comprises
   a heavy chain variable region VH that is at least 90% identical to the amino acid sequence of SEQ ID NO: 141, and
   a light chain variable region VL that is at least 90% identical to the amino acid sequence of SEQ ID NO: 142.
[27] The therapeutic agent according to any one of [1] to [26], wherein the T cell tumor is T cell acute lymphoblastic leukemia/lymphoblastic lymphoma or mature T cell tumor, preferably any selected from the group consisting of adult T cell leukemia (ATL), peripheral T cell lymphoma (PTCL), and T cell acute lymphoblastic leukemia (TALL).
[28] The therapeutic agent according to any one of [1] to [27], which is for use in a method for treating a T cell malignancy of a subject, and wherein the treatment method comprises determining subtype expressed on T cell tumor cells of the subject and administering the therapeutic agent to the subject, and the therapeutic agent comprises a bispecific antigen-binding molecule comprising at least one portion that specifically binds to a normal T cell-side target antigen of a subtype different from the subtype determined to be expressed on the T cell tumor cells of the subject.
[29] A therapeutic agent for a T cell malignancy comprising a bispecific antigen-binding molecule, wherein the bispecific antigen-binding molecule comprises
   at least one portion that specifically binds to a target tumor antigen expressed on T cell tumor cells; and
   at least one portion that specifically binds to either TRBC1 or TRBC2 expressed on normal T cells.
[30] The therapeutic agent according to [29], wherein when the T cell tumor cells are TRBC1-positive, the bispecific antigen-binding molecule comprises at least one portion that specifically binds to TRBC2 expressed on the normal T cells, and when the T cell tumor cells are TRBC2-positive, the bispecific antigen-binding molecule comprises at least one portion that specifically binds to TRBC1 expressed on the normal T cells.
[31] The therapeutic agent according to [29] or [30], wherein the bispecific antigen-binding molecule comprises at least one portion that specifically binds to TRBC1 expressed on the normal T cells, and the at least one portion comprises
   a VH domain comprising
   a heavy chain CDR1 comprising the amino acid sequence of SEQ ID NO: 10,
   a heavy chain CDR2 comprising the amino acid sequence of SEQ ID NO: 11, and
   a heavy chain CDR3 comprising the amino acid sequence of SEQ ID NO: 12, and
   a VL domain comprising
   a light chain CDR1 comprising the amino acid sequence of SEQ ID NO: 13,
   a light chain CDR2 comprising the amino acid sequence of SEQ ID NO: 14, and
   a light chain CDR3 comprising the amino acid sequence of SEQ ID NO: 15.
[32] The therapeutic agent according to [31], wherein the at least one portion that specifically binds to TRBC1 expressed on the normal T cells comprises
   a heavy chain variable region VH that is at least 90% identical to the amino acid sequence of SEQ ID NO: 4, and
   a light chain variable region VL that is at least 90% identical to the amino acid sequence of SEQ ID NO: 5.
[33] The therapeutic agent according to [29] or [30], wherein the bispecific antigen-binding molecule comprises at least one portion that specifically binds to TRBC2 expressed on the normal T cells, and the at least one portion comprises
   a VH domain comprising
   a heavy chain CDR1 comprising the amino acid sequence of SEQ ID NO: 16,
   a heavy chain CDR2 comprising the amino acid sequence of SEQ ID NO: 17, and
   a heavy chain CDR3 comprising the amino acid sequence of SEQ ID NO: 18, and
   a VL domain comprising
   a light chain CDR1 comprising the amino acid sequence of SEQ ID NO: 19,
   a light chain CDR2 comprising the amino acid sequence of SEQ ID NO: 20, and
   a light chain CDR3 comprising the amino acid sequence of SEQ ID NO: 21.
[34] The therapeutic agent according to [33], wherein the at least one portion that specifically binds to TRBC2 expressed on normal T cells comprises
   a heavy chain variable region VH that is at least 90% identical to the amino acid sequence of SEQ ID NO: 6, and
   a light chain variable region VL that is at least 90% identical to the amino acid sequence of SEQ ID NO: 7.
[35] The therapeutic agent according to any one of [29] to [34], wherein the at least one portion that specifically binds to TRBC1 or TRBC2 expressed on the normal T cells is an scFv or Fab fragment.
[36] The therapeutic agent according to any one of [29] to [35], wherein the target tumor antigen expressed on the T cell tumor cells is any one selected from the group consisting of CCR1, CCR4, CCR7, CCR8, CCR10, CXCR4, CXCR7, TIGIT, CADM1, GPR15, CXCR5, CXCL13, SLAM, ICOS, CD134, CXCR3, anaplastic lymphoma kinase, CD30, ST2(L), CCR5, Notchl, CD38, CD1a, CCR9 (CD199), CD47, IL-7Rα (CD127), and CD40L (CD154).
[37] The therapeutic agent according to any one of [29] to [36], wherein the bispecific antigen-binding molecule comprises at least one portion that specifically binds to CCR4, and the at least one portion comprises
   a VH domain comprising
   a heavy chain CDR1 comprising the amino acid sequence of SEQ ID NO: 22,
   a heavy chain CDR2 comprising the amino acid sequence of SEQ ID NO: 23, and
   a heavy chain CDR3 comprising the amino acid sequence of SEQ ID NO: 24, and
   a VL domain comprising
   a light chain CDR1 comprising the amino acid sequence of SEQ ID NO: 25,
   a light chain CDR2 comprising the amino acid sequence of SEQ ID NO: 26, and
   a light chain CDR3 comprising the amino acid sequence of SEQ ID NO: 27.
[38] The therapeutic agent according to [37], wherein the at least one portion that specifically binds to CCR4 comprises
   a heavy chain variable region VH that is at least 90% identical to the amino acid sequence of SEQ ID NO: 8, and
   a light chain variable region VL that is at least 90% identical to the amino acid sequence of SEQ ID NO: 9.
[39] The therapeutic agent according to any one of [29] to [36], wherein the bispecific antigen-binding molecule comprises at least one portion that specifically binds to CD1a, and the at least one portion comprises
   a VH domain comprising
   a heavy chain CDR1 comprising the amino acid sequence of SEQ ID NO: 34,
   a heavy chain CDR2 comprising the amino acid sequence of SEQ ID NO: 35, and
   a heavy chain CDR3 comprising the amino acid sequence of SEQ ID NO: 36, and
   a VL domain comprising
   a light chain CDR1 comprising the amino acid sequence of SEQ ID NO: 37,
   a light chain CDR2 comprising the amino acid sequence of SEQ ID NO: 38, and
   a light chain CDR3 comprising the amino acid sequence of SEQ ID NO: 39.
[40] The therapeutic agent according to [39], wherein the at least one portion that specifically binds to CD1a comprises
   a heavy chain variable region VH that is at least 90% identical to the amino acid sequence of SEQ ID NO: 40, and
   a light chain variable region VL that is at least 90% identical to the amino acid sequence of SEQ ID NO: 41.
[41] The therapeutic agent according to any one of [29] to [36], wherein the target tumor antigen expressed on the T cell tumor cells is CCR9,
   the bispecific antigen-binding molecule comprises at least one portion that specifically binds to CCR9, and the at least one portion comprises
   a VH domain comprising
   a heavy chain CDR1 comprising the amino acid sequence of SEQ ID NO: 101,
   a heavy chain CDR2 comprising the amino acid sequence of SEQ ID NO: 102, and
   a heavy chain CDR3 comprising the amino acid sequence of SEQ ID NO: 103, and
   a VL domain comprising
   a light chain CDR1 comprising the amino acid sequence of SEQ ID NO: 104,
   a light chain CDR2 comprising the amino acid sequence of SEQ ID NO: 105, and
   a light chain CDR3 comprising the amino acid sequence of SEQ ID NO: 106.
[42] The therapeutic agent according to [41], wherein the at least one portion that specifically binds to CCR9 comprises
   a heavy chain variable region VH that is at least 90% identical to the amino acid sequence of SEQ ID NO: 131, and
   a light chain variable region VL that is at least 90% identical to the amino acid sequence of SEQ ID NO: 132.
[43] The therapeutic agent according to any one of [29] to [36], wherein the target tumor antigen expressed on the T cell tumor cells is CXCR4,
   the bispecific antigen-binding molecule comprises at least one portion that specifically binds to CXCR4, and the at least one portion comprises
   a VH domain comprising
   a heavy chain CDR1 comprising the amino acid sequence of SEQ ID NO: 107,
   a heavy chain CDR2 comprising the amino acid sequence of SEQ ID NO: 108, and
   a heavy chain CDR3 comprising the amino acid sequence of SEQ ID NO: 109, and
   a VL domain comprising
   a light chain CDR1 comprising the amino acid sequence of SEQ ID NO: 110,
   a light chain CDR2 comprising the amino acid sequence of SEQ ID NO: 111, and
   a light chain CDR3 comprising the amino acid sequence of SEQ ID NO: 112.
[44] The therapeutic agent according to [43], wherein the at least one portion that specifically binds to CXCR4 comprises
   a heavy chain variable region VH that is at least 90% identical to the amino acid sequence of SEQ ID NO: 133, and
   a light chain variable region VL that is at least 90% identical to the amino acid sequence of SEQ ID NO: 134.
[45] The therapeutic agent according to any one of [29] to [36], wherein the target tumor antigen expressed on the T cell tumor cells is TIGIT,
   the bispecific antigen-binding molecule comprises at least one portion that specifically binds to TIGIT, and the at least one portion comprises
   a VH domain comprising
   a heavy chain CDR1 comprising the amino acid sequence of SEQ ID NO: 89,
   a heavy chain CDR2 comprising the amino acid sequence of SEQ ID NO: 90, and
   a heavy chain CDR3 comprising the amino acid sequence of SEQ ID NO: 91, and
   a VL domain comprising
   a light chain CDR1 comprising the amino acid sequence of SEQ ID NO: 92,
   a light chain CDR2 comprising the amino acid sequence of SEQ ID NO: 93, and
   a light chain CDR3 comprising the amino acid sequence of SEQ ID NO: 94.
[46] The therapeutic agent according to [45], wherein the at least one portion that specifically binds to TIGIT comprises
   a heavy chain variable region VH that is at least 90% identical to the amino acid sequence of SEQ ID NO: 135, and
   a light chain variable region VL that is at least 90% identical to the amino acid sequence of SEQ ID NO: 136.
[47] The therapeutic agent according to any one of [29] to [36], wherein
   the target tumor antigen expressed on the T cell tumor cells is CCR8,
   the bispecific antigen-binding molecule comprises at least one portion that specifically binds to CCR8, and the at least one portion comprises
   a VH domain comprising
   a heavy chain CDR1 comprising the amino acid sequence of SEQ ID NO: 95,
   a heavy chain CDR2 comprising the amino acid sequence of SEQ ID NO: 96, and
   a heavy chain CDR3 comprising the amino acid sequence of SEQ ID NO: 97, and
   a VL domain comprising
   a light chain CDR1 comprising the amino acid sequence of SEQ ID NO: 98,
   a light chain CDR2 comprising the amino acid sequence of SEQ ID NO: 99, and
   a light chain CDR3 comprising the amino acid sequence of SEQ ID NO: 100.
[48] The therapeutic agent according to [47], wherein the at least one portion that specifically binds to CCR8 comprises
   a heavy chain variable region VH that is at least 90% identical to the amino acid sequence of SEQ ID NO: 137, and
   a light chain variable region VL that is at least 90% identical to the amino acid sequence of SEQ ID NO: 138.
[49] The therapeutic agent according to any one of [29] to [36], wherein the target tumor antigen expressed on the T cell tumor cells is CD30,
   the bispecific antigen-binding molecule comprises at least one portion that specifically binds to CD30, and the at least one portion comprises
   a VH domain comprising
   a heavy chain CDR1 comprising the amino acid sequence of SEQ ID NO: 113,
   a heavy chain CDR2 comprising the amino acid sequence of SEQ ID NO: 114, and
   a heavy chain CDR3 comprising the amino acid sequence of SEQ ID NO: 115, and
   a VL domain comprising
   a light chain CDR1 comprising the amino acid sequence of SEQ ID NO: 116,
   a light chain CDR2 comprising the amino acid sequence of SEQ ID NO: 117, and
   a light chain CDR3 comprising the amino acid sequence of SEQ ID NO: 118.
[50] The therapeutic agent according to [49], wherein the at least one portion that specifically binds to CD30 comprises
   a heavy chain variable region VH that is at least 90% identical to the amino acid sequence of SEQ ID NO: 139, and
   a light chain variable region VL that is at least 90% identical to the amino acid sequence of SEQ ID NO: 140.
[51] The therapeutic agent according to any one of [29] to [36], wherein the target tumor antigen expressed on the T cell tumor cells is CD40L (CD154),
   the bispecific antigen-binding molecule comprises at least one portion that specifically binds to CD40L (CD154), and the at least one portion comprises
   a VH domain comprising
   a heavy chain CDR1 comprising the amino acid sequence of SEQ ID NO: 119,
   a heavy chain CDR2 comprising the amino acid sequence of SEQ ID NO: 120, and
   a heavy chain CDR3 comprising the amino acid sequence of SEQ ID NO: 121, and
   a VL domain comprising
   a light chain CDR1 comprising the amino acid sequence of SEQ ID NO: 122,
   a light chain CDR2 comprising the amino acid sequence of SEQ ID NO: 123, and
   a light chain CDR3 comprising the amino acid sequence of SEQ ID NO: 124.
[52] The therapeutic agent according to [51], wherein the at least one portion that specifically binds to CD40L (CD154) comprises
   a heavy chain variable region VH that is at least 90% identical to the amino acid sequence of SEQ ID NO: 141, and
   a light chain variable region VL that is at least 90% identical to the amino acid sequence of SEQ ID NO: 142.
[53] The therapeutic agent according to any one of [29] to [52], wherein the at least one portion that specifically binds to the target tumor antigen expressed on the T cell tumor cells comprises an scFv or Fab fragment.
[54] The therapeutic agent according to any one of [29] to [53], wherein the T cell tumor is T cell acute lymphoblastic leukemia/lymphoblastic lymphoma or mature T cell tumor, preferably any selected from the group comprising adult T cell leukemia (ATL), peripheral T cell lymphoma (PTCL), and T cell acute lymphoblastic leukemia (TALL).
[55] The therapeutic agent according to any one of [29] to [54], wherein the bispecific antigen-binding molecule is administered in combination with a chemotherapeutic agent, radiation and/or another agent used in a cancer immunotherapy.
[56] A bispecific antigen-binding molecule comprising
   (1) at least one portion that specifically binds to a target tumor antigen expressed on T cell tumor cells, and
   (2) at least one portion that specifically binds to a normal T cell-side target antigen having subtypes, wherein
   the at least one portion that specifically binds to a normal T cell-side target antigen having subtypes is at least one portion that specifically binds to TRBC1 or TRBC2 (T cell receptor beta constant region 1 or 2) expressed on normal T cells.
[57] The bispecific antigen-binding molecule according to [56], wherein the at least one portion that specifically binds to TRBC1 expressed on the normal T cells comprises
   a VH domain comprising
   a heavy chain CDR1 comprising the amino acid sequence of SEQ ID NO: 10,
   a heavy chain CDR2 comprising the amino acid sequence of SEQ ID NO: 11, and
   a heavy chain CDR3 comprising the amino acid sequence of SEQ ID NO: 12, and
   a VL domain comprising
   a light chain CDR1 comprising the amino acid sequence of SEQ ID NO: 13,
   a light chain CDR2 comprising the amino acid sequence of SEQ ID NO: 14, and
   a light chain CDR3 comprising the amino acid sequence of SEQ ID NO: 15.
[58] The bispecific antigen-binding molecule according to [57], wherein the at least one portion that specifically binds to TRBC1 expressed on the normal T cells comprises a heavy chain variable region VH that is at least 90% identical to the amino acid sequence of SEQ ID NO: 4, and
   a light chain variable region VL that is at least 90% identical to the amino acid sequence of SEQ ID NO: 5.
[59] The bispecific antigen-binding molecule according to [56], wherein the at least one portion that specifically binds to TRBC2 expressed on the normal T cells comprises a VH domain comprising
   a heavy chain CDR1 comprising the amino acid sequence of SEQ ID NO: 16,
   a heavy chain CDR2 comprising the amino acid sequence of SEQ ID NO: 17, and
   a heavy chain CDR3 comprising the amino acid sequence of SEQ ID NO: 18, and
   a VL domain comprising
   a light chain CDR1 comprising the amino acid sequence of SEQ ID NO: 19,
   a light chain CDR2 comprising the amino acid sequence of SEQ ID NO: 20, and
   a light chain CDR3 comprising the amino acid sequence of SEQ ID NO: 21.
[60] The bispecific antigen-binding molecule according to [59], wherein the at least one portion that specifically binds to TRBC2 expressed on the normal T cells comprises
   a heavy chain variable region VH that is at least 90% identical to the amino acid sequence of SEQ ID NO: 6, and
   a light chain variable region VL that is at least 90% identical to the amino acid sequence of SEQ ID NO: 7.
[61] The bispecific antigen-binding molecule according to any one of [56] to [60], wherein the target tumor antigen expressed on the T cell tumor cells is any one selected from the group consisting of CCR1, CCR4, CCR7, CCR8, CCR10, CXCR4, CXCR7, TIGIT, CADM1, GPR15, CXCR5, CXCL13, SLAM, ICOS, CD134, CXCR3, anaplastic lymphoma kinase, CD30, ST2(L), CCR5, Notchl, CD38, CD1a, CCR9 (CD199), CD47, IL-7Rα (CD127), and CD40L (CD154).
[62] The bispecific antigen-binding molecule according to [61], wherein the at least one portion that specifically binds to CCR4 comprises
   a VH domain comprising
   a heavy chain CDR1 comprising the amino acid sequence of SEQ ID NO: 22,
   a heavy chain CDR2 comprising the amino acid sequence of SEQ ID NO: 23, and
   a heavy chain CDR3 comprising the amino acid sequence of SEQ ID NO: 24, and
   a VL domain comprising
   a light chain CDR1 comprising the amino acid sequence of SEQ ID NO: 25,
   a light chain CDR2 comprising the amino acid sequence of SEQ ID NO: 26, and
   a light chain CDR3 comprising the amino acid sequence of SEQ ID NO: 27.
[63] The bispecific antigen-binding molecule according to [62], wherein the at least one portion that specifically binds to CCR4 comprises
   a heavy chain variable region VH that is at least 90% identical to the amino acid sequence of SEQ ID NO: 8, and
   a light chain variable region VL that is at least 90% identical to the amino acid sequence of SEQ ID NO: 9.
[64] The bispecific antigen-binding molecule according to [61], wherein the at least one portion that specifically binds to CD1a comprises
   a VH domain comprising
   a heavy chain CDR1 comprising the amino acid sequence of SEQ ID NO: 34,
   a heavy chain CDR2 comprising the amino acid sequence of SEQ ID NO: 35, and
   a heavy chain CDR3 comprising the amino acid sequence of SEQ ID NO: 36, and
   a VL domain comprising
   a light chain CDR1 comprising the amino acid sequence of SEQ ID NO: 37,
   a light chain CDR2 comprising the amino acid sequence of SEQ ID NO: 38, and
   a light chain CDR3 comprising the amino acid sequence of SEQ ID NO: 39.
[65] The bispecific antigen-binding molecule according to [64], wherein the at least one portion that specifically binds to CD1a comprises
   a heavy chain variable region VH that is at least 90% identical to the amino acid sequence of SEQ ID NO: 40, and
   a light chain variable region VL that is at least 90% identical to the amino acid sequence of SEQ ID NO: 41.
[66] The bispecific antigen-binding molecule according to [61], wherein the target tumor antigen expressed on the T cell tumor cells is CCR9,
   the bispecific antigen-binding molecule comprises at least one portion that specifically binds to CCR9, and the at least one portion comprises
   a VH domain comprising
   a heavy chain CDR1 comprising the amino acid sequence of SEQ ID NO: 101,
   a heavy chain CDR2 comprising the amino acid sequence of SEQ ID NO: 102, and
   a heavy chain CDR3 comprising the amino acid sequence of SEQ ID NO: 103, and
   a VL domain comprising
   a light chain CDR1 comprising the amino acid sequence of SEQ ID NO: 104,
   a light chain CDR2 comprising the amino acid sequence of SEQ ID NO: 105, and
   a light chain CDR3 comprising the amino acid sequence of SEQ ID NO: 106.
[67] The bispecific antigen-binding molecule according to [66], wherein the at least one portion that specifically binds to CCR9 comprises
   a heavy chain variable region VH that is at least 90% identical to the amino acid sequence of SEQ ID NO: 131, and
   a light chain variable region VL that is at least 90% identical to the amino acid sequence of SEQ ID NO: 132.
[68] The bispecific antigen-binding molecule according to [61], wherein the target tumor antigen expressed on the T cell tumor cells is CXCR4,
   the bispecific antigen-binding molecule comprises at least one portion that specifically binds to CXCR4, and the at least one portion comprises
   a VH domain comprising
   a heavy chain CDR1 comprising the amino acid sequence of SEQ ID NO: 107,
   a heavy chain CDR2 comprising the amino acid sequence of SEQ ID NO: 108, and
   a heavy chain CDR3 comprising the amino acid sequence of SEQ ID NO: 109, and
   a VL domain comprising
   a light chain CDR1 comprising the amino acid sequence of SEQ ID NO: 110,
   a light chain CDR2 comprising the amino acid sequence of SEQ ID NO: 111, and
   a light chain CDR3 comprising the amino acid sequence of SEQ ID NO: 112.
[69] The bispecific antigen-binding molecule according to [68], wherein the at least one portion that specifically binds to CXCR4 comprises
   a heavy chain variable region VH that is at least 90% identical to the amino acid sequence of SEQ ID NO: 133, and
   a light chain variable region VL that is at least 90% identical to the amino acid sequence of SEQ ID NO: 134.
[70] The bispecific antigen-binding molecule according to [61], wherein the target tumor antigen expressed on the T cell tumor cells is TIGIT,
   the bispecific antigen-binding molecule comprises at least one portion that specifically binds to TIGIT, and the at least one portion comprises
   a VH domain comprising
   a heavy chain CDR1 comprising the amino acid sequence of SEQ ID NO: 89,
   a heavy chain CDR2 comprising the amino acid sequence of SEQ ID NO: 90, and
   a heavy chain CDR3 comprising the amino acid sequence of SEQ ID NO: 91, and
   a VL domain comprising
   a light chain CDR1 comprising the amino acid sequence of SEQ ID NO: 92,
   a light chain CDR2 comprising the amino acid sequence of SEQ ID NO: 93, and
   a light chain CDR3 comprising the amino acid sequence of SEQ ID NO: 94.
[71] The bispecific antigen-binding molecule according to [70], wherein the at least one portion that specifically binds to TIGIT comprises
   a heavy chain variable region VH that is at least 90% identical to the amino acid sequence of SEQ ID NO: 135, and
   a light chain variable region VL that is at least 90% identical to the amino acid sequence of SEQ ID NO: 136.
[72] The bispecific antigen-binding molecule according to [61], wherein the target tumor antigen expressed on the T cell tumor cells is CCR8,
   the bispecific antigen-binding molecule comprises at least one portion that specifically binds to CCR8, and the at least one portion comprises
   a VH domain comprising
   a heavy chain CDR1 comprising the amino acid sequence of SEQ ID NO: 95,
   a heavy chain CDR2 comprising the amino acid sequence of SEQ ID NO: 96, and
   a heavy chain CDR3 comprising the amino acid sequence of SEQ ID NO: 97, and
   a VL domain comprising
   a light chain CDR1 comprising the amino acid sequence of SEQ ID NO: 98,
   a light chain CDR2 comprising the amino acid sequence of SEQ ID NO: 99, and
   a light chain CDR3 comprising the amino acid sequence of SEQ ID NO: 100.
[73] The bispecific antigen-binding molecule according to [72], wherein the at least one portion that specifically binds to CCR8 comprises
   a heavy chain variable region VH that is at least 90% identical to the amino acid sequence of SEQ ID NO: 137, and
   a light chain variable region VL that is at least 90% identical to the amino acid sequence of SEQ ID NO: 138.
[74] The bispecific antigen-binding molecule according to [61], wherein the target tumor antigen expressed on the T cell tumor cells is CD30,
   the bispecific antigen-binding molecule comprises at least one portion that specifically binds to CD30, and the at least one portion comprises
   a VH domain comprising
   a heavy chain CDR1 comprising the amino acid sequence of SEQ ID NO: 113,
   a heavy chain CDR2 comprising the amino acid sequence of SEQ ID NO: 114, and
   a heavy chain CDR3 comprising the amino acid sequence of SEQ ID NO: 115, and
   a VL domain comprising
   a light chain CDR1 comprising the amino acid sequence of SEQ ID NO: 116,
   a light chain CDR2 comprising the amino acid sequence of SEQ ID NO: 117, and
   a light chain CDR3 comprising the amino acid sequence of SEQ ID NO: 118.
[75] The bispecific antigen-binding molecule according to [74], wherein the at least one portion that specifically binds to CD30 comprises
   a heavy chain variable region VH that is at least 90% identical to the amino acid sequence of SEQ ID NO: 139, and
   a light chain variable region VL that is at least 90% identical to the amino acid sequence of SEQ ID NO: 140.
[76] The bispecific antigen-binding molecule according to [61], wherein the target tumor antigen expressed on the T cell tumor cells is CD40L (CD154),
   the bispecific antigen-binding molecule comprises at least one portion that specifically binds to CD40L (CD154), and the at least one portion comprises
   a VH domain comprising
   a heavy chain CDR1 comprising the amino acid sequence of SEQ ID NO: 119,
   a heavy chain CDR2 comprising the amino acid sequence of SEQ ID NO: 120, and
   a heavy chain CDR3 comprising the amino acid sequence of SEQ ID NO: 121, and
   a VL domain comprising
   a light chain CDR1 comprising the amino acid sequence of SEQ ID NO: 122,
   a light chain CDR2 comprising the amino acid sequence of SEQ ID NO: 123, and
   a light chain CDR3 comprising the amino acid sequence of SEQ ID NO: 124.
[77] The bispecific antigen-binding molecule according to [76], wherein the at least one portion that specifically binds to CD40L (CD154) comprises
   a heavy chain variable region VH that is at least 90% identical to the amino acid sequence of SEQ ID NO: 141, and
   a light chain variable region VL that is at least 90% identical to the amino acid sequence of SEQ ID NO: 142.
[78] The bispecific antigen-binding molecule according to any one of [56] to [77], which is for use in a method for treating a T cell malignancy.
[79] A method for treating a T cell malignancy, which comprises administering the bispecific antigen-binding molecule according to any one of [56] to [77] to a subject having a T cell malignancy.
[80] A bispecific antigen-binding molecule for use in a method for treating a T cell malignancy, which comprises
   (1) at least one portion that specifically binds to a target tumor antigen expressed on T cell tumor cells, and
   (2) at least one portion that specifically binds to a normal T cell-side target antigen having subtypes,

   provided that the target tumor antigen expressed on the T cell tumor cells is either not present on normal T cells, or if present, the normal T cells are not substantially activated when the bispecific antigen-binding molecule binds to the same antigen as the target tumor antigen present on the normal T cells,
   binding of the bispecific antigen-binding molecule to the normal T cell-side target antigen activates the normal T cells, and
   a sufficient proportion of a subtype of the normal T cell-side target antigen is present to provide a sufficient number of activated T cells for the treatment of the T cell tumor.
[81] A method for treating a T cell malignancy in a subject, comprising administering a bispecific antigen-binding molecule to a subject having a T cell malignancy, wherein the bispecific antigen-binding molecule comprises
   (1) at least one portion that specifically binds to a target tumor antigen expressed on T cell tumor cells, and
   (2) at least one portion that specifically binds to a normal T cell-side target antigen having subtypes,

   provided that the target tumor antigen expressed on the T cell tumor cells is either not present on normal T cells, or if present, the normal T cells are not substantially activated when the bispecific antigen-binding molecule binds to the same antigen as the target tumor antigen present on the normal T cells,
   binding of the bispecific antigen-binding molecule to the normal T cell-side target antigen activates the normal T cells, and
   a sufficient proportion of a subtype of the normal T cell-side target antigen is present to provide a sufficient number of activated T cells for the treatment of the T cell tumor.
[82] The treatment method according to [81], which further comprises determining subtype expressed on the T cell tumor cells of the subject, and wherein the bispecific antigen-binding molecule comprises at least one portion that specifically binds to a normal T cell-side target antigen of a subtype different from the subtype determined to be expressed on the T cell tumor cells of the subject.
[83] Use of a bispecific antigen-binding molecule in manufacture of a pharmaceutical for a T cell malignancy, wherein the bispecific antigen-binding molecule comprises
   (1) at least one portion that specifically binds to a target tumor antigen expressed on T cell tumor cells, and
   (2) at least one portion that specifically binds to a normal T cell-side target antigen having subtypes,

   provided that the target tumor antigen expressed on the T cell tumor cells is either not present on normal T cells, or if present, the normal T cells are not substantially activated when the bispecific antigen-binding molecule binds to the same antigen as the target tumor antigen present on the normal T cells,
   binding of the bispecific antigen-binding molecule to the normal T cell-side target antigen activates the normal T cells, and
   a sufficient proportion of a subtype of the normal T cell-side target antigen is present to provide a sufficient number of activated T cells for the treatment of the T cell tumor.

### Brief Description of the Drawings

[Fig. 1A] Fig. 1A shows a state that bispecific antibodies simultaneously bind to CD3 expressed on tumor cells and target antigens expressed on tumor cells, and thereby cross-link the tumor cells to one another.

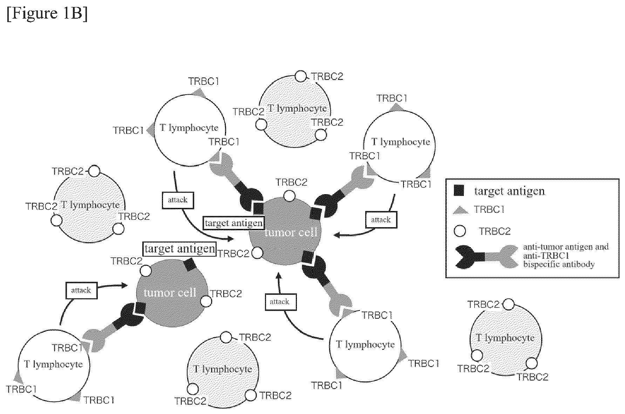

[Fig. 1b] Fig. 1b is a diagram showing the concept of the present invention. It shows a state that the bispecific antigen-binding molecules of the present invention recognize target antigens expressed on (T cell) tumor cells and "normal T cell-side target antigens having subtypes" (in the diagram, the subtype expressed on T lymphocytes is TRBC1), and thereby cross-link the tumor cells and T lymphocytes. In the diagram, the bispecific antigen-binding molecules simultaneously bind to the TRBC1 antigens on the T lymphocytes and the target antigens expressed on the tumor cells. The TRBC2 subtype is expressed on the tumor cells as well as on the T lymphocytes. In the diagram, the bispecific antigen-binding molecules do not recognize T lymphocytes expressing the TRBC2 subtype.

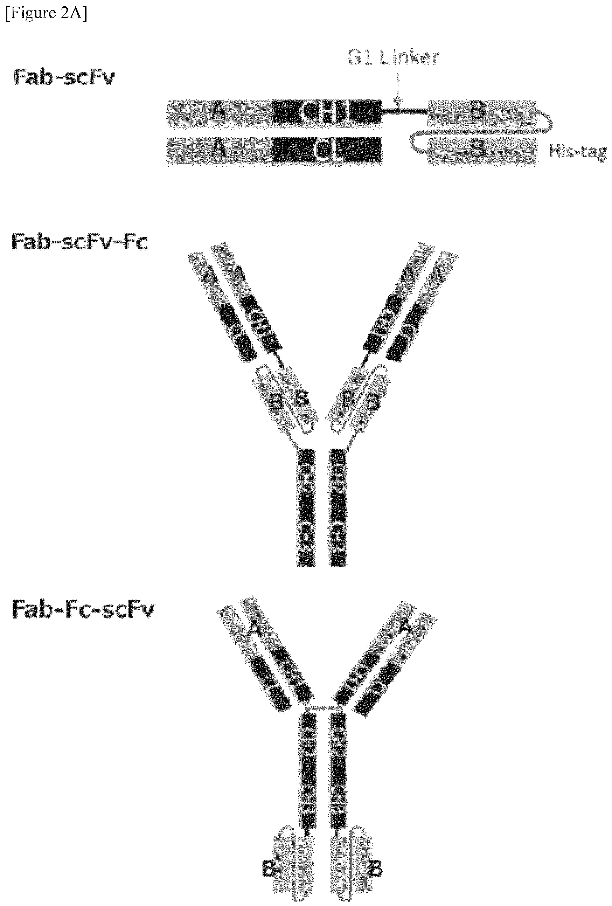

[Fig. 2A] Fig. 2A shows the structures of the bispecific antibodies prepared in the examples. The Fab-scFv type has a structure having scFv that binds to one antigen and a Fab portion that binds to the other antigen, which are linked with a linker. The Fab-scFv-Fc type has a structure having two of the structures having scFv that binds to one antigen and a Fab portion that binds to the other antigen linked with a linker, and further having Fc. The Fab-Fc-scFv type has a structure having IgG that binds to one antigen and scFv that binds to the other antigen, which is linked to the C-terminus of IgG. A is the portion that binds to one antigen, and B is the portion that binds to the other antigen.

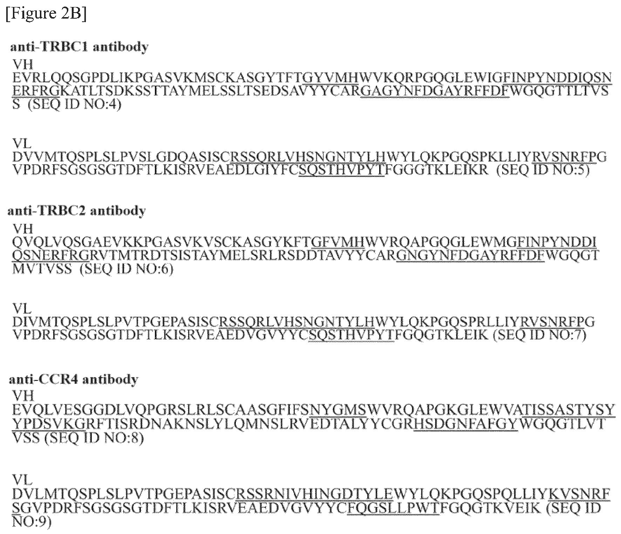

[Fig. 2B] Fig. 2B shows the amino acid sequences of the heavy and light chains of the anti-TRBC1 antibody, anti-TRBC2 antibody, and anti-CCR4 antibody used to create the bispecific antibodies. The underlines indicate the respective CDRs. The heavy and light chain amino acid sequences of the anti-CCR4 antibody are the amino acid sequences described in Japanese Patent No. 4052515 (Human-type CDR-transplanted antibody and antibody fragment thereof) as SEQ ID NOS: 9 and 14. The heavy and light chain amino acid sequences of the anti-TRBC1 antibody are the amino acid sequences described in US10730942 (Protein-based T cell receptor knockdown) as SEQ ID NOS: 15 and 16. The heavy and light chain amino acid sequences of the anti-TRBC2 antibody are the sequences of the VH domain derived from the VH domain of hJOVI-1 described in WO2020/089644 (Binding Domain, SEQ ID NO: 1 in WO2020/089644) by three amino acid substitutions T28K, Y32F and A100N (Table 1 in WO2020/089644 (page 23)) and the VL domain of hJOVI-1 (SEQ ID NO: 2 in WO2020/089644). The underlined CDRs were defined according to the Kabat numbering scheme.
[Figs. 3A to 3K] Figs. 3A to 3K show the amino acid sequences constituting the heavy and light chains of the bispecific antibodies prepared in Example 1.
[Fig. 4A] Fig. 4A shows the increase of TRBC1-positive cell ratio after the addition of anti-TRBC1 antibody (N=3).
[Fig. 4B] Fig. 4B shows the results obtained by gating live cells by Fixable Viability Dye eFluor^{™} 780 staining, and plotting the cells based on FSC and SSC to gate major cell populations, followed by plotting for CD3 and TRBC1, and calculating the ratio of TRBC1-positive cells among CD3-positive cells (T cells).
[Fig. 5A] Fig. 5A shows the increase of cytotoxic T cells induced by the addition of anti-TRBC1 antibody.

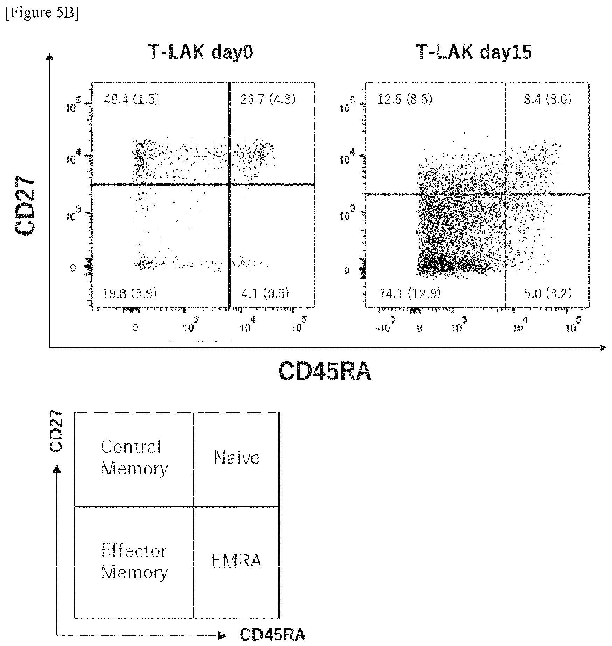

[Fig. 5B] Fig. 5B shows the results obtained by gating live cells by Fixable Viability Dye eFluor^{™} 780 staining, plotting the cells based on FSC and SSC to gate major cell populations, and then gating the cell populations for TRBC1-positive cells, followed by plotting of the TRBC1-positive cells for CD27 and CD45RA, and calculating the ratios of the respective fractions among TRBC1-positive cells. *Effector memory (EM): cells circulating primarily in secondary lymphoid tissues (lymph nodes) and peripheral tissues, and rapidly produce cytokines and immune response upon re-exposure to the same antigen. Central memory (CM): cells residing in secondary lymphoid tissues (lymph nodes) and producing antigenic responses. Naive: T cells that have never been exposed to the antigen. EM: T cells that re-express CD45RA. EMRA: Effector memory cells ultimately differentiated. N=3.
[Fig. 6A] Fig. 6A shows the ratios (%) of cells positive for the respective activation markers in the TRBC1-positive cells (N=3, but N=2 only for the day 0 data of *perforin)
[Fig. 6B] Fig. 6B shows the results obtained by gating the TRBC1-positive cells and then plotting them for CD25 and CD69, and calculating ratios of the cells positive for the respective markers in the TRBC1-positive cells.
[Fig. 6C] Fig. 6C shows the results obtained by plotting the cells for TRBC1 and granzyme B, and calculating the ratio of granzyme B-positive cells in TRBC1-positive cells.
[Fig. 6D] Fig. 6D shows the results obtained by plotting the cells for TRBC1 and perforin, and calculating the ratio of perforin-positive cells in TRBC1-positive cells. The data are shown as mean (SD).
[Fig. 7A] Fig. 7A shows the change in the ratio of PD-1-positive cells induced by the addition of anti-TRBC1 antibody (N=3).
[Fig. 7B] Fig. 7B shows the results obtained by gating live cells by Fixable Viability Dye eFluor^{™} 780 staining, and plotting the cells based on FSC and SSC to gate major cell populations, followed by plotting for TRBC1 and PD-1, and calculating the ratio of PD-1-positive cells in the TRBC1-positive cells.
[Fig. 8] Fig. 8 shows the results of the quantification of cytokines released by T-LAK cells stimulated with anti-TRBC1 antibody.
[Fig. 9] Fig. 9 shows the increase in the number of cells, including TRBC2-positive T cells, after the addition of anti-TRBC2 antibody.

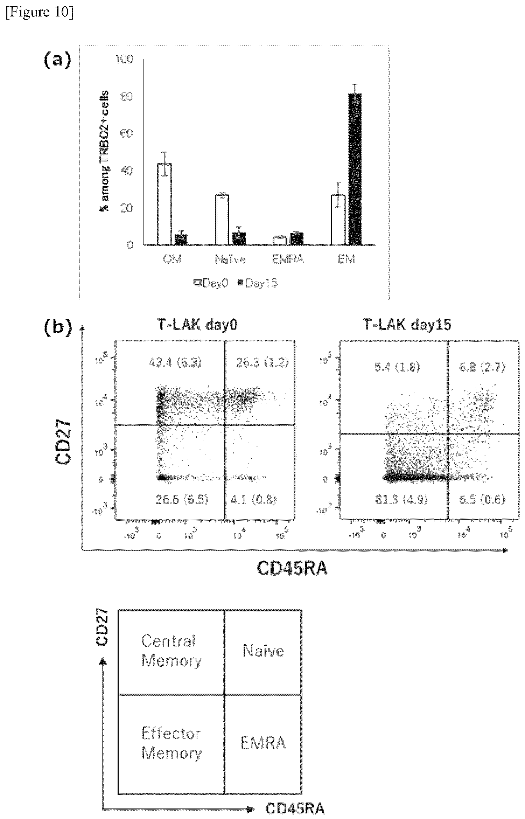

[Fig. 10] (a) A graph showing the increase in cytotoxic T cells induced by the addition of anti-TRBC2 antibody. (b) Diagrams showing the results obtained by gating live cells by Fixable Viability Dye eFluor^{™} 780 staining, plotting the cells based on FSC and SSC to gate major cell populations, and then gating the cell populations for CD3-positive and TRBC1-positive cells (gating the cells for TRBC2-positive cells), followed by plotting for CD27 and CD45RA, and calculating the ratios of the respective fractions among TRBC1-positive cells. EM, effector memory; CM, central memory, and EMRA, EM T cells that re-express CD45RA.

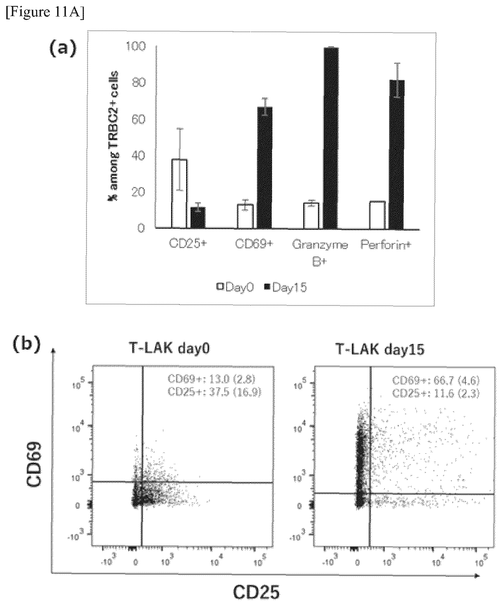

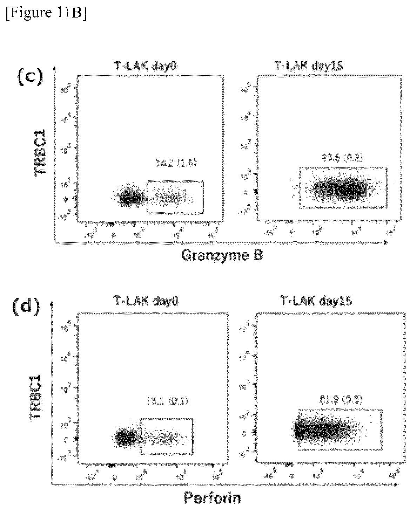

[Figs. 11A and 11B] (a) A graph showing the ratios (%) of cells positive for the respective activation markers in the TRBC2-positive cells (N=3). (b) A diagram showing the results obtained by gating TRBC2-positive cells, and plotting the cells for CD25 and CD69, and used for calculating the ratios of cells positive for the respective markers in the TRBC2-positive cells. (c) A diagram showing the results obtained by plotting the cells for TRBC1 and granzyme B, and calculating the ratio of granzyme B-positive cells in TRBC2-positive cells. (d) A diagram showing the results obtained by plotting the cells for TRBC1 and perforin, and calculating the ratio of perforin-positive cells in TRBC2-positive cells. The data are shown as mean (SD).
[Fig. 12] (a) A graph showing the change in the ratio of PD-1-positive cells induced by the addition of anti-TRBC2 antibody (N=3). (b) A diagram showing the results obtained by gating live cells by Fixable Viability Dye eFluor^{™} 780 staining, plotting the cells based on FSC and SSC to gate major cell populations, and then gating the cell populations for TRBC1-positive cells, followed by plotting for TRBC1 and PD-1, and calculating the ratio of PD-1-positive cells in TRBC2-positive cells.
[Fig. 13] Fig. 13 shows the results of the quantification of cytokines released by T-LAK cells stimulated with anti-TRBC2 antibody. The vertical axis of each graph indicates the concentration.
[Figs. 14A to 14C] Figs. 14A to 14C show the cytotoxicities of the bispecific antibodies CCR0001 and CCR0004.
[Fig. 15] Fig. 15 shows the cytotoxicities of the bispecific antibodies CD1a1001 and CD1a1004.
[Fig. 16] Fig. 16 shows the cytotoxicity of the anti-CCR4/anti-TRBC2 bispecific antibody against the ATL-derived cell strain MT-2.
[Fig. 17] Fig. 17 shows the cytotoxicity of the anti-CD1a/anti-TRBC2 bispecific antibody against the TALL-derived cell strain JM (expressing CD1a and TRBC1).
[Fig. 18] Fig. 18 shows the cytotoxicity of the anti-CCR9/anti-TRBC1 bispecific antibody against the TALL-derived cell strain CCRF-CEM9 (expressing CCR9 and TRBC2).
[Fig. 19] Fig. 19 shows the cytotoxicity of the anti-CXCR4/anti-TRBC1 bispecific antibody against the TALL-derived cell line CCRF-CEM9 (expressing CXCR4 and TRBC2).
[Fig. 20] Fig. 20 shows the cytotoxicity of the anti-CCR8/anti-TRBC1 bispecific antibody against the ATL-derived cell strain MT-1 (expressing CCR8).
[Fig. 21] Fig. 21 shows the cytotoxicity of the anti-TIGIT/anti-TRBC1 bispecific antibody against the ATL-derived cell strain ILT-Mat (expressing TIGIT).
[Fig. 22] Fig. 22 shows the cytotoxicity of the anti-CD30/anti-TRBC1 bispecific antibody against the PTCL-derived cell strain DL40 (expressing CD30).
[Fig. 23] Fig. 23 shows the cytotoxicity of the anti-CD40L/anti-TRBC1 bispecific antibody against the TALL-derived cell strain MOLT3 (expressing CD40L).
[Fig. 24] Fig. 24 shows the results obtained by adding T-LAK or NK cells to MOLT4 cells forced to express CCR4 and measuring the cytotoxicities of the anti-CCR4/anti-TRBC1 bispecific antibody CCR0001 and mogamulizumab.
[Fig. 25] Fig. 25 shows the results obtained by adding T-LAK cells to HPB-ALL cells, a TALL-derived cell strain expressing CD3, and HPB-ALL CD3 KO cells, CD3-knockout HPB-ALL cells, and measuring the cytotoxicity of the anti-CD1a/anti-CD3 bispecific antibody, CD1a1016 (a), as well as the cytotoxicity of the anti-CD1a/anti-TRBC1 bispecific antibody, CD1a1005 (b).
[Fig. 26] Fig. 26 shows the results obtained by adding anti-TRBC2 antibody-sensitized T-LAK cells to CCRF-CEM9 cells, a TALL-derived cell strain expressing TRBC2, and JM cells, a TALL-derived cell strain expressing TRBC1, and measuring the cytotoxicity of the anti-CD1a/anti-TRBC2 bispecific antibody, CD1a1006.
[Fig. 27] Fig. 27 is a graph of IVIS measurement values for each group of NOG mice transplanted with tumor cells on days 14 and 28.
[Fig. 28] Fig. 28 shows images of tumor distribution in NOG mice transplanted with tumor cells.
[Fig. 29] Fig. 29 shows the results obtained by adding T-LAK cells to CCRF-CEM9 cells, a T-ALL-derived cell strain expressing CD1a, and CCRF-CEM CD1a KO cells, and measuring the cytotoxicities of the bispecific antibodies CD1a1002 and CD1a1005.
[Fig. 30] Fig. 30 shows the results obtained by adding anti-TRBC2 antibody-sensitized T-LAK cells to JM cells, a TALL-derived cell strain expressing CD1a, and JM CD1a KO cells, and measuring the cytotoxicities of the bispecific antibodies CD1a1003 and CD1a1006.
[Fig. 31] Fig. 31 shows the results obtained by adding healthy human-derived T cells to CCRF-CEM-Luc cells, which are CCRF-CEM cells, a TALL-derived cell strain, made to express luciferase, and measuring the cytotoxicities of the anti-CD1a/anti-TRBV1 bispecific antibody (CD1a1005), and anti-CD1a/anti-TRBV5-5 bispecific antibodies (TRBV50003 and TRBV50004).
[Fig. 32] Fig. 32 shows the results obtained by adding healthy human-derived T cells to JM-Luc-BFP cells, which are JM cells, a TALL-derived cell strain, made to express luciferase, and measuring the cytotoxicities of the anti-CD1a/anti-TRBC2 bispecific antibody (CD1a1006) and anti-CD1a/anti-TRBV5-5 bispecific antibodies (TRBV50003 and TRBV50004).
[Fig. 33] Fig. 33 shows the results obtained by adding T-LAK cells stimulated with the anti-TRBV5-5 antibody (TRBV50001) to CCRF-CEM9 cells, a TALL-derived cell strain, and measuring the cytotoxicities of anti-CD1a/anti-TRBV5 -5 bispecific antibodies (TRBV50003 and TRBV50004).

### Modes for Carrying out the Invention

Although the explanations of the present invention described below may be made for representative embodiments or specific examples, the present invention is not limited to such embodiments and examples. In this description, ranges of numerical values expressed by using "to" means ranges that includes the numerical values mentioned before and after "to" as the lower and upper limits.

The present invention relates to the treatment of T cell malignancies. Hematologic malignant tumors can be broadly classified into lymphoid tumors and myeloid tumors on the basis of the origin or maturity of the tumor cells thereof, and lymphoid tumors can be further roughly divided into T cell malignancies and B cell malignancies. The lymphoid tumors originate from lymphoid progenitor cells, while myeloid tumors originate from myeloid progenitor cells. T lymphocytes undergone malignant transformation are T cell tumor cells, and B lymphocytes undergone malignant transformation are B cell tumor cells.

In this description, the "T cell malignancies" include T cell acute lymphoblastic leukemia/lymphoblastic lymphoma and mature T cell malignancies, but not limited to those, and may include tumors of T cells or progenitor cells thereof.

The acute lymphoblastic leukemia/lymphoblastic lymphoma (ALL/LBL) is defined as acute lymphoblastic leukemia (ALL) when lymphoblasts infiltrate the bone marrow (>25%) and appear in the peripheral blood, or as lymphoblastic lymphoma (LBL) when lymphoblasts infiltrate lymph nodes and extranodal organs. The acute lymphoblastic leukemia/lymphoblastic lymphoma include those of B cell and T cell types, which are referred to as "B cell acute lymphoblastic leukemia/lymphoblastic lymphoma" and "T cell acute lymphoblastic leukemia/lymphoblastic lymphoma", respectively. T cell acute lymphoblastic leukemia/lymphoblastic lymphoma includes the followings (Swerdlow SH, et al (Editors). WHO classification of tumours of haematopoietic and lymphoid tissues. Lyon: IARC Press; 2017, the entire description of which is specifically incorporated herein by reference): T-lymphoblastic leukemia/lymphoma (provisional entity: early T cell precursor lymphoblastic leukemia).

The mature T cell malignancies include the followings (Swerdlow SH, et al (Editors). WHO classification of tumours of haematopoietic and lymphoid tissues. Lyon: IARC Press; 2017): T cell prolymphocytic leukemia, T cell large granular lymphocytic leukemia, systemic EBV positive T- cell lymphoma of childhood, hydroa vacciniforme-like lymphoproliferative disorder, adult T cell leukemia/lymphoma, extranodal T cell lymphoma (nasal type), enteropathy-associated T cell lymphoma, monomorphic epitheliotropic intestinal T cell lymphoma, indolent T cell lymphoproliferative disorder of the GI tract, hepatosplenic T cell lymphoma, subcutaneous panniculitis-like T cell lymphoma, mycosis fungoides, Sezary syndrome, primary cutaneous CD30-positive T cell lymphoproliferative disorders, lymphomatoid papulosis, primary cutaneous anaplastic large cell lymphoma, primary cutaneous gamma-delta T cell lymphoma, primary cutaneous CD8-positive aggressive epidermotropic cytotoxic T cell lymphoma, primary cutaneous acral CD8-positive T cell lymphoma, primary cutaneous CD4-positive small/medium T cell lymphoproliferative disorder, peripheral T cell lymphoma (NOS), angioimmunoblastic T cell lymphoma, follicular T cell lymphoma, nodal peripheral T cell lymphoma with TFH phenotype, anaplastic large cell lymphoma (ALK-positive), anaplastic large cell lymphoma (ALK-negative), and breast implant-associated anaplastic large cell lymphoma.

In this description, the term "antigen-binding molecule" refers to a molecule that specifically binds to an epitope (antigenic determinant), e.g., an antibody or antibody fragment. The term "bispecific" means that the antigen-binding molecule specifically binds to two different antigenic determinants. Such a bispecific antigen-binding molecule comprises at least two antigen-binding portions, which bind to different antigenic determinants.

Antibodies have a structure in which two heavy chains (H chains) and two light chains (L chains) are bound together. The light chain and the heavy chain are bound together by disulfide bonds (SS bonds) to form a heterodimer, and two of the heterodimers are bound together to form a Y-shaped heterotetramer. Typically, the heavy chain is composed of a heavy chain variable region VH, heavy chain constant regions CH1, CH2, and CH3, and a hinge region located between CH1 and CH2, and the light chain is composed of a light chain variable region VL and a light chain constant region CL. The variable region contains a complementarity-determining region (CDR) and a framework region (FR). The light chain and heavy chain variable regions each have three CDRs (heavy chain CDR1 to 3 and light chain CDR1 to 3) and four FRs (heavy chain FR1 to 4 and light chain FR1 to 4). Methods for identifying CDRs are known, and can be found in, for example, IMGT/V-QUEST Search page (http://www.imgt.org/IMGT_vquest/input), Brochet, X. et al., Nucl. Acids Res. 36, W503-508 (2008), and Giudicelli, V., Brochet, X., Lefranc, M.-P., Cold Spring Harb Protoc. 2011 Jun 1;2011(6). pii: pdb.prot5633. doi: 10.1101/pdb.prot5633. PMID: 21632778, the entire descriptions of both references are specifically incorporated herein by reference. Any other means known in this field may also be used so long as similar results can be obtained (Kabat et al., Sequence of Proteins of Immunological Interest (1987), National Institute of Health, Bethesda, Md.; and Chothia et al., Nature (1989) 342: 877, the entire descriptions of both references are incorporated herein by reference).

In this description, an antibody fragment is an antigen-binding antibody fragment. Examples of such an antibody fragment include, but are not limited to, Fab, F(ab')₂, Fv, scFv, etc. Fab refers to an antibody fragment consisting of the VL-CL and VH-CH1 domains linked by a disulfide bond. F(ab')₂ refers to an antibody fragment consisting of two Fabs linked by a disulfide bond at the hinge regions. The Fv fragment consists of VL and VH. scFv is a fusion protein consisting of VH and VL linked by a linker peptide (about 10 to 25 amino acids in length).

The first aspect of the present invention relates to a therapeutic agent for a T cell malignancy comprising a bispecific antigen-binding molecule. The bispecific antigen-binding molecule that constitutes the therapeutic agent of the present invention comprises
(1) at least one portion that specifically binds to a target tumor antigen expressed on T cell tumor cells; and
(2) at least one portion that specifically binds to a normal T cell-side target antigen having subtypes,
provided that
the target tumor antigen expressed on the T cell tumor cells is either not present on normal T cells, or if present, the normal T cells are not substantially activated when the bispecific antigen-binding molecule binds to the same antigen as the target tumor antigen present on the normal T cells, but
binding of the bispecific antigen-binding molecule to the normal T cell-side target antigen activates the normal T cells, and
a sufficient proportion of a subtype of the normal T cell-side target antigen is present to provide a sufficient number of activated T cells for the treatment of the T cell tumor.

In the present invention, the term "portion that specifically binds to a target tumor antigen expressed on T cell tumor cells" (hereinafter referred to as tumor antigen-binding portion) refers to a polypeptide molecule that specifically binds to a target tumor antigen expressed on T cell tumor cells. In one embodiment, the tumor antigen-binding portion can direct normal T cells to T cell tumor cells expressing the target tumor antigen. The tumor antigen-binding portion contains an antibody or a fragment thereof.

In this description, the term "to specifically bind" means to selectively bind to a specific antigen, which action is distinguishable from nonspecific interactions. The binding ability of an antigen-binding molecule to a specific antigen can be measured by enzyme-linked immunosorbent assay (ELISA), surface plasmon resonance (SPR) techniques, etc. In a specific embodiment, the antigen-binding molecule can bind to the target antigen at a dissociation constant (K_{D}) of 1 x 10⁻⁵ M (mol/L) or lower, 5 x 10⁻⁶ M or lower, 1 x 10⁻⁶ M or lower, 5 x 10⁻⁷ M or lower, 1 x 10⁻⁷ M or lower, 5 x 10⁻⁸ M or lower, 1 x 10⁻⁸ M or lower, 5 x 10⁻⁹ M or lower, 1 x 10⁻⁹ M or lower, 5 x 10⁻¹⁰ M or lower, or 1 x 10⁻¹⁰ M or lower.

The target tumor antigen expressed on T cell tumor cells is not present on normal T cells, or, if present, normal T cells are not substantially activated when the bispecific antigen-binding molecule binds to the same antigen as the target tumor antigen present on normal T cells. The absence on normal T cells of the target tumor antigen expressed on T cell tumor cells can be determined by flow cytometric analysis using fluorescent dye-labeled antibodies that specifically bind to the target tumor antigen. Whether a certain antigen is present or absent on normal T cells, or whether expression of a certain antigen is positive or negative on normal T cells can be determined on the basis of a chart obtained by flow cytometric analysis. Details of the flow cytometric analysis are described below. In a preferred embodiment, absence on normal T cells of the target tumor antigen expressed on T cell tumor cells allows the bispecific antigen-binding molecules to properly cross-link normal T cells and T cell tumor cells (e.g., Fig. 1B). Further, the condition that "normal T cells are not substantially activated when the bispecific antigen-binding molecule binds to the same antigen as the target tumor antigen present on the normal T cells" can be achieved by preparing the tumor antigen-binding portion based on an antibody that does not have agonist activity, since activation of normal T cells caused by binding of the bispecific antigen-binding molecule (e.g., an antibody) to an antigen is thought to be largely due to the agonistic activity of the antibody.

CD3 is expressed on both normal T cells and T cell tumor cells. Bispecific antibodies targeting CD3 and tumor antigen bind simultaneously to the CD3 antigen expressed on T cell malignant tumors and the target antigen expressed on T cell malignant tumors to link malignant tumor cells together (Fig. 1A), and therefore the therapeutic effect of bispecific antibodies is highly likely to be insufficient. In the examples described below, the cytotoxicity of bispecific antibodies that bind to CD3 and a tumor antigen against a tumor cell strain expressing CD3 has been shown to be reduced compared with that activity against a tumor cell strain that does not express CD3 (CD3 knockout tumor cell strains). This result suggests that CD3 expression in tumor cell strains attenuates the cytotoxicity. It is considered that bispecific antigen-binding molecules for use against T cell malignancies, which are CD3-positive in many cases, should target other molecules besides CD3 for directing effector T cells.

The target tumor antigen expressed on T cell tumor cells is an antigen different from the normal T cell-side target antigen. The "(1) at least one portion that specifically binds to a target tumor antigen expressed on T cell tumor cells" and "(2) at least one portion that specifically binds to a normal T cell-side target antigen having subtypes" of the bispecific antigen-binding molecule are preferably constituted to bind to different types of antigens, so that they bind to antigens of different types expressed on T cell tumor cells and normal T cells, respectively.

In a specific embodiment, it is preferred that even if the target tumor antigen expressed on T cell tumor cells is present on normal T cells, it is expressed at a higher frequency on the T cell tumor cells compared with on the normal T cells. For example, it is sufficient that the ratio of the number of molecules of the target tumor antigen expressed per one T cell tumor cell to the number of molecules of the same expressed per one normal T cell is 110% or higher, and it is preferably, for example, 120%, 130%, 140%, 150%, 200%, 300%, 400%, 500%, 600%, 700%, 800%, 900%, or 1000% or higher. The number of expressed molecules of the target tumor antigen per cell can be determined by flow cytometric analysis using a fluorescent dye-labeled antibody that specifically binds to the target tumor antigen. Expression of the target tumor antigen expressed on T cell tumor cells at a higher frequency on the T cell tumor cells than on the normal T cells allows the bispecific antigen-binding molecule to cross-link sufficient numbers of normal T cells and T cell tumor cells for the treatment, and thereby the therapeutic effect can be obtained. Specific examples of the target tumor antigen will be described below.

### <Normal T cell-side antigen-binding portion>

In the present invention, the term "a portion that specifically binds to a normal T cell-side target antigen having subtypes" (hereinafter referred to as normal T cell-side antigen-binding portion) refers to a polypeptide molecule that specifically binds to a target antigen expressed on normal T cells. The normal T cell-side antigen-binding portion contains an antibody or a fragment thereof. In one embodiment, the normal T cell-side antigen-binding portion can activate TCR signal transduction (activation of T cells) via the normal T cell-side target antigen. Furthermore, it is preferably capable of inducing cellular responses such as cytokine production or cytotoxicity of T cells.

The term "antigen having subtypes" refers to a substance that constitutes T cells (e.g., peptide, protein, sugar, lipid, or other organic or inorganic substance) and may be present as variants having the same or substantially the same function but has different structures (e.g., amino acid sequence or steric structure of peptide or protein, constituent unit or type of sugar, sequence and types of constituent elements of lipid), and the individual variants are referred to as subtypes. In one embodiment, it is preferred that only one type of the subtypes of the "antigen having subtypes" should be expressed on each T cell. For example, the "antigen having subtypes" may be, but is not limited to, an antigen having subtypes based on TCR diversity produced by somatic recombination (also called "V(D)J gene reconstitution"). Most T cell receptors are composed of α and β chains. The TCR α and β chains consist of an N-terminal variable region and a C-terminal constant region. TCR diversity is produced by somatic recombination, which occurs when the variable region (V), diversity region (D), joining region (J) and constant region (C) are selected for each TCR chain. The V, D, and J gene fragments are contained in the β chain gene, and the V and J gene fragments are contained in the α chain gene. For example, one of the 30 types of TRBV polypeptides resulting from V(D)J gene reconstitution and allele exclusion is expressed on each T cell (WO2022/119955, the entire description of which is specifically incorporated herein by reference). The TRBV polypeptides are examples of an antigen having 30 subtypes. However, the "antigen having subtypes" is an antigen expressed at any location on T cells and is not limited to antigens present on the TCR.

The number of subtypes of the "antigen having subtypes" is not particularly limited, as long as the number of subtypes is 2 or larger. A large number of subtypes results in a small number of normal T cells to which the normal T cell-side antigen-binding portion of the bispecific antigen-binding molecule can bind via the target antigen, and therefore may not provide a sufficient number of activated T cells for the treatment of the T cell tumor. In one embodiment, the number of subtypes of the "antigen having subtypes" may be, for example, 50 or lower, and is preferably 40 or lower, 30 or lower, 20 or lower, 10 or lower, 9 or lower, 8 or lower, 7 or lower, 6 or lower, 5 or lower, 4 or lower, 3 or lower, or 2.

In a normal T cell population, normal T cells that express any one of the subtypes of the "normal T cell-side target antigen having subtypes" are mixed together. For example, if the "antigen having subtypes" has three subtypes (subtypes A, B, and C), cells expressing subtype A, cells expressing subtype B, and cells expressing subtype C are mixed together in a normal T cell population. T cell tumor cells usually also express any of the subtypes mentioned above (e.g., any of the subtypes A to C mentioned above). The normal T cell-side antigen-binding portion of the bispecific antigen-binding molecule is constituted to specifically bind to a subtype different from the subtype expressed on the T cell tumor cells. For example, if the subtype expressed on the T cell tumor cells is the subtype A, the normal T cell-side antigen-binding portion of the bispecific antigen-binding molecule is constituted to specifically bind to the subtype B or subtype C. The tumor antigen-binding portion of the bispecific antigen-binding molecule is constituted to specifically bind to an antigen that is expressed at a high frequency on T cell tumor cells and different from the "normal T cell-side target antigen having subtypes". This allows the bispecific antigen-binding molecule to properly cross-link normal T cells and T cell tumor cells.

In the present invention, it is preferred that normal T cells are activated by the binding of the bispecific antigen-binding molecule to the normal T cell-side target antigen. Activation of T cells can be evaluated by culturing T cells in the presence of IL-2 and the normal T cell-side antigen-binding portion of the bispecific antigen-binding molecule, and observing or measuring cell phenotype, T cell activation marker, cell proliferation, cell viability, and secreted cytokine levels as described in the reference example mentioned below.

In the present invention, it is preferred that a sufficient ratio of subtype of the normal T cell-side target antigen be present to provide a sufficient number of activated T cells for the treatment of T cell tumor. It is preferred that the subtype of the normal T cell-side target antigen be expressed on 25% or higher of the cells in the entire normal T cell population, and it is more preferred that it should be expressed on 30%, 40%, or 50% or more of the cells. When the "antigen having subtypes" is selected, average values of ratios of subtypes of the normal T cell-side target antigen in humans may be referred to, or it may be selected on the basis of analysis value for the patient to be treated. In one embodiment, in order to provide a sufficient number of activated T cells for the treatment of T cell tumor, it is sufficient that the blood level of cells expressing the subtype of the normal T cell-side target antigen is 50 cells/µL or higher, and it is preferably 100 cells/µL or higher, 200 cells/µL or higher, or 300 cells/µL or higher. Further, it is preferred that the "antigen having subtypes" should not be an antigen present on γδ T cells but on αβ T cells, since γδ T cells account for only several percents or lower of the total T cells and therefore a sufficient number of T cells cannot be mobilized to injure tumor cells and achieve a therapeutic effect, if the "antigen having subtypes" is an antigen present on γδ T cells.

In one embodiment, the subtype of the normal T cell-side target antigen is a subtype selected from the subtypes of the antigen that are not a subtype of the antigen expressed on the T cell tumor cells. For example, since a cell population of clonally proliferated T cell tumor cells has one of the subtypes, a subtype different from the subtype expressed on the cell population of the T cell tumor cells can be selected as the normal T cell-side target antigen of the bispecific antigen-binding molecule.

In one embodiment, the normal T cell-side target antigen is not expressed on tumor cells, if the subtype of the normal T cell-side target antigen is one of the antigen subtypes that are not the subtype of the antigen expressed on the T cell tumor cells.

In one embodiment, the normal T cell-side target antigen having subtypes is TRBC (T cell receptor β constant region), the subtype expressed on T cell tumor cells is TRBC1, and the subtype of the normal T cell-side target antigen is TRBC2. That is, if the subtype of TRBC expressed on T cell tumor cells in a subject is analyzed and determined to be TRBC1, the normal T cell-side target antigen of the bispecific antigen-binding molecule can be TRBC2. This allows the bispecific antigen-binding molecule to mobilize normal T cells as effector cells.

In a further embodiment, the normal T cell-side target antigen having subtypes is TRBC, the subtype expressed on T cell tumor cells is TRBC2, and the subtype of the normal T cell-side target antigen is TRBC1. That is, if the subtype of TRBC expressed on T cell tumor cells in a subject is analyzed and determined to be TRBC2, the normal T cell-side target antigen of the bispecific antigen-binding molecule can be TRBC1.
This allows the bispecific antigen-binding molecule to mobilize normal T cells as effector cells.

In a further embodiment, the normal T cell-side target antigen having subtypes is TRBC, the T cell tumor cells are TRBC1-negative and TRBC2-negative, and the subtype of the normal T cell-side target antigen is TRBC1 or TRBC2. That is, if the TRBC subtype expressed on the T cell tumor cells in a subject is analyzed and determined to be TRBC1-negative and TRBC2-negative, the normal T cell-side target antigen of the bispecific antigen-binding molecule can be either TRBC1 or TRBC2. In this case, since neither TRBC1 nor TRBC2 is expressed on the T cell tumor cells, but either of them is expressed on the normal T cells, normal T cells can be mobilized as effector cells by the action of the bispecific antigen-binding molecule having the normal T cell-side target antigen-binding portion that binds to TRBC1 or TRBC2.

In this description, TRBC1 and TRBC2 refer to two functionally identical proteins of the TCR β-chain constant region. T cell receptor (TCR) is an antigen receptor molecule expressed on T cells, and recognizes an antigen bound to a major histocompatibility gene complex (MHC) molecule. Two functionally identical genes, TRBC1 (Gene ID: 28639) and TRBC2 (Gene ID: 28636), are present on the locus (Chr7:q34) of the TCR β-chain constant region. The mature proteins of TRBC1 and TRBC2 differ only by four amino acid residues. The TCRs having TRBC1 and TRBC2 (TRBC1-TCR and TRBC2-TCR) can be distinguished by antibodies, despite having nearly identical amino acid sequences (Maciocia PM, et al., supra). In addition, peripheral blood T cells from healthy individuals contain approximately 35% of TRBC1-positive cells and 65% of TRBC2-positive cells mixed together, and monoclonality of TRBC have been confirmed in many types of T cell malignant tumors by flow cytometry and immunohistochemistry (IHC) techniques (Maciocia PM, et al., supra).

It has been reported that while normal T cell populations contain TRBC1-expressing cells and TRBC2-expressing cells mixed together, either TRBC1 or TRBC2 is exclusively expressed in the entire cell population of T cell tumor cells (Maciocia PM, et al., supra). Therefore, it has been proposed to use TRBC1 or TRBC2 as the target antigen on the tumor cell side (Japanese Patent No. 6767872 (Japanese Patent Application No. 2016-554603, the entire description of which is specifically incorporated herein by reference)). However, use of a TRBC subtype different from the TRBC subtype expressed on tumor cells as a target antigen on the normal T cell side or effector cell side had not been known prior to the present invention.

In a specific embodiment, the therapeutic agent for a T cell malignancy of the present invention comprises a bispecific antigen-binding molecule comprising
at least one portion that specifically binds to a target tumor antigen expressed on T cell tumor cells; and
at least one portion that specifically binds to either TRBC1 or TRBC2 expressed on normal T cells

In the present invention, the term "a portion that specifically binds to TRBC1 or TRBC2 expressed on normal T cells" (hereinafter referred to as TRBC1 or TRBC2-binding portion) refers to a polypeptide molecule that specifically binds to TRBC1 or TRBC2 expressed on normal T cells. The TRBC1 or TRBC2-binding portion contains an antibody or a fragment thereof. In one embodiment, the TRBC1 or TRBC2-binding portion can activate TCR signal transduction through TRBC1 or TRBC2 (activation of T cells). Furthermore, it preferably induces cellular responses such as cytokine production or cytotoxicity of T cells.

The bispecific antigen-binding molecule preferably simultaneously binds to TRBC1 or TRBC2 expressed on normal T cells and a target tumor antigen expressed on T cell tumor cells.

In one embodiment, when the T cell tumor cells are TRBC1-positive, the bispecific antigen-binding molecule comprises at least one portion that specifically binds to TRBC2 expressed on normal T cells, and when the T cell tumor cells are TRBC2-positive, the bispecific antigen-binding molecule comprises at least one portion that specifically binds to TRBC1 expressed on normal T cells. By using TRBC of a subtype different from that of TRBC expressed on the T cell tumor cells as the effector cell (T lymphocyte) side target (if the tumor cells express TRBC1, by using TRBC2 as the effector cell-side target, or if the tumor cells express TRBC2, by using TRBC1 as the effector cell-side target), possibility of T cell immunodeficiency can be eliminated, and a high therapeutic effect can be achieved.

In the examples described below, bispecific antibodies whose normal T cell-side antigen-binding portion binds to TRBC2 have been shown to have higher cytotoxicity against a TRBC1-positive tumor cell strain compared with cytotoxicity against a TRBC2-positive tumor cell strain (Example 15). Further, bispecific antibodies whose normal T cell-side antigen-binding portion binds to TRBC1 have been shown to have high cytotoxicity against a TRBC2-positive tumor cell strain (Example 4). From these results, it was confirmed that high cytotoxicity can be obtained when the normal T cell-side antigen-binding portion of the bispecific antibody for treating tumors recognizes TRBC of a subtype different from that of TRBC expressed on T cell tumor cells.

In the present invention, the term "TRBC1-positive" used for T cell tumor cells can be understood to mean that the T cell tumor cells are "TRBC2-negative". Also, in the present invention, the term "TRBC2-positive" used for T cell tumor cells can be understood to mean that the T cell tumor cells are "TRBC1-negative". This is based on that it has been reported that normal T cell populations contain cells expressing TRBC1 and cells expressing TRBC2 mixed together, but either TRBC1 or TRBC2 is exclusively expressed in the entire cell population of T cell tumor cells (Maciocia PM, et al., supra).

In one embodiment, the T cell tumor cells may be TRBC1-negative and TRBC2-negative. If the T cell tumor cells are TRBC1-negative and TRBC2-negative, the normal T cell-side target antigen of the bispecific antigen-binding molecule may be either TRBC1 or TRBC2, that is, the bispecific antigen-binding molecule can contain either at least one portion that specifically binds to TRBC1 expressed on normal T cells or at least one portion that specifically binds to TRBC2 expressed on normal T cells.

In one embodiment, the target tumor antigen is neither TRBC1 nor TRBC2. That is, the at least one portion that specifically binds to the target tumor antigen expressed on T cell tumor cells is designed to specifically bind to an antigen selected from those other than TRBC1 and TRBC2, and therefore does not bind or substantially bind to either TRBC1 or TRBC2.

It has been proposed to treat T cell lymphoma or leukemia by using TRBC1 as the tumor cell target antigen in the case of TRBC1-positive T cell malignant tumors or TRBC2 as the tumor cell target antigen in the case of TRBC2-positive malignant tumors, for example, by using a chimeric antigen receptor (CAR) comprising an antigen-binding domain that selectively binds to either TRBC1 or TRBC2 (Maciocia PM, et al. supra, Japanese Patent No. 6767872 (Japanese Patent Application No. 2016-554603)). However, CAR-T cells using a TRBC1 antibody kill not only TRBC1-positive cancer cells but also TRBC1-positive T lymphocytes. In such a case, approximately 50% of T lymphocytes may be killed, which may result in T cell immunodeficiency. In the present invention, a bispecific antigen-binding molecule that targets an antigen other than the TRBC expressed on T cell malignant tumors as the target on the tumor cell side and further targets TRBC of a subtype different from the subtype of TRBC expressed on T cell malignant tumors as the target on the effector cell (T lymphocyte) side is used to guide normal T cells and thereby kill the tumor cells, and therefore the likelihood of causing T cell immunodeficiency is low.

T cell tumor cells have been reported to exclusively express either TRBC1 or TRBC2 in the entire cell population thereof. However, it is possible that T cell tumor cells express neither TRBC1 nor TRBC2. For example, this is the case when TCR non-expressing T cells become malignant. According to the present invention, even when the T cell tumor cells express neither TRBC1 nor TRBC2, T cell malignancies can be treated. This is because normal T cells express either TRBC1 or TRBC2, and therefore a bispecific antigen-binding molecule that targets either TRBC1 or TRBC2 as the T cell-side target antigen and also targets a non-TRBC antigen expressed on the T cell tumor cells as the tumor cell-side target can be used to guide about half of the normal T cells population to T cell tumor cells and kill them.

In the examples described below, it has been shown that for a tumor cell strain that expresses neither TRBC1 nor TRBC2, both bispecific antibody whose normal T cell-side antigen-binding portion binds to TRBC1 and bispecific antibody whose normal T cell-side antigen-binding portion binds to TRBC2 have cytotoxicity (Examples 3 and 5).

Cells can be determined to be TRBC1-positive or negative by flow cytometric analysis using a fluorescent dye-labeled antibody that specifically binds to TRBC1. Examples of antibody that specifically binds to TRBC1 include, for example, the JOVI-1 clone (US10730942, Protein-based T cell receptor knockdown, SEQ ID NOS: 15 and 16, the entire description of US10730942 is specifically incorporated herein by reference). Similarly, T cells can be determined to be TRBC2-positive or negative by flow cytometric analysis using a fluorescent dye-labeled antibody that specifically binds to TRBC2. Examples of antibody that specifically binds to TRBC2 include, for example, the various antibodies described in WO2020/089644 (the entire description of which is specifically incorporated herein by reference).

Whether cells are positive or negative for expression of a cellular antigen can be determined on the basis of a chart obtained by flow cytometric analysis. Although the positions of the cells appearing in such a chart may vary depending on the voltage setting and sensitivity setting of the instrument, antibody clones used, staining conditions, dyes used, etc., those skilled in the art can appropriately draw a line so as not to divide cell populations recognized as one group in the obtained chart. Whether cells are positive or negative for expression of a cell antigen of interest can be determined by referring to a case using an isotype control antibody as a negative control. The isotype control antibody is an antibody that does not react with a specific antigen. In general, in experiments using antibodies, background may occur due to nonspecific binding to proteins other than the target or to Fc receptors on the cell surfaces. By comparison with a system using an antibody that serves as a negative control, specificity of the reaction of the primary antibody to the antigen of interest can be determined. The influences of the background are also eliminated, resulting in accurate interpretation of signal strengths.

### <TRBC1-binding portion>

The bispecific antigen-binding molecule that constitutes the therapeutic agent as the first aspect of the present invention may comprise a TRBC1-binding portion and a tumor antigen-binding portion. The TRBC1-binding portion may be the anti-TRBC1 antibody described in U.S. Patent No. 1,073,0942 (US10730942, Protein-based T cell receptor knockdown, SEQ ID NOS. 15 and 16, the entire description of which is specifically incorporated herein by reference).

In one embodiment, the bispecific antigen-binding molecule comprises at least one portion that specifically binds to TRBC1 expressed on normal T cells, and the at least one portion comprises
a VH domain comprising
a heavy chain CDR1 comprising the amino acid sequence of SEQ ID NO: 10,
a heavy chain CDR2 comprising the amino acid sequence of SEQ ID NO: 11, and
a heavy chain CDR3 comprising the amino acid sequence of SEQ ID NO: 12, and
a VL domain comprising
a light chain CDR1 comprising the amino acid sequence of SEQ ID NO: 13,
a light chain CDR2 comprising the amino acid sequence of SEQ ID NO: 14, and
a light chain CDR3 comprising the amino acid sequence of SEQ ID NO: 15.

In a specific embodiment, the at least one portion that specifically binds to TRBC1 expressed on normal T cells comprises a heavy chain variable region VH that is at least 90% identical to the amino acid sequence of SEQ ID NO: 4 and a light chain variable region VL that is at least 90% identical to the amino acid sequence of SEQ ID NO: 5. In a further specific embodiment, the at least one portion that specifically binds to TRBC1 expressed on normal T cells comprises a heavy chain variable region VH that is at least 90%, 91%, 92%, 93%, 94%, 95%, 96%, 97%, 98% or 99% identical to the amino acid sequence of SEQ ID NO: 4 and a light chain variable region VL that is at least 90%, 91%, 92%, 93%, 94%, 95%, 96%, 97%, 98% or 99% identical to the amino acid sequence of SEQ ID NO: 5. The amino acid mutations (deletion, insertion, and substitution) in the amino acid sequence of SEQ ID NO: 4 or 5 can be present in a complementarity determining region (CDR) or framework region (FR).

In this description, the "percent (%) amino acid sequence identity" based on a reference polypeptide sequence is defined as the percentage of amino acid residues in a subject sequence that are identical to the amino acid residues of the reference polypeptide observed when the sequences are aligned and gaps are introduced as required to obtain the maximum percent sequence identity, provided that any conservative substitution is not considered as a part of sequence identity. Alignment for the purpose of determining percent amino acid sequence identity can be accomplished by using various methods within the skill of the art, for example, by using publicly available computer software such as BLAST, BLAST-2, ALIGN, or Megalign (DNASTAR) software. Those skilled in the art can determine the appropriate parameters for aligning sequences, including any algorithms necessary to achieve the best alignment for the full lengths of the sequences to be compared.

In a specific embodiment, an amino acid sequence variant of the bispecific antigen-binding molecule comprising the TRBC1-binding portion is prepared to improve the binding affinity and/or other biological properties of the TRBC1-binding portion. Such an amino acid sequence variant can be prepared by introducing appropriate mutations (deletion, insertion, and substitution) into the nucleotide sequence encoding the anti-TRBC1 antibody or by peptide synthesis. The mutations can be introduced into a complementarity determining region (CDR) or framework region (FR) of the antibody depending on the properties to be improved. Amino acid sequence variants are screened for maintenance or improvement of binding affinity, maintenance or improvement of antigen specificity, reduction of immunogenicity, etc.

### <TRBC2-binding portion>

The bispecific antigen-binding molecule that constitutes the therapeutic agent as the first aspect of the present may comprise a TRBC2-binding portion and a tumor antigen-binding portion. The TRBC2-binding portion may be any of the various anti-TRBC2 antibodies described in WO2020/089644 or an antigen-binding domain that binds to TRBC2. The TRBC2-binding portion may be one comprising a VH domain derived from the VH domain of hJOVI-1 described in WO2020/089644 (SEQ ID NO: 1 in WO2020/089644) by three amino acid substitutions T28K, Y32F and A100N (Table 1 in WO2020/089644 (page 23)) and the VL domain of hJOVI-1 (SEQ ID NO: 2 in WO2020/089644).

In one embodiment, the bispecific antigen-binding molecule comprises at least one portion that specifically binds to TRBC2 expressed on normal T cells, and the at least one portion comprises
a VH domain comprising
a heavy chain CDR1 comprising the amino acid sequence of SEQ ID NO: 16,
a heavy chain CDR2 comprising the amino acid sequence of SEQ ID NO: 17, and
a heavy chain CDR3 comprising the amino acid sequence of SEQ ID NO: 18, and
a VL domain comprising
a light chain CDR1 comprising the amino acid sequence of SEQ ID NO: 19,
a light chain CDR2 comprising the amino acid sequence of SEQ ID NO: 20, and
a light chain CDR3 comprising the amino acid sequence of SEQ ID NO: 21.

In a specific embodiment, the at least one portion that specifically binds to TRBC2 expressed on normal T cells comprises a heavy chain variable region VH that is at least 90% identical to the amino acid sequence of SEQ ID NO: 6 and a light chain variable region VL that is at least 90% identical to the amino acid sequence of SEQ ID NO: 7. In a further specific embodiment, the at least one portion that specifically binds to TRBC2 expressed on normal T cells comprises a heavy chain variable region VH that is at least 90%, 91%, 92%, 93%, 94%, 95%, 96%, 97%, 98% or 99% identical to the amino acid sequence of SEQ ID NO: 6, and a light chain variable region VL that is at least 90%, 91%, 92%, 93%, 94%, 95%, 96%, 97%, 98% or 99% identical to the amino acid sequence of SEQ ID NO: 7. The amino acid mutations (deletion, insertion, and substitution) in the amino acid sequence of SEQ ID NO: 6 or 7 can be present in a complementarity determining region (CDR) or framework region (FR).

In a specific embodiment, an amino acid sequence variant of the bispecific antigen-binding molecule comprising the TRBC2-binding portion is prepared to improve the binding affinity and/or other biological properties of the TRBC2-binding portion. Such an amino acid sequence variant can be prepared, for example, by introducing appropriate mutations (deletion, insertion, and substitution) into the nucleotide sequence encoding the anti-TRBC2 antibody or by peptide synthesis. The mutations can be introduced into a complementarity determining region (CDR) or framework region (FR) of the antibody, depending on the properties to be improved. Amino acid sequence variants are screened for maintenance or improvement of binding affinity, maintenance or improvement of antigen specificity, reduction of immunogenicity, etc.

In a specific embodiment, the at least one portion that specifically binds to TRBC1 or TRBC2 expressed on normal T cells may be an scFv or Fab fragment.

The CDR sequences of the TRBC1 or TRBC2 antigen-binding portions are listed in the following table.

**[Table 1]**

| Antigen-binding portion | Region | Sequence | SEQ ID No: |
|---|---|---|---|
| TRBC1 | CDR H1 | GYVMH | 10 |
| | CDR H2 | FINPYNDDIQSNERFRG | 11 |
| | CDR H3 | GAGYNFDGAYRFFDF | 12 |
| | CDR L1 | RSSQRLVHSNGNTYLH | 13 |
| | CDR L2 | RVSNRFP | 14 |
| | CDR L3 | SQSTHVPYT | 15 |
| TRBC2 | CDR H1 | GFVMH | 16 |
| | CDR H2 | FINPYNDDIQSNERFRG | 17 |
| | CDR H3 | GNGYNFDGAYRFFDF | 18 |
| | CDR L1 | RSSQRLVHSNGNTYLH | 19 |
| | CDR L2 | RVSNRFP | 20 |
| | CDR L3 | SQSTHVPYT | 21 |

### <Tumor antigen-binding portion>

The bispecific antigen-binding molecule that constitutes the therapeutic agent as the first aspect of the present invention can comprise a TRBC1- or TRBC2-binding portion and a tumor antigen-binding portion. The tumor antigen as the target for the present invention is an antigen expressed on T cell tumor cells. A large number of antigens expressed on T cell tumor cells are known in the art, and can be the target tumor antigen for the present invention. Examples of the target tumor antigen are described elsewhere in this description. The tumor antigen-binding portion can be prepared on the basis of known antibodies. For the T cell tumor antigens, a large number of antibodies are also known in the art and can be used in the present invention. Those skilled in the art can select an appropriate light chain variable region and heavy chain variable region based on the amino acid sequence information of known antibodies and construct the bispecific antibody by genetic engineering techniques. In the genetic engineering techniques, a gene sequence encoding an antibody can be incorporated into an expression vector, which is then transformed into host cells, and the host cells can be cultured to produce the antibody. Examples of the Fc domain, peptide linker and antibody format that can be employed for the bispecific antibody to be constructed are described elsewhere in this description.

If there is no known monoclonal antibody that specifically binds to the tumor antigen of interest, it can be produced by using the hybridoma method, phage display method, or the like and employed in the bispecific antigen-binding molecule. In the hybridoma method, hybridomas are prepared by fusing B cells collected from the spleen or lymph node of an animal, especially rat or mouse, immunized with a peptide of the tumor antigen of interest with myeloma cells, a hybridoma that produces an antibody that reacts with the antigen can be selected, and the selected hybridoma can be used to produce a monoclonal antibody. The phage display method is a technique for selecting an antibody having affinity for a target molecule using a library of functionally presented variable regions of antibodies on phages. The genes of the antibodies contained in the phages can be sequenced, and a monoclonal antibody can be generated on the basis of the sequence information.

In one embodiment, the tumor antigen targeted in the present invention is not pan-T cell antigen such as CD2, CD3, CD5, or CD7. This is because these can lead to T cell depletion and produce clinically unacceptable levels of immunodeficiency. In one embodiment, when the TRBC1-binding portion is employed as the normal T cell-side antigen-binding portion of the bispecific antibody of the present invention, the tumor antigen targeted in the present invention is not TRBC1, or when the TRBC2-binding portion is employed as the normal T cell-side antigen-binding portion of the bispecific antibody of the present invention, the tumor antigen targeted in the present invention is not TRBC2. This is also because these can lead to T cell depletion and produce clinically unacceptable levels of immunodeficiency.. For example, WO2022/177889A1 (the entire description of which is specifically incorporated herein by reference) discloses that bispecific antibodies that bind to healthy TRBC1-positive T cells and TRBC1-positive cancer cells can kill healthy TRBC1-positive T cells by fratricide.

### (Target tumor antigen)

Examples of the target tumor antigen expressed on T cell tumor cells for the present invention include, but are not limited to, the following T cell antigens: CD4, CD8, CD13, CD16, CD17, CD18, CD19, CD20, CD21, CD23, CD25, CD26, CD27, CD28, CD29, CD30, CD31, CD32b, CD35, CD37, CD38, CD39, CD43, CD44, CD45, CD45RA, CD45RB, CD45RC, CD45RO, CD46, CD47, CD48, CD49, CD49b, CD49c, CD49d, CD49e, CD49f, CD50, CD52, CD53, CD54, CD55, CD56, CD57, CD58, CD59, CD60a, CD62L, CD63, CD68, CD69, CD70, CD71, CD73, CD74, CD75S, CD80, CD81, CD82, CD84, CD85A, CD85J, CD86, CD87, CD92, CD94, CD95, CD96, CD97, CD98, CD99, CD99R, CD100, CD101, CD102, CD103, CD107a, CD107b, CD108, CD109, CD119, CD120a, CD120b, CD121a, CD121b, CD122, CD124, CD126, CD127, CD128, CD129, CD130, CD132, CD134, CD137, CD146, CD147, CD148, CD150, CD152, CD153, CD40L (CD154), CD156b, CD158a, CD158b1, CD158b2, CD158e1/e2, CD158f, CD158g, CD158h, CD158h, CD158i, CD158j, CD158k, CD159a, CD160, CD161, CD162, CD164, CD172g, CD178, CD181, CD182, CD183, CD184, CD185, CD186, CD191, CD192, CD193, CD194, CD195, CD196, CD197, CDw198, CDw199, CD205, CD210a, CDw210b, CD212, CD215, CD217, CD218a, CD218b, CD220, CD221, CD222, CD223, CD224, CD225, CD226, CD227, CD229, CD230, CD231, CD244, CD245, CD246, CD247, CD253, CD254, CD255, CD256, CD257, CD258, CD259, CD260, CD261, CD262, CD263, CD264, CD267, CD268, CD270, CD272, CD273, CD274, CD275, CD277, CD278, CD279, CD283, CD288, CD289, CD290, CD294, CD295, CD296, CD298, CD300a, CD300c, CD300e, CD305, CD306, CD307c, CD314, CD316, CD317, CD319, CD321, CD328, CD351, CD352, CD352, CD354, CD355, CD357, CD358, CD359, CD360, CD361, CD362, and CD363.

In one embodiment, the target tumor antigen expressed on T cell tumor cells may be selected from the group consisting of CCR1, CCR4, CCR7, CCR8, CCR10, CXCR4, CXCR7, TIGIT, CADM1, GPR15, CXCR5, CXCL13, SLAM, ICOS, CD134, CXCR3, anaplastic lymphoma kinase, CD30, ST2(L), CCR5, Notchl, CD38, CD1a, CCR9 (CD199), CD47, IL-7Rα (CD127), and CD40L (CD154), but is not limited to these.

CCR1 (C-C chemokine receptor 1), CCR4, CCR5, CCR7, CCR8, CCR9 (CD199), CCR10, CXCR (C-X-C chemokine receptor) 3, CXCR4, CXCR5 and CXCR7 are chemokine receptors. CCR1 is also known as CD191.

CXCL13 (C-X-C chemokine ligand 13) is a chemokine ligand.

TIGIT (T cell immunoreceptor with Ig and ITIM domains) is an immune checkpoint receptor present on NK cells, cytotoxic T cells, memory T cells and regulatory T cells (Treg).

CADM1 (cell adhesion molecule 1) is an intercellular adhesion molecule belonging to the immunoglobulin superfamily, cell adhesion molecule group (IgCAM).

GPR15 (G protein-coupled receptor 15) is a class A orphan G protein-coupled receptor, and expression thereof is observed on epithelial cells, synovial macrophages, endothelial cells, and lymphocytes, especially T cells.

SLAM (signaling lymphocytic activation molecule) means a family of signaling lymphocytic activation molecules (SLAMs).

ICOS (inducible T cell co-stimulator) is a 55 to 60 kDa disulfide homodimeric T cell surface glycoprotein, and is also known as CD278.

CD134 is the member 4 of the tumor necrosis factor receptor (TNFR) superfamily.

Anaplastic lymphoma kinase (ALK) is a 220 kDa transmembrane glycoprotein that is primarily expressed in developing nervous system, and is also known as CD246.

CD30 is a membrane-bound glycoprotein with a molecular weight of 105 to 120 kD belonging to the TNF receptor superfamily, and is highly expressed on mononuclear Hodgkin cells and multinuclear Reed-Sternberg cells in Hodgkin lymphoma and tumor cells in anaplastic large cell lymphoma (ALCL)).

ST2(L) is a ST2 gene product, and includes secretion type (ST2) and transmembrane receptor type (ST2L). The ST2 gene has been cloned as a gene specifically expressed during the cell proliferation initiation process.

Notch1 is a member of the Notch family.

CD38 is also known as cyclic ADP-ribose hydrolase.

CD1a is a CD1 isoform. Four CD1 isoforms (CD1a, CD1b, CD1c, and CD1d) exist in humans. CD1a is a lipid-presenting molecule whose expression is essentially limited to those observed in cortical/thymic T cell acute lymphoblastic leukemia (cortical T-ALL) and Langerhans cell (LC) histiocytosis, and does not substantially exist in human tissues except for developing cortical thymocytes and LCs (Blood. 2019;133(21):2291-2304, of which entire description is specifically incorporated herein by reference).

CD47 is also known as integrin-associated protein (IAP).

IL-7Rα (CD127) is expressed mainly on thymocytes, dendritic cells, monocytes, mature T cells, etc. IL-7R is a heterodimer of the type I cytokine receptor, α chain (CD127), and the common γ chain (γc chain).

CD40L (CD40 ligand, CD154) is a ligand protein belonging to the TNF superfamily and expressed primarily on activated CD4⁺ T cells. The CD40/CD40L system is suggested to be involved in tumorigenesis.

CCR1, CCR4, CCR7, CCR8, CCR10, CXCR4, CXCR7, TIGIT, CADM1, and GPR15 are expressed on adult T cell leukemia (ATL) cells and therefore can be target antigens for ATL therapy (Leukemia & Lymphoma, 2006; 47(10): 2163-2173; the entire description of which is specifically incorporated herein by reference).

CXCR5, CXCL13, SLAM, ICOS, CD134, and CXCR3 are expressed on angioimmunoblastic T cell lymphoma (AITL) cells and therefore can be target antigens for AITL therapy (Blood. 2004;103:236-241; the entire description of which is specifically incorporated herein by reference ).

Anaplastic lymphoma kinases, CD30, ST2(L), and CCR5 are expressed on anaplastic large cell lymphoma cells and therefore can be target antigens for the treatment of peripheral T cell lymphoma (PTCL), including anaplastic large cell lymphoma (Blood. 2004;103:236-241).

Notchl, CXCR4, CD38, CD1a, CCR9 (CD199), CD47, IL-7Rα (CD127), and CD40L (CD154) are expressed on T cell acute lymphoblastic leukemia (TALL) cells and therefore can be target antigens for TALL therapy (see Blood. 2019;133(21):2291-2304 for CD1a; Blood 2021;138(12):1040-1052 for L-7Rα; Blood. 2022; 140(1): 25-37 for CCR9 (CD199); Blood 2022; 140(1): 45-57 for CD38 and CD47; and Blood Cancer Discov 2021;2:19-31 for Notchl, CXCR4, and CD38, the entire descriptions of these references are specifically incorporated herein by reference).

### (CCR4-binding portion)

The target tumor antigen for the bispecific antigen-binding molecule that constitutes the therapeutic agent as the first aspect of the present invention may be CCR4. Therefore, the bispecific antigen-binding molecule can contain a CCR4-binding portion and a TRBC1-binding portion or a CCR4-binding portion and a TRBC2-binding portion. The CCR4-binding portion may be the anti-CCR4 antibody, mogamulizumab, described in Japanese Patent No. 4052515 (the entire description of which is specifically incorporated herein by reference).

In one embodiment, the bispecific antigen-binding molecule is a bispecific antigen-binding molecule comprising at least one portion that specifically binds to CCR4, and the at least one portion comprises
a VH domain comprising
a heavy chain CDR1 comprising the amino acid sequence of SEQ ID NO: 22,
a heavy chain CDR2 comprising the amino acid sequence of SEQ ID NO: 23, and
a heavy chain CDR3 comprising the amino acid sequence of SEQ ID NO: 24, and
a VL domain comprising
a light chain CDR1 comprising the amino acid sequence of SEQ ID NO: 25,
a light chain CDR2 comprising the amino acid sequence of SEQ ID NO: 26, and
a light chain CDR3 comprising the amino acid sequence of SEQ ID NO: 27.

In a specific embodiment, the at least one portion that specifically binds to CCR4 comprises a heavy chain variable region VH that is at least 90% identical to the amino acid sequence of SEQ ID NO: 8 and a light chain variable region VL that is at least 90% identical to the amino acid sequence of SEQ ID NO: 9. In a further specific embodiment, the at least one portion that specifically binds to CCR4 comprises a heavy chain variable region VH that is at least 90%, 91%, 92%, 93%, 94%, 95%, 96%, 97%, 98% or 99% identical to the amino acid sequence of SEQ ID NO: 8 and a light chain variable region VL that is at least 90%, 91%, 92%, 93%, 94%, 95%, 96%, 97%, 98% or 99% identical to the amino acid sequence of SEQ ID NO: 9. The amino acid mutations (deletion, insertion, and substitution) in the amino acid sequence of SEQ ID NO: 8 or 9 can be present in a complementarity determining region (CDR) or framework region (FR).

In a specific embodiment, the at least one portion that specifically binds to CCR4 may be an scFv or Fab fragment.

In a specific embodiment, in the bispecific antigen-binding molecule that constitutes the therapeutic agent of the present invention,
the at least one portion that specifically binds to TRBC1 or TRBC2 expressed on normal T cells comprises
   (a) a VH domain comprising
      a heavy chain CDR1 comprising the amino acid sequence of SEQ ID NO: 10,
      a heavy chain CDR2 comprising the amino acid sequence of SEQ ID NO: 11, and
      a heavy chain CDR3 comprising the amino acid sequence of SEQ ID NO: 12, and
      a VL domain comprising
      a light chain CDR1 comprising the amino acid sequence of SEQ ID NO: 13,
      a light chain CDR2 comprising the amino acid sequence of SEQ ID NO: 14, and
      a light chain CDR3 comprising the amino acid sequence of SEQ ID NO: 15, or
   (b) a VH domain comprising
      a heavy chain CDR1 comprising the amino acid sequence of SEQ ID NO: 16,
      a heavy chain CDR2 comprising the amino acid sequence of SEQ ID NO: 17, and
      a heavy chain CDR3 comprising the amino acid sequence of SEQ ID NO: 18, and
      a VL domain comprising
      a light chain CDR1 comprising the amino acid sequence of SEQ ID NO: 19,
      a light chain CDR2 comprising the amino acid sequence of SEQ ID NO: 20, and
      a light chain CDR3 comprising the amino acid sequence of SEQ ID NO: 21, and
the at least one portion that specifically binds to a target tumor antigen expressed on T cell tumor cells comprises
   a VH domain comprising
   a heavy chain CDR1 comprising the amino acid sequence of SEQ ID NO: 22,
   a heavy chain CDR2 comprising the amino acid sequence of SEQ ID NO: 23, and
   a heavy chain CDR3 comprising the amino acid sequence of SEQ ID NO: 24, and
   a VL domain comprising
   a light chain CDR1 comprising the amino acid sequence of SEQ ID NO: 25,
   a light chain CDR2 comprising the amino acid sequence of SEQ ID NO: 26, and
   a light chain CDR3 comprising the amino acid sequence of SEQ ID NO: 27.

In a specific embodiment, in the bispecific antigen-binding molecule that constitutes the therapeutic agent of the present invention,
the at least one portion that specifically binds to TRBC1 or TRBC2 expressed on normal T cells comprises,
   (a) a heavy chain variable region VH that is at least 90% identical to the amino acid sequence of SEQ ID NO: 4 and a light chain variable region VL that is at least 90% identical to the amino acid sequence of SEQ ID NO: 5, or
   (b) a heavy chain variable region VH that is at least 90% identical to the amino acid sequence of SEQ ID NO: 6 and a light chain variable region VL that is at least 90% identical to the amino acid sequence of SEQ ID NO: 7, and
the at least one portion that specifically binds to a target tumor antigen expressed on T cell tumor cells comprises a heavy chain variable region VH that is at least 90% identical to the amino acid sequence of SEQ ID NO: 8 and a light chain variable region VL that is at least 90% identical to the amino acid sequence of SEQ ID NO: 9.

### (CD1a-binding portion)

The target tumor antigen for the bispecific antigen-binding molecule that constitutes the therapeutic agent as the first aspect of the present invention may be CD1a. Therefore, the bispecific antigen-binding molecule can contain a CD1a-binding portion and a TRBC1-binding portion or a CD1a-binding portion and a TRBC2-binding portion. The CD1a-binding portion may be the anti-CD1a antibody SC02-113 described in WO2005/063819 (the entire description of which is specifically incorporated herein by reference).

In one embodiment, the bispecific antigen-binding molecule is a bispecific antigen-binding molecule comprising at least one portion that specifically binds to CD1a, and the at least one portion comprises
a VH domain comprising
a heavy chain CDR1 comprising the amino acid sequence of SEQ ID NO: 34,
a heavy chain CDR2 comprising the amino acid sequence of SEQ ID NO: 35, and
a heavy chain CDR3 comprising the amino acid sequence of SEQ ID NO: 36, and
a VL domain comprising
a light chain CDR1 comprising the amino acid sequence of SEQ ID NO: 37,
a light chain CDR2 comprising the amino acid sequence of SEQ ID NO: 38, and
a light chain CDR3 comprising the amino acid sequence of SEQ ID NO: 39.

In a specific embodiment, the at least one portion that specifically binds to CD1a comprises a heavy chain variable region VH that is at least 90% identical to the amino acid sequence of SEQ ID NO: 40 and a light chain variable region VL that is at least 90% identical to the amino acid sequence of SEQ ID NO: 41. In a further specific embodiment, the at least one portion that specifically binds to CD1a comprises a heavy chain variable region VH that is at least 90%, 91%, 92%, 93%, 94%, 95%, 96%, 97%, 98% or 99% identical to the amino acid sequence of SEQ ID NO: 40, and a light chain variable region VL that is at least 90%, 91%, 92%, 93%, 94%, 95%, 96%, 97%, 98% or 99% identical to the amino acid sequence of SEQ ID NO: 41. The amino acid mutations (deletion, insertion, and substitution) in the amino acid sequence of SEQ ID NO: 40 or 41 can be present in a complementarity determining region (CDR) or framework region (FR).

In a specific embodiment, in the bispecific antigen-binding molecule that constitutes the therapeutic agent of the present invention,
the at least one portion that specifically binds to TRBC1 or TRBC2 expressed on normal T cells comprises
   (a) a VH domain comprising
      a heavy chain CDR1 comprising the amino acid sequence of SEQ ID NO: 10,
      a heavy chain CDR2 comprising the amino acid sequence of SEQ ID NO: 11, and
      a heavy chain CDR3 comprising the amino acid sequence of SEQ ID NO: 12, and
      a VL domain comprising
      a light chain CDR1 comprising the amino acid sequence of SEQ ID NO: 13,
      a light chain CDR2 comprising the amino acid sequence of SEQ ID NO: 14, and
      a light chain CDR3 comprising the amino acid sequence of SEQ ID NO: 15, or
   (b) a VH domain comprising
      a heavy chain CDR1 comprising the amino acid sequence of SEQ ID NO: 16,
      a heavy chain CDR2 comprising the amino acid sequence of SEQ ID NO: 17, and
      a heavy chain CDR3 comprising the amino acid sequence of SEQ ID NO: 18, and
      a VL domain comprising
      a light chain CDR1 comprising the amino acid sequence of SEQ ID NO: 19,
      a light chain CDR2 comprising the amino acid sequence of SEQ ID NO: 20, and
      a light chain CDR3 comprising the amino acid sequence of SEQ ID NO: 21, and
the at least one portion that specifically binds to a target tumor antigen expressed on T cell tumor cells comprises
   a VH domain comprising
   a heavy chain CDR1 comprising the amino acid sequence of SEQ ID NO: 34,
   a heavy chain CDR2 comprising the amino acid sequence of SEQ ID NO: 35, and
   a heavy chain CDR3 comprising the amino acid sequence of SEQ ID NO: 36, and
   a VL domain comprising
   a light chain CDR1 comprising the amino acid sequence of SEQ ID NO: 37,
   a light chain CDR2 comprising the amino acid sequence of SEQ ID NO: 38, and
   a light chain CDR3 comprising the amino acid sequence of SEQ ID NO: 39.

In a specific embodiment, in the bispecific antigen-binding molecule that constitutes the therapeutic agent of the present invention,
the at least one portion that specifically binds to TRBC1 or TRBC2 expressed on normal T cells is
   (a) at least one portion that specifically binds to TRBC1 expressed on normal T cells, comprising a heavy chain variable region VH that is at least 90% identical to the amino acid sequence of SEQ ID NO: 4 and a light chain variable region VL that is at least 90% identical to the amino acid sequence of SEQ ID NO: 5, or
   (b) at least one portion that specifically binds to TRBC2 expressed on normal T cells, comprising a heavy chain variable region VH that is at least 90% identical to the amino acid sequence of SEQ ID NO: 6 and a light chain variable region VL that is at least 90% identical to the amino acid sequence of SEQ ID NO: 7; and
the at least one portion that specifically binds to a target tumor antigen expressed on T cell tumor cells comprises a heavy chain variable region VH that is at least 90% identical to the amino acid sequence of SEQ ID NO: 41 and a light chain variable region VL that is at least 90% identical to the amino acid sequence of SEQ ID NO: 42.

### (CCR9-binding portion)

The target tumor antigen for the bispecific antigen-binding molecule that constitutes the therapeutic agent as the first aspect of the present invention may be CCR9. Therefore, the bispecific antigen-binding molecule can contain a CCR9-binding portion and a TRBC1-binding portion or a CCR9-binding portion and a TRBC2-binding portion. The CCR9-binding portion may be the anti-CCR9 antibody 9G7 described in WO2023/037125 (the entire description of which is specifically incorporated herein by reference).

In one embodiment, the bispecific antigen-binding molecule is a bispecific antigen-binding molecule comprising at least one portion that specifically binds to CCR9, and the at least one portion comprises
a VH domain comprising
a heavy chain CDR1 comprising the amino acid sequence of SEQ ID NO: 101,
a heavy chain CDR2 comprising the amino acid sequence of SEQ ID NO: 102, and
a heavy chain CDR3 comprising the amino acid sequence of SEQ ID NO: 103, and
a VL domain comprising
a light chain CDR1 comprising the amino acid sequence of SEQ ID NO: 104,
a light chain CDR2 comprising the amino acid sequence of SEQ ID NO: 105, and
a light chain CDR3 comprising the amino acid sequence of SEQ ID NO: 106.

In a specific embodiment, the at least one portion that specifically binds to CCR9 comprises a heavy chain variable region VH that is at least 90% identical to the amino acid sequence of SEQ ID NO: 131 and a light chain variable region VL that is at least 90% identical to the amino acid sequence of SEQ ID NO: 132. In a further specific embodiment, the at least one portion that specifically binds to CCR9 comprises a heavy chain variable region VH that is at least 90%, 91%, 92%, 93%, 94%, 95%, 96%, 97%, 98% or 99% identical to the amino acid sequence of SEQ ID NO: 131 and a light chain variable region VL that is at least 90%, 91%, 92%, 93%, 94%, 95%, 96%, 97%, 98% or 99% identical to the amino acid sequence of SEQ ID NO: 132. The amino acid mutations (deletion, insertion, and substitution) in the amino acid sequence of SEQ ID NO: 131 or 132 can be present in a complementarity determining region (CDR) or framework region (FR).

In a specific embodiment, in the bispecific antigen-binding molecule that constitutes the therapeutic agent of the present invention,
the at least one portion that specifically binds to TRBC1 or TRBC2 expressed on normal T cells comprises
   (a) a VH domain comprising
      a heavy chain CDR1 comprising the amino acid sequence of SEQ ID NO: 10,
      a heavy chain CDR2 comprising the amino acid sequence of SEQ ID NO: 11, and
      a heavy chain CDR3 comprising the amino acid sequence of SEQ ID NO: 12, and
      a VL domain comprising
      a light chain CDR1 comprising the amino acid sequence of SEQ ID NO: 13,
      a light chain CDR2 comprising the amino acid sequence of SEQ ID NO: 14, and
      a light chain CDR3 comprising the amino acid sequence of SEQ ID NO: 15, or
   (b) a VH domain comprising
      a heavy chain CDR1 comprising the amino acid sequence of SEQ ID NO: 16,
      a heavy chain CDR2 comprising the amino acid sequence of SEQ ID NO: 17, and
      a heavy chain CDR3 comprising the amino acid sequence of SEQ ID NO: 18, and
      a VL domain comprising
      a light chain CDR1 comprising the amino acid sequence of SEQ ID NO: 19,
      a light chain CDR2 comprising the amino acid sequence of SEQ ID NO: 20, and
      a light chain CDR3 comprising the amino acid sequence of SEQ ID NO: 21, and
the at least one portion that specifically binds to a target tumor antigen expressed on T cell tumor cells comprises
   a VH domain comprising
   a heavy chain CDR1 comprising the amino acid sequence of SEQ ID NO: 101,
   a heavy chain CDR2 comprising the amino acid sequence of SEQ ID NO: 102, and
   a heavy chain CDR3 comprising the amino acid sequence of SEQ ID NO: 103, and
   a VL domain comprising
   a light chain CDR1 comprising the amino acid sequence of SEQ ID NO: 104,
   a light chain CDR2 comprising the amino acid sequence of SEQ ID NO: 105, and
   a light chain CDR3 comprising the amino acid sequence of SEQ ID NO: 106.

In a specific embodiment, in the bispecific antigen-binding molecule that constitutes the therapeutic agent of the present invention,
the at least one portion that specifically binds to TRBC1 or TRBC2 expressed on normal T cells is
   (a) at least one portion that specifically binds to TRBC1 expressed on normal T cells, comprising a heavy chain variable region VH that is at least 90% identical to the amino acid sequence of SEQ ID NO: 4 and a light chain variable region VL that is at least 90% identical to the amino acid sequence of SEQ ID NO: 5, or
   (b) at least one portion that specifically binds to TRBC2 expressed on normal T cells, comprising a heavy chain variable region VH that is at least 90% identical to the amino acid sequence of SEQ ID NO: 6 and a light chain variable region VL that is at least 90% identical to the amino acid sequence of SEQ ID NO: 7; and
the at least one portion that specifically binds to a target tumor antigen expressed on T cell tumor cells comprises a heavy chain variable region VH that is at least 90% identical to the amino acid sequence of SEQ ID NO: 131 and a light chain variable region VL that is at least 90% identical to the amino acid sequence of SEQ ID NO: 132.

### (CXCR4-binding portion)

The target tumor antigen for the bispecific antigen-binding molecule that constitutes the therapeutic agent as the first aspect of the present invention may be CXCR4. Therefore, the bispecific antigen-binding molecule can contain a CXCR4-binding portion and a TRBC1-binding portion or a CXCR4-binding portion and a TRBC2-binding portion. The CXCR4-binding portion may be the anti-CXCR4 antibody (hz515H7 VH1 D76N-VL2) described in WO2010/125162 (the entire description of which is specifically incorporated herein by reference).

In one embodiment, the bispecific antigen-binding molecule is a bispecific antigen-binding molecule comprising at least one portion that specifically binds to CXCR4, and the at least one portion comprises
a VH domain comprising
a heavy chain CDR1 comprising the amino acid sequence of SEQ ID NO: 107,
a heavy chain CDR2 comprising the amino acid sequence of SEQ ID NO: 108, and
a heavy chain CDR3 comprising the amino acid sequence of SEQ ID NO: 109, and
a VL domain comprising
a light chain CDR1 comprising the amino acid sequence of SEQ ID NO: 110,
a light chain CDR2 comprising the amino acid sequence of SEQ ID NO: 111, and
a light chain CDR3 comprising the amino acid sequence of SEQ ID NO: 112.

In a specific embodiment, the at least one portion that specifically binds to CXCR4 comprises a heavy chain variable region VH that is at least 90% identical to the amino acid sequence of SEQ ID NO: 133 and a light chain variable region VL that is at least 90% identical to the amino acid sequence of SEQ ID NO: 134. In a further specific embodiment, the at least one portion that specifically binds to CXCR4 comprises a heavy chain variable region VH that is at least 90%, 91%, 92%, 93%, 94%, 95%, 96%, 97%, 98% or 99% identical to the amino acid sequence of SEQ ID NO: 133 and a light chain variable region VL that is at least 90%, 91%, 92%, 93%, 94%, 95%, 96%, 97%, 98% or 99% identical to the amino acid sequence of SEQ ID NO: 134. The amino acid mutations (deletion, insertion, and substitution) in the amino acid sequence of SEQ ID NO: 133 or 134 can be present in a complementarity determining region (CDR) or framework region (FR).

In a specific embodiment, in the bispecific antigen-binding molecule that constitutes the therapeutic agent of the present invention,
the at least one portion that specifically binds to TRBC1 or TRBC2 expressed on normal T cells comprises
   (a) a VH domain comprising
      a heavy chain CDR1 comprising the amino acid sequence of SEQ ID NO: 10,
      a heavy chain CDR2 comprising the amino acid sequence of SEQ ID NO: 11, and
      a heavy chain CDR3 comprising the amino acid sequence of SEQ ID NO: 12, and
      a VL domain comprising
      a light chain CDR1 comprising the amino acid sequence of SEQ ID NO: 13,
      a light chain CDR2 comprising the amino acid sequence of SEQ ID NO: 14, and
      a light chain CDR3 comprising the amino acid sequence of SEQ ID NO: 15, or
   (b) a VH domain comprising
      a heavy chain CDR1 comprising the amino acid sequence of SEQ ID NO: 16,
      a heavy chain CDR2 comprising the amino acid sequence of SEQ ID NO: 17, and
      a heavy chain CDR3 comprising the amino acid sequence of SEQ ID NO: 18, and
      a VL domain comprising
      a light chain CDR1 comprising the amino acid sequence of SEQ ID NO: 19,
      a light chain CDR2 comprising the amino acid sequence of SEQ ID NO: 20, and
      a light chain CDR3 comprising the amino acid sequence of SEQ ID NO: 21, and
the at least one portion that specifically binds to a target tumor antigen expressed on T cell tumor cells comprises
   a VH domain comprising
   a heavy chain CDR1 comprising the amino acid sequence of SEQ ID NO: 107,
   a heavy chain CDR2 comprising the amino acid sequence of SEQ ID NO: 108, and
   a heavy chain CDR3 comprising the amino acid sequence of SEQ ID NO: 109, and
   a VL domain comprising
   a light chain CDR1 comprising the amino acid sequence of SEQ ID NO: 110,
   a light chain CDR2 comprising the amino acid sequence of SEQ ID NO: 111, and
   a light chain CDR3 comprising the amino acid sequence of SEQ ID NO: 112.

In a specific embodiment, in the bispecific antigen-binding molecule that constitutes the therapeutic agent of the present invention,
the at least one portion that specifically binds to TRBC1 or TRBC2 expressed on normal T cells is
   (a) at least one portion that specifically binds to TRBC1 expressed on normal T cells, comprising a heavy chain variable region VH that is at least 90% identical to the amino acid sequence of SEQ ID NO: 4 and a light chain variable region VL that is at least 90% identical to the amino acid sequence of SEQ ID NO: 5, or
   (b) at least one portion that specifically binds to TRBC2 expressed on normal T cells, comprising a heavy chain variable region VH that is at least 90% identical to the amino acid sequence of SEQ ID NO: 6 and a light chain variable region VL that is at least 90% identical to the amino acid sequence of SEQ ID NO: 7; and
the at least one portion that specifically binds to a target tumor antigen expressed on T cell tumor cells comprises a heavy chain variable region VH that is at least 90% identical to the amino acid sequence of SEQ ID NO: 133 and a light chain variable region VL that is at least 90% identical to the amino acid sequence of SEQ ID NO: 134.

### (TIGIT-binding portion)

The target tumor antigen for the bispecific antigen-binding molecule that constitutes the therapeutic agent as the first aspect of the present invention may be TIGIT. Therefore, the bispecific antigen-binding molecule can contain a TIGIT-binding portion and a TRBC1-binding portion or a TIGIT-binding portion and a TRBC2-binding portion. The TIGIT-binding portion may be the anti TIGIT antibody vibostolimab described in WO2016/028656 (the entire description of which is specifically incorporated herein by reference).

In one embodiment, the bispecific antigen-binding molecule is a bispecific antigen-binding molecule comprising at least one portion that specifically binds to TIGIT, and the at least one portion comprises
a VH domain comprising
a heavy chain CDR1 comprising the amino acid sequence of SEQ ID NO: 89,
a heavy chain CDR2 comprising the amino acid sequence of SEQ ID NO: 90, and
a heavy chain CDR3 comprising the amino acid sequence of SEQ ID NO: 91, and
a VL domain comprising
a light chain CDR1 comprising the amino acid sequence of SEQ ID NO: 92,
a light chain CDR2 comprising the amino acid sequence of SEQ ID NO: 93, and
a light chain CDR3 comprising the amino acid sequence of SEQ ID NO: 94.

In a specific embodiment, the at least one portion that specifically binds to TIGIT comprises a heavy chain variable region VH that is at least 90% identical to the amino acid sequence of SEQ ID NO: 135 and a light chain variable region VL that is at least 90% identical to the amino acid sequence of SEQ ID NO: 136. In a further specific embodiment, the at least one portion that specifically binds to TIGIT comprises a heavy chain variable region VH that is at least 90%, 91%, 92%, 93%, 94%, 95%, 96%, 97%, 98% or 99% identical to the amino acid sequence of SEQ ID NO: 135 and a light chain variable region VL that is at least 90%, 91%, 92%, 93%, 94%, 95%, 96%, 97%, 98% or 99% identical to the amino acid sequence of SEQ ID NO: 136. The amino acid mutations (deletion, insertion, and substitution) in the amino acid sequence of SEQ ID NO: 135 or 136 can be present in a complementarity determining region (CDR) or framework region (FR).

In a specific embodiment, in the bispecific antigen-binding molecule that constitutes the therapeutic agent of the present invention,
the at least one portion that specifically binds to TRBC1 or TRBC2 expressed on normal T cells comprises
   (a) a VH domain comprising
      a heavy chain CDR1 comprising the amino acid sequence of SEQ ID NO: 10,
      a heavy chain CDR2 comprising the amino acid sequence of SEQ ID NO: 11, and
      a heavy chain CDR3 comprising the amino acid sequence of SEQ ID NO: 12, and
      a VL domain comprising
      a light chain CDR1 comprising the amino acid sequence of SEQ ID NO: 13,
      a light chain CDR2 comprising the amino acid sequence of SEQ ID NO: 14, and
      a light chain CDR3 comprising the amino acid sequence of SEQ ID NO: 15, or
   (b) a VH domain comprising
      a heavy chain CDR1 comprising the amino acid sequence of SEQ ID NO: 16,
      a heavy chain CDR2 comprising the amino acid sequence of SEQ ID NO: 17, and
      a heavy chain CDR3 comprising the amino acid sequence of SEQ ID NO: 18, and
      a VL domain comprising
      a light chain CDR1 comprising the amino acid sequence of SEQ ID NO: 19,
      a light chain CDR2 comprising the amino acid sequence of SEQ ID NO: 20, and
      a light chain CDR3 comprising the amino acid sequence of SEQ ID NO: 21, and
the at least one portion that specifically binds to a target tumor antigen expressed on T cell tumor cells comprises
   a VH domain comprising
   a heavy chain CDR1 comprising the amino acid sequence of SEQ ID NO: 89,
   a heavy chain CDR2 comprising the amino acid sequence of SEQ ID NO: 90, and
   a heavy chain CDR3 comprising the amino acid sequence of SEQ ID NO: 91, and
   a VL domain comprising
   a light chain CDR1 comprising the amino acid sequence of SEQ ID NO: 92,
   a light chain CDR2 comprising the amino acid sequence of SEQ ID NO: 93, and
   a light chain CDR3 comprising the amino acid sequence of SEQ ID NO: 94.

In a specific embodiment, in the bispecific antigen-binding molecule that constitutes the therapeutic agent of the present invention,
the at least one portion that specifically binds to TRBC1 or TRBC2 expressed on normal T cells is
   (a) at least one portion that specifically binds to TRBC1 expressed on normal T cells, comprising a heavy chain variable region VH that is at least 90% identical to the amino acid sequence of SEQ ID NO: 4 and a light chain variable region VL that is at least 90% identical to the amino acid sequence of SEQ ID NO: 5, or
   (b) at least one portion that specifically binds to TRBC2 expressed on normal T cells, comprising a heavy chain variable region VH that is at least 90% identical to the amino acid sequence of SEQ ID NO: 6 and a light chain variable region VL that is at least 90% identical to the amino acid sequence of SEQ ID NO: 7; and
the at least one portion that specifically binds to a target tumor antigen expressed on T cell tumor cells comprises a heavy chain variable region VH that is at least 90% identical to the amino acid sequence of SEQ ID NO: 135 and a light chain variable region VL that is at least 90% identical to the amino acid sequence of SEQ ID NO: 136.

### (CCR8-binding portion)

The target tumor antigen for the bispecific antigen-binding molecule that constitutes the therapeutic agent as the first aspect of the present invention may be CCR8. Therefore, the bispecific antigen-binding molecule can contain a CCR8-binding portion and a TRBC1-binding portion or a CCR8-binding portion and a TRBC2-binding portion. The CCR8-binding portion may be the anti-CCR8 antibody ABBV-514 described in WO2023/010054 (the entire description of which is specifically incorporated herein by reference).

In one embodiment, the bispecific antigen-binding molecule is a bispecific antigen-binding molecule comprising at least one portion that specifically binds to CCR8, and the at least one portion comprises
a VH domain comprising
a heavy chain CDR1 comprising the amino acid sequence of SEQ ID NO: 95,
a heavy chain CDR2 comprising the amino acid sequence of SEQ ID NO: 96, and
a heavy chain CDR3 comprising the amino acid sequence of SEQ ID NO: 97, and
a VL domain comprising
a light chain CDR1 comprising the amino acid sequence of SEQ ID NO: 98,
a light chain CDR2 comprising the amino acid sequence of SEQ ID NO: 99, and
a light chain CDR3 comprising the amino acid sequence of SEQ ID NO: 100.

In a specific embodiment, the at least one portion that specifically binds to CCR8 comprises a heavy chain variable region VH that is at least 90% identical to the amino acid sequence of SEQ ID NO: 137 and a light chain variable region VL that is at least 90% identical to the amino acid sequence of SEQ ID NO: 138. In a further specific embodiment, the at least one portion that specifically binds to CCR8 comprises a heavy chain variable region VH that is at least 90%, 91%, 92%, 93%, 94%, 95%, 96%, 97%, 98% or 99% identical to the amino acid sequence of SEQ ID NO: 137 and a light chain variable region VL that is at least 90%, 91%, 93%, 94%, 95%, 96%, 97%, 98% or 99% identical to the amino acid sequence of SEQ ID NO: 138. The amino acid mutations (deletion, insertion, and substitution) in the amino acid sequence of SEQ ID NO: 137 or 138 can be present in a complementarity determining region (CDR) or framework region (FR).

In a specific embodiment, in the bispecific antigen-binding molecule that constitutes the therapeutic agent of the present invention,
the at least one portion that specifically binds to TRBC1 or TRBC2 expressed on normal T cells comprises
   (a) a VH domain comprising
      a heavy chain CDR1 comprising the amino acid sequence of SEQ ID NO: 10,
      a heavy chain CDR2 comprising the amino acid sequence of SEQ ID NO: 11, and
      a heavy chain CDR3 comprising the amino acid sequence of SEQ ID NO: 12, and
      a VL domain comprising
      a light chain CDR1 comprising the amino acid sequence of SEQ ID NO: 13,
      a light chain CDR2 comprising the amino acid sequence of SEQ ID NO: 14, and
      a light chain CDR3 comprising the amino acid sequence of SEQ ID NO: 15, or
   (b) a VH domain comprising
      a heavy chain CDR1 comprising the amino acid sequence of SEQ ID NO: 16,
      a heavy chain CDR2 comprising the amino acid sequence of SEQ ID NO: 17, and
      a heavy chain CDR3 comprising the amino acid sequence of SEQ ID NO: 18, and
      a VL domain comprising
      a light chain CDR1 comprising the amino acid sequence of SEQ ID NO: 19,
      a light chain CDR2 comprising the amino acid sequence of SEQ ID NO: 20, and
      a light chain CDR3 comprising the amino acid sequence of SEQ ID NO: 21, and
   the at least one portion that specifically binds to a target tumor antigen expressed on T cell tumor cells comprises
   a VH domain comprising
   a heavy chain CDR1 comprising the amino acid sequence of SEQ ID NO: 95,
   a heavy chain CDR2 comprising the amino acid sequence of SEQ ID NO: 96, and
   a heavy chain CDR3 comprising the amino acid sequence of SEQ ID NO: 97, and
   a VL domain comprising
   a light chain CDR1 comprising the amino acid sequence of SEQ ID NO: 98,
   a light chain CDR2 comprising the amino acid sequence of SEQ ID NO: 99, and
   a light chain CDR3 comprising the amino acid sequence of SEQ ID NO: 100.

In a specific embodiment, in the bispecific antigen-binding molecule that constitutes the therapeutic agent of the present invention,
the at least one portion that specifically binds to TRBC1 or TRBC2 expressed on normal T cells is
   (a) at least one portion that specifically binds to TRBC1 expressed on normal T cells, comprising a heavy chain variable region VH that is at least 90% identical to the amino acid sequence of SEQ ID NO: 4 and a light chain variable region VL that is at least 90% identical to the amino acid sequence of SEQ ID NO: 5, or
   (b) at least one portion that specifically binds to TRBC2 expressed on normal T cells, comprising a heavy chain variable region VH that is at least 90% identical to the amino acid sequence of SEQ ID NO: 6 and a light chain variable region VL that is at least 90% identical to the amino acid sequence of SEQ ID NO: 7; and
the at least one portion that specifically binds to a target tumor antigen expressed on T cell tumor cells comprises a heavy chain variable region VH that is at least 90% identical to the amino acid sequence of SEQ ID NO: 137 and a light chain variable region VL that is at least 90% identical to the amino acid sequence of SEQ ID NO: 138.

### (CD30-binding portion)

The target tumor antigen for the bispecific antigen-binding molecule that constitutes the therapeutic agent as the first aspect of the present invention may be CD30. Therefore, the bispecific antigen-binding molecule can contain a CD30-binding portion and a TRBC1-binding portion or a CD30-binding portion and a TRBC2-binding portion. The CD30-binding portion may be the anti-CD30 antibody AC10 described in Japanese Patent No. 4303964 (the entire description of which is specifically incorporated herein by reference).

In one embodiment, the bispecific antigen-binding molecule is a bispecific antigen-binding molecule comprising at least one portion that specifically binds to CD30, and the at least one portion comprises
a VH domain comprising
a heavy chain CDR1 comprising the amino acid sequence of SEQ ID NO: 113,
a heavy chain CDR2 comprising the amino acid sequence of SEQ ID NO: 114, and
a heavy chain CDR3 comprising the amino acid sequence of SEQ ID NO: 115, and
a VL domain comprising
a light chain CDR1 comprising the amino acid sequence of SEQ ID NO: 116,
a light chain CDR2 comprising the amino acid sequence of SEQ ID NO: 117, and
a light chain CDR3 comprising the amino acid sequence of SEQ ID NO: 118.

In a specific embodiment, the at least one portion that specifically binds to CD30 comprises a heavy chain variable region VH that is at least 90% identical to the amino acid sequence of SEQ ID NO: 139 and a light chain variable region VL that is at least 90% identical to the amino acid sequence of SEQ ID NO: No. 140. In a further specific embodiment, the at least one portion that specifically binds to CD30 comprises a heavy chain variable region VH that is at least 90%, 91%, 92%, 93%, 94%, 95%, 96%, 97%, 98% or 99% identical to the amino acid sequence of SEQ ID NO: 139 and a light chain variable region VL that is at least 90%, 91%, 92%, 93%, 94%, 95%, 96%, 97%, 98% or 99% identical to the amino acid sequence of SEQ ID NO: No. 140. The amino acid mutations (deletion, insertion, and substitution) in the amino acid sequence of SEQ ID NO: 139 or 140 can be present in a complementarity determining region (CDR) or framework region (FR).

In a specific embodiment, in the bispecific antigen-binding molecule that constitutes the therapeutic agent of the present invention,
the at least one portion that specifically binds to TRBC1 or TRBC2 expressed on normal T cells comprises
   (a) a VH domain comprising
      a heavy chain CDR1 comprising the amino acid sequence of SEQ ID NO: 10,
      a heavy chain CDR2 comprising the amino acid sequence of SEQ ID NO: 11, and
      a heavy chain CDR3 comprising the amino acid sequence of SEQ ID NO: 12, and
      a VL domain comprising
      a light chain CDR1 comprising the amino acid sequence of SEQ ID NO: 13,
      a light chain CDR2 comprising the amino acid sequence of SEQ ID NO: 14, and
      a light chain CDR3 comprising the amino acid sequence of SEQ ID NO: 15, or
   (b) a VH domain comprising
      a heavy chain CDR1 comprising the amino acid sequence of SEQ ID NO: 16,
      a heavy chain CDR2 comprising the amino acid sequence of SEQ ID NO: 17, and
      a heavy chain CDR3 comprising the amino acid sequence of SEQ ID NO: 18, and
      a VL domain comprising
      a light chain CDR1 comprising the amino acid sequence of SEQ ID NO: 19,
      a light chain CDR2 comprising the amino acid sequence of SEQ ID NO: 20, and
      a light chain CDR3 comprising the amino acid sequence of SEQ ID NO: 21, and
the at least one portion that specifically binds to a target tumor antigen expressed on T cell tumor cells comprises
   a VH domain comprising
   a heavy chain CDR1 comprising the amino acid sequence of SEQ ID NO: 113,
   a heavy chain CDR2 comprising the amino acid sequence of SEQ ID NO: 114, and
   a heavy chain CDR3 comprising the amino acid sequence of SEQ ID NO: 115, and
   a VL domain comprising
   a light chain CDR1 comprising the amino acid sequence of SEQ ID NO: 116,
   a light chain CDR2 comprising the amino acid sequence of SEQ ID NO: 117, and
   a light chain CDR3 comprising the amino acid sequence of SEQ ID NO: 118.

In a specific embodiment, in the bispecific antigen-binding molecule that constitutes the therapeutic agent of the present invention,
the at least one portion that specifically binds to TRBC1 or TRBC2 expressed on normal T cells is
   (a) at least one portion that specifically binds to TRBC1 expressed on normal T cells, comprising a heavy chain variable region VH that is at least 90% identical to the amino acid sequence of SEQ ID NO: 4 and a light chain variable region VL that is at least 90% identical to the amino acid sequence of SEQ ID NO: 5, or
   (b) at least one portion that specifically binds to TRBC2 expressed on normal T cells, comprising a heavy chain variable region VH that is at least 90% identical to the amino acid sequence of SEQ ID NO: 6 and a light chain variable region VL that is at least 90% identical to the amino acid sequence of SEQ ID NO: 7; and
the at least one portion that specifically binds to a target tumor antigen expressed on T cell tumor cells comprises a heavy chain variable region VH that is at least 90% identical to the amino acid sequence of SEQ ID NO: 139 and a light chain variable region VL that is at least 90% identical to the amino acid sequence of SEQ ID NO: 140.

### (CD40L-binding portion)

The target tumor antigen for the bispecific antigen-binding molecule that constitutes the therapeutic agent as the first aspect of the present invention may be CD40L (CD154). Therefore, the bispecific antigen-binding molecule can contain a CD40L-binding portion and a TRBC1-binding portion or a CD40L-binding portion and a TRBC2-binding portion. The CD40L-binding portion may be the anti-CD40L (CD154) antibody ABI793 described in the Japanese Patent Publication (Kohyo) No. 2003-526371 (WO01/068860, of which entire description is specifically incorporated herein by reference).

In one embodiment, the bispecific antigen-binding molecule is a bispecific antigen-binding molecule comprising at least one portion that specifically binds to CD40L (CD154), and the at least one portion comprises
a VH domain comprising
a heavy chain CDR1 comprising the amino acid sequence of SEQ ID NO: 119,
a heavy chain CDR2 comprising the amino acid sequence of SEQ ID NO: 120, and
a heavy chain CDR3 comprising the amino acid sequence of SEQ ID NO: 121, and
a VL domain comprising
a light chain CDR1 comprising the amino acid sequence of SEQ ID NO: 122,
a light chain CDR2 comprising the amino acid sequence of SEQ ID NO: 123, and
a light chain CDR3 comprising the amino acid sequence of SEQ ID NO: 124.

In a specific embodiment, the at least one portion that specifically binds to CD40L (CD154) comprises a heavy chain variable region VH that is at least 90% identical to the amino acid sequence of SEQ ID NO: 141 and a light chain variable region VL that is at least 90% identical to the amino acid sequence of SEQ ID NO: 142. In a further specific embodiment, the at least one portion that specifically binds to CD40L (CD154) comprises a heavy chain variable region VH that is at least 90%, 91%, 92%, 93%, 94%, 95%, 96%, 97%, 98% or 99% identical to the amino acid sequence of SEQ ID NO: 141 and a light chain variable region VL that is at least 90%, 91%, 92%, 93%, 94%, 95%, 96%, 97%, 98% or 99% identical to the amino acid sequence of SEQ ID NO: 142. The amino acid mutations (deletion, insertion, and substitution) in the amino acid sequence of SEQ ID NO: 141 or 142 can be present in a complementarity determining region (CDR) or framework region (FR).

In a specific embodiment, in the bispecific antigen-binding molecule that constitutes the therapeutic agent of the present invention,
the at least one portion that specifically binds to TRBC1 or TRBC2 expressed on normal T cells comprises
   (a) a VH domain comprising
      a heavy chain CDR1 comprising the amino acid sequence of SEQ ID NO: 10,
      a heavy chain CDR2 comprising the amino acid sequence of SEQ ID NO: 11, and
      a heavy chain CDR3 comprising the amino acid sequence of SEQ ID NO: 12, and
      a VL domain comprising
      a light chain CDR1 comprising the amino acid sequence of SEQ ID NO: 13,
      a light chain CDR2 comprising the amino acid sequence of SEQ ID NO: 14, and
      a light chain CDR3 comprising the amino acid sequence of SEQ ID NO: 15, or
   (b) a VH domain comprising
      a heavy chain CDR1 comprising the amino acid sequence of SEQ ID NO: 16,
      a heavy chain CDR2 comprising the amino acid sequence of SEQ ID NO: 17, and
      a heavy chain CDR3 comprising the amino acid sequence of SEQ ID NO: 18, and
      a VL domain comprising
      a light chain CDR1 comprising the amino acid sequence of SEQ ID NO: 19,
      a light chain CDR2 comprising the amino acid sequence of SEQ ID NO: 20, and
      a light chain CDR3 comprising the amino acid sequence of SEQ ID NO: 21, and
the at least one portion that specifically binds to a target tumor antigen expressed on T cell tumor cells comprises
   a VH domain comprising
   a heavy chain CDR1 comprising the amino acid sequence of SEQ ID NO: 119,
   a heavy chain CDR2 comprising the amino acid sequence of SEQ ID NO: 120, and
   a heavy chain CDR3 comprising the amino acid sequence of SEQ ID NO: 121, and
   a VL domain comprising
   a light chain CDR1 comprising the amino acid sequence of SEQ ID NO: 122,
   a light chain CDR2 comprising the amino acid sequence of SEQ ID NO: 123, and
   a light chain CDR3 comprising the amino acid sequence of SEQ ID NO: 124.

In a specific embodiment, in the bispecific antigen-binding molecule that constitutes the therapeutic agent of the present invention,
the at least one portion that specifically binds to TRBC1 or TRBC2 expressed on normal T cells is
   (a) at least one portion that specifically binds to TRBC1 expressed on normal T cells, comprising a heavy chain variable region VH that is at least 90% identical to the amino acid sequence of SEQ ID NO: 4 and a light chain variable region VL that is at least 90% identical to the amino acid sequence of SEQ ID NO: 5, or
   (b) at least one portion that specifically binds to TRBC2 expressed on normal T cells, comprising a heavy chain variable region VH that is at least 90% identical to the amino acid sequence of SEQ ID NO: 6 and a light chain variable region VL that is at least 90% identical to the amino acid sequence of SEQ ID NO: 7; and
the at least one portion that specifically binds to a target tumor antigen expressed on T cell tumor cells comprises a heavy chain variable region VH that is at least 90% identical to the amino acid sequence of SEQ ID NO: 141 and a light chain variable region VL that is at least 90% identical to the amino acid sequence of SEQ ID NO: 142.

In a further specific embodiment, an amino acid sequence variant of the bispecific antigen-binding molecule comprising the tumor antigen-binding portion is prepared to improve the binding affinity and/or other biological properties of the tumor antigen-binding portion. Such an amino acid sequence variant can be prepared by, for example, introducing an appropriate mutation (deletion, insertion, and substitution) into the nucleotide sequence encoding the antibody or by peptide synthesis. Mutations can be introduced into a complementarity determining region (CDR) or framework region (FR) of the antibody, depending on the property to be improved. Amino acid sequence variants are screened for maintenance or improvement of binding affinity, maintenance or improvement of antigen specificity, and reduction of immunogenicity.

The CDR sequences of the tumor antigen-binding portions are listed in the following table.

**[Table 2]**

| Target of antigen-binding portion | Region | Sequence | SEQ ID No: |
|---|---|---|---|
| CCR4 | CDR H1 | NYGMS | 22 |
| | CDR H2 | TISSASTYSYYPDSVKG | 23 |
| | CDR H3 | HSDGNFAFGY | 24 |
| | CDR L1 | RSSRNIVHINGDTYLE | 25 |
| | CDR L2 | KVSNRFS | 26 |
| | CDR L3 | FQGSLLPWT | 27 |
| CD1a | CDR H1 | GYYW | 34 |
| | CDR H2 | YIYYSGSTNYNPSLKS | 35 |
| | CDR H3 | APYMMYFDS | 36 |
| | CDR L1 | RASQSISSYLN | 37 |
| | CDR L2 | AASSLQS | 38 |
| | CDR L3 | QQSYSTPPT | 39 |
| TIGIT | CDR H1 | SYVMH | 89 |
| | CDR H2 | YIDPYNDGAKYAQKFQG | 90 |
| | CDR H3 | GGPYGWYFDV | 91 |
| | CDR L1 | RASEHIYSYLS | 92 |
| | CDR L2 | NAKTLAE | 93 |
| | CDR L3 | QHHFGSPLT | 94 |
| CCR8 | CDR H1 | NAVMY | 95 |
| | CDR H2 | RIKTKFNNYATYYADAVKG | 96 |
| | CDR H3 | GDRNKPFAY | 97 |
| | CDR L1 | RASTSVITLLH | 98 |
| | CDR L2 | GASNLES | 99 |
| | CDR L3 | QQSWNDPYT | 100 |
| CCR9 | CDR H1 | SYDMH | 101 |
| | CDR H2 | IIWANGNTHYNSGLKS | 102 |
| | CDR H3 | GGFAY | 103 |
| | CDR L1 | RSSQSLVHNNGNTYLS | 104 |
| | CDR L2 | KVSNRFS | 105 |
| | CDR L3 | GQGTQYPT | 106 |
| CXCR4 | CDR H1 | DNYMS | 107 |
| | CDR H2 | FIRNKANGYTTEYAASVKG | 108 |
| | CDR H3 | DVGSNYFDY | 109 |
| | CDR L1 | KSSQSLFNSRTRKNYLA | 110 |
| | CDR L2 | WASARDS | 111 |
| | CDR L3 | MQSFNLRT | 112 |
| CD30 | CDR H1 | DYYIT | 113 |
| | CDR H2 | WIYPGSGNTKYNEKFKG | 114 |
| | CDR H3 | YGNYWFAY | 115 |
| | CDR L1 | KASQSVDFDGDSYMN | 116 |
| | CDR L2 | AASNLES | 117 |
| | CDR L3 | QQSNEDPWT | 118 |
| CD40L | CDR H1 | NFAFN | 119 |
| | CDR H2 | RILPSLDIASYAQKFQD | 120 |
| | CDR H3 | EVDGGGFDY | 121 |
| | CDR L1 | RASQGISSWLA | 122 |
| | CDR L2 | AASSLQS | 123 |
| | CDR L3 | QQYNSYPFT | 124 |

### (Format)

In a specific embodiment, in the bispecific antigen-binding molecule that constitutes the therapeutic agent of the present invention, the at least one portion that specifically binds to an antigen having subtypes expressed on normal T cells is scFv, the at least one portion that specifically binds to a target tumor antigen expressed on T cell tumor cells is a Fab fragment, and the scFv is linked to the C-terminus of the heavy chain of the Fab fragment via a peptide linker. In a further specific embodiment, in the bispecific antigen-binding molecule, the at least one portion that specifically binds to an antigen having subtypes expressed on normal T cells is scFv, the at least one portion that specifically binds to a target tumor antigen expressed on T cell tumor cells is scFv, and the two scFVs are linked via a peptide linker.

The "peptide linker" is a peptide comprising about 2 to 20 amino acids, and is, for example, a peptide linker represented as (G₄S)ₙ, (SG₄)ₙ or G₄(SG₄)ₙ, where "n" is generally an integer of 1 to 10. Typically, the peptide linker is selected from the group consisting of GGGGS (SEQ ID NO: 51), GGGGSGGGGS (SEQ ID NO: 52), GGGGSGGGGSGGGGS (SEQ ID NO: 64), SGGGGSGGGG (SEQ ID NO: 53) and GGGGSGGGGSGGGG (SEQ ID NO: 54), while sequences of GSPGSSSSGS (SEQ ID NO: 55), (G₄S)₄, GSGSGSGS (SEQ ID NO: 56), GSGSGNGS (SEQ ID NO: 57), GGSGSGSG (SEQ ID NO: 58), GGSGSG (SEQ ID NO: 59), GGSG (SEQ ID NO: 60), GGSGNGSG (SEQ ID NO: 61), GGNGSGSG (SEQ ID NO: 62) and GGNGSG (SEQ ID NO: 63) may also be used.

In a specific embodiment, the bispecific antigen-binding molecule that constitutes the therapeutic agent of the present invention further comprises an Fc domain. In a further specific embodiment, the Fc domain is the Fc domain of IgG1, IgG2, IgG 3 or IgG4, and the Fc domain is preferably the IgG1 Fc domain. In a further specific embodiment, the polypeptide of the IgG1 Fc domain can have one or more amino acid mutations, and the polypeptide preferably has, in particular, an amino acid mutation that alters the binding ability to the Fc receptor, more preferably an amino acid mutation that reduces the binding ability to the Fc receptor.

In a specific embodiment, the Fc domain can be a human IgG1 isotype and have L234AL235A mutation (LALA mutation), which is replacement of leucines at positions 234 and 235 with alanines, or corresponding mutations. Such a mutation can take place at equivalent positions in other isotypes and subtypes. The LALA mutation disables binding to the complement component (C1q) and the Fc gamma receptor (FcgR), and therefore prevents *in vitro* FcgR-mediated co-activation of innate immune effector cells including natural killer (NK) cells, monocytes/macrophages, and neutrophils without altering functional binding to FcRn (fetal Fc receptor) (Clin Cancer Res (2016) 22 (13): 3286-3297, the entire description of which is incorporated herein by reference). Therefore, the bispecific antigen-binding molecule having an Fc domain bearing the LALA mutation lacks complement-dependent cytotoxicity (CDC) and antibody-dependent cytotoxicity (ADCC), and the only immune effector cells involved may be T cells.

In a specific embodiment, the format of the bispecific antigen-binding molecule may be any one of the structures/formats described in ULRICH H. WEIDLE et al, "The Intriguing Options of Multispecific Antibody Formats for the Treatment of Cancer" Cancer Genomics & Proteomics January 2013,10(1)1-18 (of which entire description is specifically incorporated herein by reference). Examples of such structures/formats include, for example, Cross-Mab, IgG-dssFv2, DVD, IgG-dsFv, IgG-scFab, scFab-dssFv, Fv2-Fc, Fab-scFv2, Fab-scFv, Fab-scFv-Fc, BiTE (scFv-scFv), diabody, DART, and so forth, but are not limited to these.

In a specific embodiment, the bispecific antigen-binding molecule is monovalent for both normal T cell-side target antigen and target tumor antigen expressed on T cell tumor cells.

In a specific embodiment, the bispecific antigen-binding molecule is bivalent for both normal T cell-side target antigen and target tumor antigen expressed on T cell tumor cells.

In a specific embodiment, the bispecific antigen-binding molecule is trivalent for both normal T cell-side target antigen and target tumor antigen expressed on the T cell cellular tumor cells.

In a specific embodiment, the bispecific antigen-binding molecule is monovalent for normal T cell-side target antigen and bivalent for target tumor antigen expressed on the T cell tumor cells.

In a specific embodiment, the bispecific antigen-binding molecule is bivalent for normal T cell-side target antigen and monovalent for target tumor antigen expressed on the T cell tumor cells.

In a specific embodiment, the bispecific antigen-binding molecule has a structure having two of structures each consisting of scFv that specifically binds to the normal T cell-side target antigen (e.g., TRBC1 or TRBC2) and an Fab fragment that specifically binds to the target tumor antigen expressed on the T cell tumor cells, which are linked with a peptide linker (i.e., bivalent + bivalent) and further having an Fc domain (Fab-scFv-Fc).

In a specific embodiment, the T cell tumor may be adult T cell leukemia (ATL), peripheral T cell lymphoma (PTCL), or T cell acute lymphoblastic leukemia (TALL). Peripheral T cell lymphoma (PTCL) is the general term for lymphomas that originate from T cells that have differentiated and matured in the thymus and migrated to peripheral organs. PTCL accounts for about 10% of the total lymphomas in Japan. PTCL types are classified into peripheral T cell lymphoma, not otherwise specified (PTCL-NOS), angioimmunoblastic T cell lymphoma (AITL), ALK-positive anaplastic large cell lymphoma (ALCL), and ALK-negative ALCL. PTCL-NOS is difficult to be histopathologically differentiated from ATL (adult T cell lymphoma) and requires screening for anti-HTLV-1 antibodies.

In a specific embodiment, the therapeutic agent of the present invention can be administered in combination with chemotherapeutic agents, radiation and/or other agents used in cancer immunotherapies.

The dose and interval for administration of the therapeutic agent of the present invention may be individually adjusted to provide a plasma level of the bispecific antigen-binding molecule of the present invention sufficient to maintain the therapeutic effect. The dose ranges from about 0.1 to 50 mg/kg/day, depending on the desired therapeutic effect, method of administration, treatment period, age, body weight etc. The therapeutically effective plasma level can be achieved by daily multiple administrations. The plasma level can be measured by, for example, HPLC.

In the combination therapy, the therapeutic agent of the present invention and one or more drugs or therapies may be administered or performed simultaneously or sequentially. When administered or performed simultaneously or sequentially, all the agents or therapies may be administered or performed through the same route or different routes.

In this description, simultaneous administration means that two or more types drugs or therapies are administered or performed with an interval of few seconds to few minutes or shorter. For example, two types of drugs or therapies are administered or performed with an interval of about 15 to 1 minutes or shorter. Sequential administration referred to in this description means that drugs or therapies are administered or performed with an interval of several minutes, several hours, several days, or several weeks. For example, two types of drugs or therapies are administered or performed with an interval of 15 minutes or longer, 30 minutes or longer, 60 minutes or longer, or an interval of 1 day, 2 days, 3 days, 4 days, 5 days, 6 days, 7 days, 2 weeks, 3 weeks, or 4 weeks.

The therapeutic agent of the present invention may be a pharmaceutical composition. Such a pharmaceutical composition can further contain a pharmaceutically acceptable carrier. Formulations of pharmaceutical compositions containing pharmaceutically acceptable carriers are known in the art (e.g., Remington: The Science and Practice of Pharmacy (23rd edition (2020)). Examples of pharmacologically acceptable carrier include buffers such as phosphates, citrates, and other organic acids; antioxidants such as ascorbic acid; proteins such as serum albumin, gelatin, and immunoglobulin; hydrophilic polymers such as polyvinylpyrrolidone; amino acids such as glycine, glutamine, asparagine, histidine, arginine, and lysine; monosaccharides, disaccharides, and other carbohydrates including glucose, mannose, and dextrin; chelating agents such as EDTA; sugars such as sucrose, mannitol, trehalose, and sorbitol; salt forming counter ions such as sodium; metal complexes (e.g., Zn-protein complexes); and nonionic surfactants such as polyethylene glycol (PEG), but are not limited to these.

The pharmaceutical composition of the present invention is preferably produced under conditions that conform to the manufacturing control and quality control regulations for pharmaceuticals and quasi-drugs (good manufacturing practice, GMP).

In one embodiment, the pharmaceutical composition is a liquid such as a solution, suspension, emulsion, microemulsion, or gel. The pharmaceutical composition may be an aqueous formulation and can contain at least 50% (w/w) or more of water. In one embodiment, the pharmaceutical composition is in a form suitable for injection. The injection may be, for example, subcutaneous, intramuscular, intraperitoneal, intravitreal, or intravenous injection.

In one embodiment, the pharmaceutical composition may be in a solid dosage form. Examples of the solid dosage form include, for example, lyophilized or spray-dried forms, and the pharmaceutical composition in such forms may be administered, for example, after addition of a solvent and/or diluent.

The pharmaceutical composition may be provided with a packaging such as syringes, vials, or injection bags. Such syringes may contain the pharmaceutical composition in a lyophilized form (which must be solubilized with, for example, water for injection prior to administration) or in an aqueous form. Other examples of the solid dosage form include powders, granules, tablets and capsules.

In a specific embodiment, the therapeutic agent of the present invention can be used in a method for treating a T cell malignancy of a subject, the treatment method comprises determining subtype of an antigen expressed on the T cell tumor cells of the subject and administering the therapeutic agent to the subject, and the therapeutic agent comprises a bispecific antigen-binding molecule comprising at least one portion that specifically binds to a normal T cell-side target antigen of a subtype different from that determined to be expressed on the T cell tumor cells of the subject. The subtype of the antigen expressed on the target T cell tumor cells of the subject can be determined by flow cytometric analysis using a fluorescent dye-labeled antibody that specifically binds to the antigen of that subtype. If the antigen is TRBC and the subtype is TRBC1, the JOVI-1 clone antibody can be used, and if the subtype is TRBC2, the various antibodies described in WO2020/089644 can be used.

The second aspect of the present invention is a method for treating a T cell malignancy in a subject, which comprises administering a bispecific antigen-binding molecule to a subject having a T cell malignancy.

In a specific embodiment, the treatment method further comprises determining subtype of a normal T cell-side target antigen expressed on T cell tumor cells of the subject, and the bispecific antigen-binding molecule comprises at least one portion that specifically binds to the normal T cell-side target antigen of a subtype different from the subtype of the antigen determined to be expressed on the T cell tumor cells of the subject.

The third aspect of the present invention is a bispecific antigen-binding molecule for use in a method for of treating a T cell malignancy.

The fourth aspect of the present invention is use of the bispecific antigen-binding molecule in manufacture of a pharmaceutical agent for T cell malignancies.

The bispecific antigen-binding molecule used in the second, third, and fourth aspects of the present invention is the same as the bispecific antigen-binding molecule that constitutes the therapeutic agent as the first aspect described above in this description.

The term "subject" used in this description means a mammal, e.g., mouse, rat, and primate, especially human.

### <Bispecific antigen-binding molecule>

The fifth aspect of the present invention is a bispecific antigen-binding molecule. According to the present invention, there is provided a bispecific antigen-binding molecule that constitutes the therapeutic agent of the first aspect.

The bispecific antigen-binding molecule of the present invention comprises
(1) at least one portion that specifically binds to a target tumor antigen expressed on T cell tumor cells, and
(2) at least one portion that specifically binds to a normal T cell-side target antigen having subtypes, and
the at least one portion that specifically binds to a normal T cell-side target antigen having subtypes is at least one portion that specifically binds to TRBC1 or TRBC2 (T cell receptor β constant region 1 or 2) of normal T cells (TRBC1-binding portion or TRBC2-binding portion).

The amino acid sequences of the TRBC1-binding portion or TRBC2-binding portion and target tumor antigen-binding portion that the bispecific antigen-binding molecule of the present invention may have are the same as those described above for the bispecific antigen-binding molecule that constitutes the therapeutic agent of the first aspect. Further, the formats that may be of the bispecific antigen-binding molecule of the present invention are the same as those described above in this description for the bispecific antigen-binding molecule that constitutes the therapeutic agent of the first aspect.

The present invention further provides a nucleic acid molecule encoding the heavy and light chains of the bispecific antigen-binding molecule of the fifth aspect. The nucleic acid molecule of the present invention can be RNA, DNA or cDNA.

The nucleic acid molecule of the present invention may be in the form of a vector, may be present in a vector, and/or may be a part of a vector such as plasmid, cosmid or YAC. The vector may be, especially, an expression vector, or a vector that provides expression of the bispecific antigen-binding molecule of the present invention in host cells, host organism, and/or expression system. Such an expression vector typically contains at least one nucleic acid of the present invention operably linked to one or more appropriate expression regulatory elements (e.g., promoter, enhancer, terminator, etc.). The selection of elements for expression in a specific host and sequences thereof is within common knowledge to those skilled in the art. Specific examples of the regulatory and other elements useful or essential for expression of the heavy and light chains of the bispecific antigen-binding molecule of the present invention include promoter, enhancer, terminator, incorporation element, selection marker, leader sequence, reporter gene, etc.

The nucleic acid molecule of the present invention can be prepared or obtained, and/or can be isolated from a suitable natural resource by known methods (e.g., by automated DNA synthesis and/or recombinant DNA techniques) on the basis of the information on the amino acid sequences of the heavy and light chains of the bispecific antigen-binding molecule of the present invention disclosed in this description.

The present invention further provides a host cell that expresses or is capable of expressing one or more of the heavy and light chains of the bispecific antigen-binding molecule of the fifth aspect. The host cell of the present invention can contain a nucleic acid or vector. Preferred examples of cells for the host cell of the present invention include bacterial cells, fungal/yeast cells or mammalian cells.

Examples of suitable bacterial cells include cells of Gram-negative bacteria strains (e.g., *Escherichia, Proteus* and *Pseudomonas* bacteria) and Gram-positive bacteria strains (e.g., *Bacillus, Streptomyces, Staphylococcus,* and *Lactococcus* bacteria).

Examples of suitable fungal and yeast cells include cells of *Trichoderma, Neurospora,* and *Aspergillus* species; and cells of *Saccharomyces* species, e.g., *Saccharomyces cerevisiae, Schizosaccharomyces* species, e.g., *Schizosaccharomyces pombe, Pichia* species, e.g., *Pichia pastoris* and *Pichia methanolica,* as well as *Hansenula* species. Examples of suitable mammalian cells include, for example, HEK293 cells, CHO cells, BHK cells, HeLa cells, COS cells, etc.

However, amphibian cells, insect cells, plant cells, and any other cells used for expressing heterologous proteins in this technical field can also be used in the present invention.

The heavy and light chains of the bispecific antigen-binding molecules of the present invention can be produced in host cells as described above and then isolated from the host cells, optionally followed by further purification. Alternatively, they can be produced extracellularly (e.g., in the medium in which the host cells are cultured), followed by isolation from the medium and optionally further purification.

Methods and reagents for recombinant production of polypeptides, such as specific suitable expression vectors, transformation or transfection methods, selection markers, methods for inducing protein expression, and culture conditions, are known in the art. Similarly, protein isolation and purification techniques suitable for the method for preparing the bispecific antigen-binding molecule of the present invention are well known to those skilled in the art.

However, the bispecific antigen-binding molecule of the present invention can also be obtained by other protein-producing methods known in the art, such as chemical synthesis including solid-phase and liquid-phase syntheses.

### (Cytotoxicity)

The bispecific antigen-binding molecule of the present invention having a TRBC1-binding portion or TRBC2-binding portion can mobilize TRBC1-positive or TRBC2-positive T cells and induce cytotoxicity against tumor cells and/or cell death of tumor cells. The cytotoxicity of the bispecific antigen-binding molecule can be determined by culturing target tumor cells with the bispecific antigen-binding molecule *in vitro* in the presence of effector cells, and measuring viability of the target tumor cells. As the effector cells, for example, activated T cells (e.g., T-LAK) can be used. The mixing ratio of the effector cells to the target tumor cells may be 10:1 to 1:10. In one embodiment, the bispecific antigen-binding molecule of the present invention can induce cytotoxicity against tumor cells *in vitro* at an EC50 lower than about 500 pM, and in a preferred embodiment, it can induce cytotoxicity against tumor cells at an EC50 lower than about 400 pM, lower than about 300 pM, lower than about 200 pM, lower than about 100 pM, or lower than about 50 pM.

The anti-TRBC1 antibody used for the TRBC1-binding portion of the bispecific antigen-binding molecule of the present invention can increase the TRBC1-positive cell ratio in human peripheral blood mononuclear cells (PBMCs). Specifically, as shown in Reference Example 1 described below, when 10 nM anti-TRBC1 antibody was added to human PBMCs and the cells were cultured in the presence of IL-2, the ratio of TRBC1-positive cells increased from 30% on day 0 to over 80% on day 15. Thus, it was suggested that the addition of anti-TRBC1 antibody selectively proliferated TRBC1-positive cells. It is considered that addition of a bispecific antibody having the same antigen-binding portion as the anti-TRBC1 antibody would similarly selectively proliferate TRBC1-positive cells.

The anti-TRBC1 antibody used for the TRBC1-binding portion of the bispecific antigen-binding molecule of the present invention can increase cytotoxic T cells in the presence of IL-2. Specifically, as shown in Reference Example 1 described below, on day 15, the ratio of effector memory T cells significantly increased to about 70% from about 20% in the presence of the anti-TRBC1 antibody and IL-2. Based on these results, it was considered that stimulation with anti-TRBC1 antibody can increase T cells that induce cytotoxicity. It is considered that stimulation with a bispecific antibody having the same antigen-binding portion as the anti-TRBC1 antibody would similarly increase T cells that induce cytotoxicity.

The anti-TRBC1 antibody used for the TRBC1-binding portion of the bispecific antigen-binding molecule of the present invention can activate T cells. Specifically, as shown in Reference Example 1 described below, stimulation with the anti-TRBC1 antibody increased the ratios of CD69, granzyme B, and perforin-positive cells, suggesting that TRBC1-positive cells were activated by the anti-TRBC1 antibody. A bispecific antibody having the same antigen-binding portion as the anti-TRBC1 antibody used in the example is also expected to have a similar T cell-activating activity.

Stimulation with the anti-TRBC1 antibody used for the TRBC1-binding portion of the bispecific antigen-binding molecule of the present invention did not significantly alter the ratio of PD-1 positive cells, as shown in Reference Example 1 described below. This suggested that T cells are less likely to be exhausted by activation of T cells with an anti-TRBC1 antibody. It is considered that T cells are similarly unlikely to be exhausted by a bispecific antibody having the same antigen-binding portion as the anti-TRBC1 antibody used in the example.

The anti-TRBC1 antibody used for the TRBC1-binding portion of the bispecific antigen-binding molecule of the present invention could induce T cell activation and release of cytokines as shown in Reference Example 1 described below, but the amount of released IL-6, an inflammatory cytokine, was very small. This result indicated that the risk of cytokine release syndrome by anti-TRBC1 antibody may be low. Low risk of cytokine release syndrome is similarly suggested also for the bispecific antibody of the present invention that has the same antigen-binding portion as the anti-TRBC1 antibody.

The anti-TRBC2 antibody used for the TRBC2-binding portion of the bispecific antigen-binding molecule of the present invention can increase the TRBC2-positive cell ratio in PBMCs. Specifically, as shown in Reference Example 2 described below, when cells that bind to TRBC1 antibody (TRBC1-positive T cells) were removed from human PBMCs using beads, then 10 nM anti-TRBC2 antibody was added, and the cells were cultured in the presence of IL-2, the cell count on day 0, approximately 5 x10⁶ to 7 x10⁶ cells, increased to about 18 x 10⁶ to 42 x 10⁶ cells on day 15. Thus, it was suggested that the addition of anti-TRBC2 antibody selectively proliferated TRBC2-positive cells. It is considered that addition of a bispecific antibody having the same antigen-binding portion as the anti-TRBC2 antibody would similarly selectively proliferate TRBC2-positive cells.

The anti-TRBC2 antibody used for the TRBC2-binding portion of the bispecific antigen-binding molecule of the present invention can increase cytotoxic T cells in the presence of IL-2. Specifically, as shown in Reference Example 2 described below, the ratio of the effector memory T cells significantly increased from about 27% to about 81% on day 15 in the presence of anti-TRBC2 antibody and IL-2. Based on this result, it was considered that stimulation with an anti-TRBC2 antibody can increase T cells that induce cytotoxicity. It is considered that stimulation with a bispecific antibody having the same antigen-binding portion as the anti-TRBC2 antibody would similarly increase T cells that induce cytotoxicity.

The anti-TRBC2 antibody used for the TRBC2-binding portion of the bispecific antigen-binding molecule of the present invention can activate T cells. Specifically, as shown in Reference Example 2 described below, stimulation with anti-TRBC2 antibody increased the ratios of CD69, granzyme B, and perforin-positive cells, suggesting that TRBC2-positive cells were activated by the anti-TRBC2 antibody. A bispecific antibody having the same antigen-binding portion as the anti-TRBC2 antibody are expected to similarly have a T cell-activating effect.

Stimulation with the anti-TRBC2 antibody used for the TRBC2-binding portion of the bispecific antigen-binding molecule of the present invention did not significantly alter the ratio of PD-1-positive cells, as shown in the reference example described below. This suggested that T cells are less likely to be exhausted by activation of T cells with an anti-TRBC2 antibody. It is considered that T cells are similarly unlikely to be exhausted by the bispecific antibody of the present invention having the same antigen-binding portion as the anti-TRBC2 antibody.

The anti-TRBC2 antibody used for the TRBC2-binding portion of the bispecific antigen-binding molecule of the present invention could induce T cell activation and release of cytokines, as shown in Reference Example 2 described below, but the amount of released IL-6, an inflammatory cytokine, was very small. This result indicated that the risk of cytokine release syndrome caused by anti-TRBC2 antibodies may be low. Low risk of cytokine release syndrome is similarly suggested also for the bispecific antibody of the present invention that has the same antigen-binding portion as the anti-TRBC2 antibody.

The bispecific antigen-binding molecule of the present invention having a TRBC1-binding portion or TRBC2-binding portion is used in a method for
(i) inducing cytotoxicity against tumor cells,
(ii) increasing TRBC1-positive cell ratio or increasing TRBC2-positive cell ratio,
(iii) increasing cytotoxic T cells,
(iv) activating T cells,
(v) stimulating or enhancing cellular responses without exhausting T cells,
(vi) stimulating or enhancing cellular responses without increasing the risk of cytokine release syndrome,
(vii) treating a T cell malignancy,
(viii) delaying progression of a T cell malignancy, or
(ix) prolonging survival of a subject suffering from a T cell malignancy.

The bispecific antigen-binding molecule of the present invention can have an antitumor activity. The antitumor activity can be measured, for example, *in vivo.* The antitumor activity can be evaluated, for example, as follows. For example, 1 x 10⁷ cells/animal of human PBMCs are intravenously transplanted to the immunocompromised mice, NSG mice (Jackson Laboratory Japan), and 7 to 10 days later, 1 x 10⁷ cells/animal of cells of T cell tumor strain stably expressing luciferase are intravenously transplanted. Then, the bispecific antigen-binding molecule of the present invention is administered at a dose of 0.5 mg/kg twice/week. Two to three weeks after the start of the administration, 150 mg/kg of luciferin is intraperitoneally administered to the mice, and the *in vivo* luciferase activity is quantified by using IVIS Imaging System (Revvity) or the like to measure the T cell tumor volume. The anti-tumor activity can be evaluated by comparing the T cell tumor volumes of the control group and the group administered with the bispecific antigen-binding molecule of the present invention.

### Examples

The present invention will be more specifically explained with reference to the following examples. However, the present invention is not limited to these examples.

### <Example 1> Preparation of bispecific antibody

### 1.1 Bispecific antibody

In this example, bispecific antibodies that bind to TRBC1 or TRBC2 and a target tumor antigen were prepared.

### TRBC1- or TRBC2-binding portion

The TRBC1-binding portion was prepared on the basis of the sequence information of the anti-TRBC1 antibody JOVI-1 clone described in U.S. Patent No. 10730942 (US10730942, Protein-based T-cell receptor knockdown, SEQ ID NOS: 15 and SEQ ID NO: 16).

The TRBC2-binding portion was prepared on the basis of a VH domain derived from the VH domain of hJOVI-1 described in WO2020/089644 (SEQ ID NO: 1 in WO2020/089644) by three amino acid substitutions T28K, Y32F and A100N (Table 1 in WO2020/089644 (page 23)) and the VL domain of hJOVI-1 (SEQ ID NO: 2 in WO2020/089644) (the heavy and light chain amino acid sequences described in this description as SEQ ID NOS: 6 and 7).

The portions that bind to target tumor antigens expressed on T cell tumor cells were prepared on the basis of sequence information disclosed in the references listed in the following table.

**[Table 3]**

| Target tumor antigen | Antibody name | Reference |
|---|---|---|
| CCR4 | Mogamulizumab | Japanese Patent No. 4052515 (VH:SEQ ID NO:9,VL:SEQ ID NO:14) |
| CD1a | SC02-113 | WO2005/063819 (VH: amino acid positions 1-117 from the N-terminus of SEQ ID NO:26, VL: amino acid positions 133-240 from the N-terminus of SEQ ID NO:26) |
| CCR9 | 9G7 | WO 2023/037125 (VH: SEQ ID NO:7, VL: SEQ ID NO:8) |
| CXCR4 | hz515H7 VH1 D76N-VL2 | WO2010/125162 (VH: SEQ ID NO:64, VL: SEQ ID NO:65) |
| TIGIT | Vibostolimab | WO2016/028656 (VH: SEQ ID NO:128, VL: SEQ ID NO: 132) |
| CCR8 | ABBV-514 | WO2023/010054 (VH: SEQ ID NO:7, VL: SEQ ID NO:8) |
| CD30 | AC10 | Japanese Patent No. 4303964 (VH: SEQ ID NO:2, VL: SEQ ID NO:10) |
| CD40L | ABI793 | Japanese Unexamined Patent Application Publication (Translation of PCT Application) No. 2003-526371 (VH: SEQ ID NO:2, VL: SEQ ID NO:4) |

The sequences of the heavy and light chains of the bispecific antibodies prepared in this example are shown below.

**[Table 4]**

| Antibody name | Antibody format | Tumor antigen | T cell antigen | Amino acid sequence of heavy chain | | Amino acid sequence of light chain | |
|---|---|---|---|---|---|---|---|
| CCR0001 | Fab-scFv | CCR4 | TRBC1 | SEQ ID NO:1 | CCR0001 VH | SEQ ID NO:2 | CCR0001 VL |
| CCR0004 | Fab-scFv-Fc | CCR4 | TRBC1 | SEQ ID NO:3 | CCR0004 VH | SEQ ID NO:2 | CCR0001 VL |
| CD1a1001 | Fab-scFv | CD1a | TRBC1 | SEQ ID NO:30 | CD1a1001 VH | SEQ ID NO:29 | CD1a1001 VL |
| CD1a1003 | Fab-scFv | CD1a | TRBC2 | SEQ ID NO:31 | CD1a1003 VH | SEQ ID NO:29 | CD1a1003 VL (CD1a1001 VL) |
| CD1a1004 | Fab-scFv-Fc | CD1a | TRBC1 | SEQ ID NO:32 | CD1a1004 VH | SEQ ID NO:29 | CD1a1001 VL |
| CD1a1006 | Fab-scFv-Fc | CD1a | TRBC2 | SEQ ID NO:33 | CD1a1006 VH | SEQ ID NO:29 | CD1a1003 VL(CD1al1001 VL) |
| CCR9-1002 | Fab-scFv-Fc | CCR9 | TRBC1 | SEQ ID NO:66 | CCR91002 VH | SEQ ID NO:67 | CCR91002 VL |
| CCR9-1004 | Fab-Fc-scFv | CCR9 | TRBC1 | SEQ ID NO:68 | CCR91004 VH | SEQ ID NO:67 | CCR91002 VL |
| CXCR4-1002 | Fab-scFv-Fc | CXCR4 | TRBC1 | SEQ ID NO:69 | CXCR41002 VH | SEQ ID NO:70 | CXCR41002 VL |
| CXCR4-1004 | Fab-Fc-scFv | CXCR4 | TRBC1 | SEQ ID NO:71 | CXCR41004 VH | SEQ ID NO:70 | CXCR41002 VL |
| TIGIT2001 | Fab-scFv-Fc | TIGIT | TRBC1 | SEQ ID NO:72 | TIG2001 VH | SEQ ID NO:73 | TIG2001 VL |
| TIGIT2002 | Fab-Fc-scFv | TIGIT | TRBC1 | SEQ ID NO:74 | TIG2002 VH | SEQ ID NO:73 | TIG2001 VL |
| CCR81001 | Fab-scFv-Fc | CCR8 | TRBC1 | SEQ ID NO:75 | CCR81001 VH | SEQ ID NO:76 | CCR81001 VL |
| CCR81002 | Fab-Fc-scFv | CCR8 | TRBC1 | SEQ ID NO:77 | CCR81002 VH | SEQ ID NO:76 | CCR81001 VL |
| CCR0010 | Fab-scFv | CCR4 | TRBC2 | SEQ ID NO:28 | CCR0010 VH | SEQ ID NO:2 | CCR0010 VL (CCR0001VL) |
| CD301001 | Fab-scFv | CD30 | TRBC1 | SEQ ID NO:78 | CD301001 VH | SEQ ID NO:79 | CD301001 VL |
| CD301003 | Fab-scFv-Fc | CD30 | TRBC1 | SEQ ID NO:80 | CD301003 VH | SEQ ID NO:79 | CD301001 VL |
| CD40L1007 | Fab-Fc-scFv | CD40L | TRBC1 | SEQ ID NO:81 | CD40L1007 VH | SEQ ID NO:82 | CD40L1007 VL |
| CD40L1008 | Fab-scFv-Fe | CD40L | TRBC1 | SEQ ID NO:83 | CD40L1008 VH | SEQ ID NO:82 | CD40L1007 VL |

Schematic diagrams of the Fab-scFv, Fab-scFv-Fc, and Fab-Fc-scFv type antibodies are shown in Fig. 2A.

CCR0010 was obtained by introducing T255K, Y259F and A327N mutations into the heavy chain of CCR0001, and the structure thereof is Fab-scFv type. The anti-TRBC-2 antibodies developed so far have been obtained by introducing mutations into the anti-TRBC-1 antibody (JOVI-1) (e.g., WO2015/132598A1 and WO2020/089644A1), and the amino acid sequences of CDRs of the both are highly homologous.

### 1.2 Preparation and purification of recombinant antibody

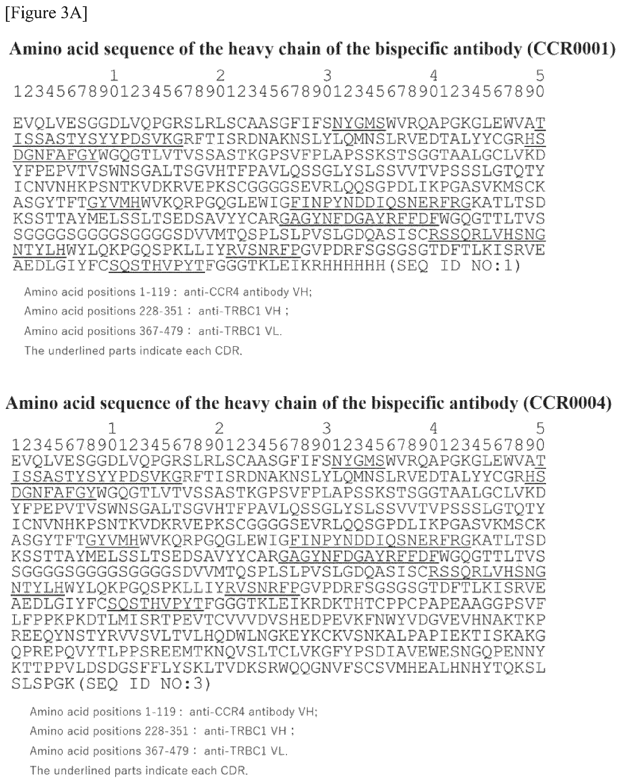

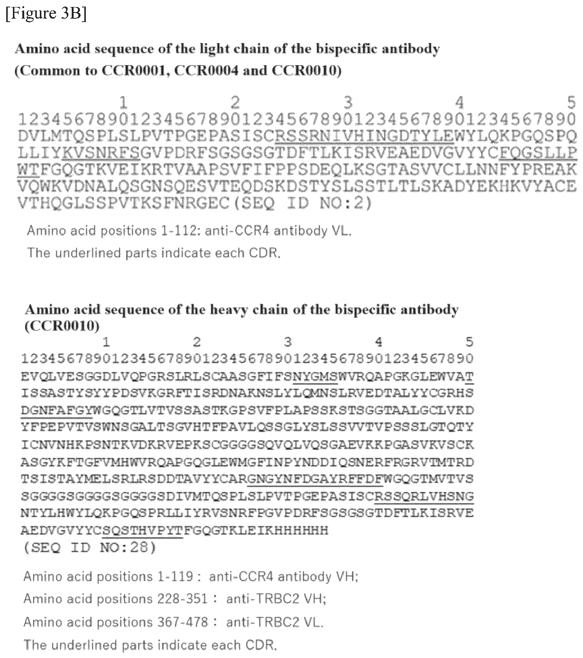

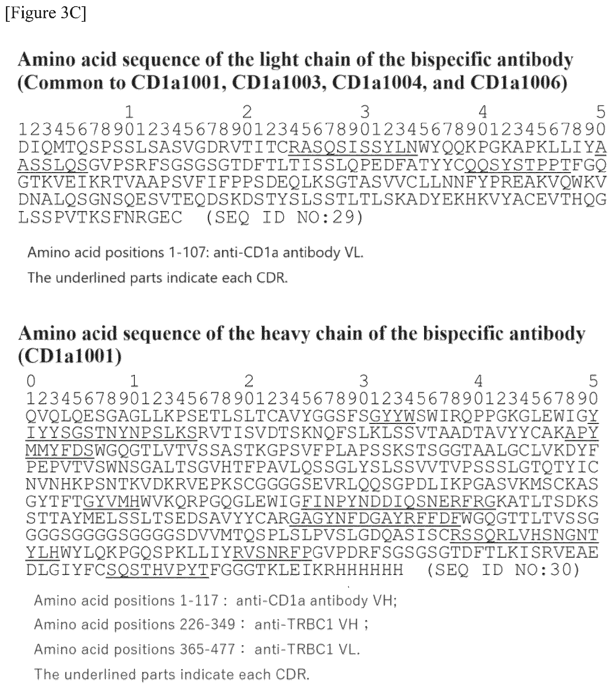

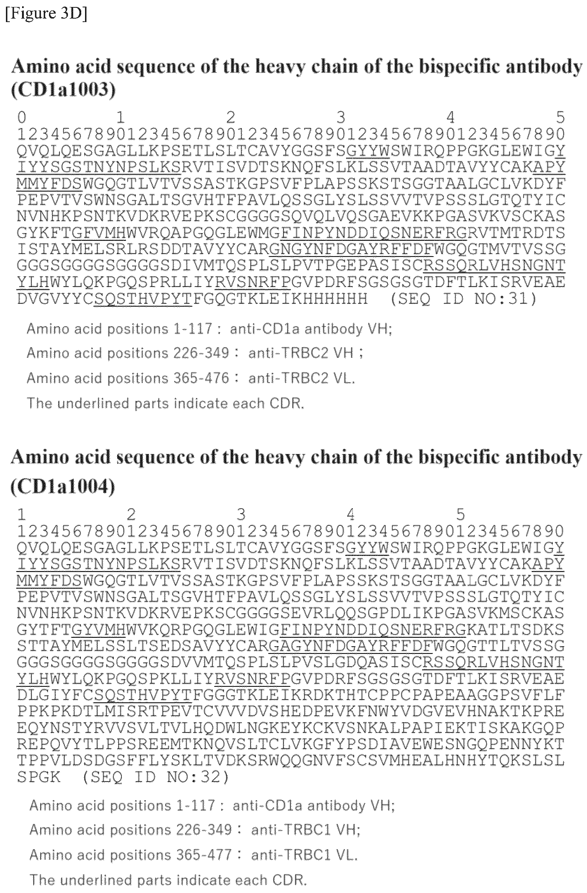

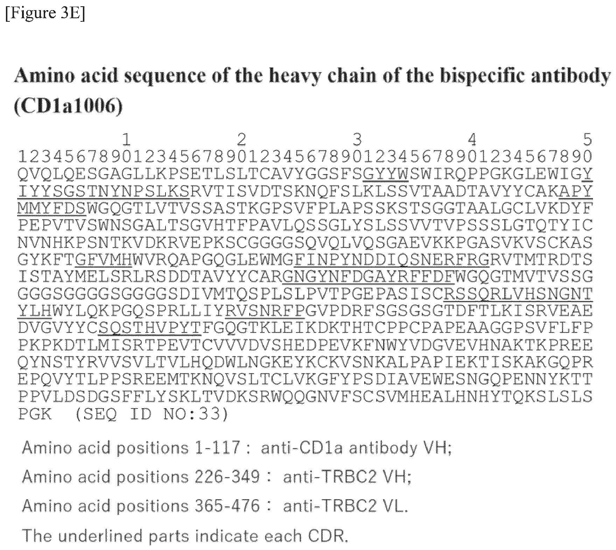

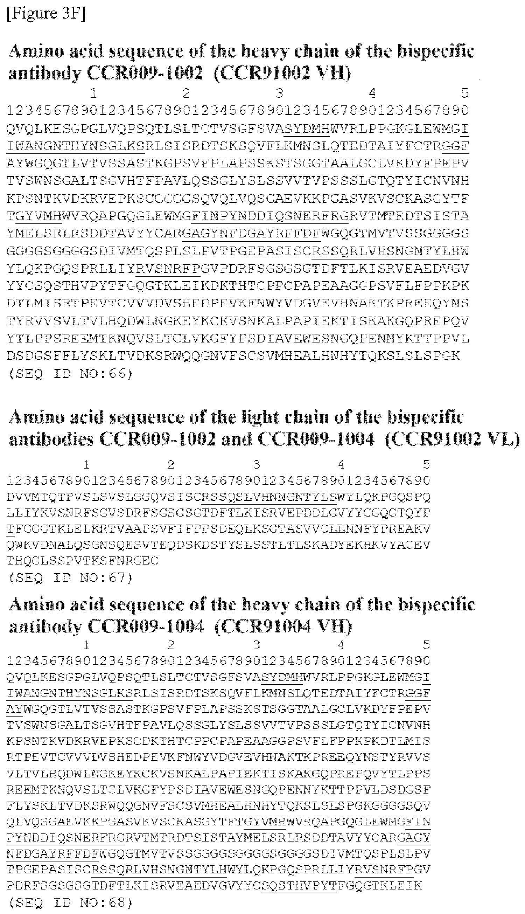

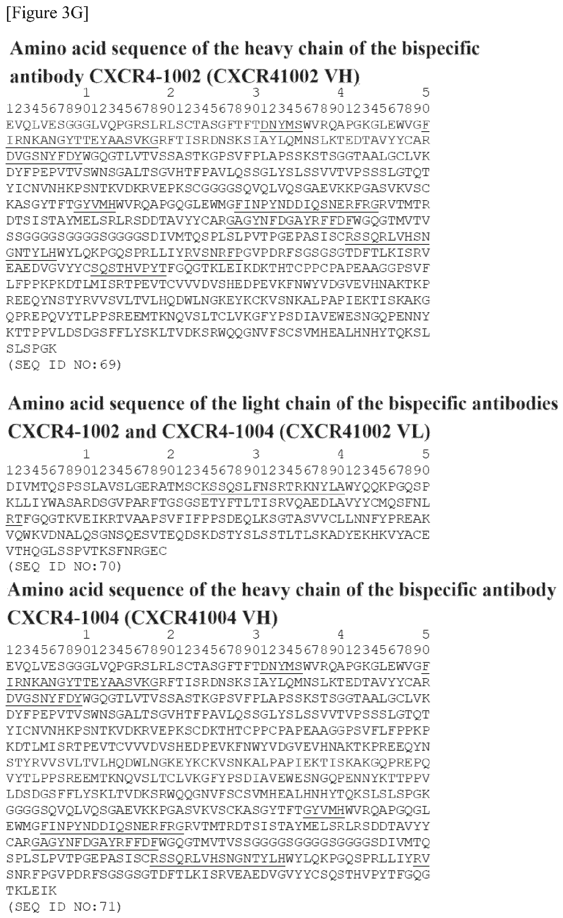

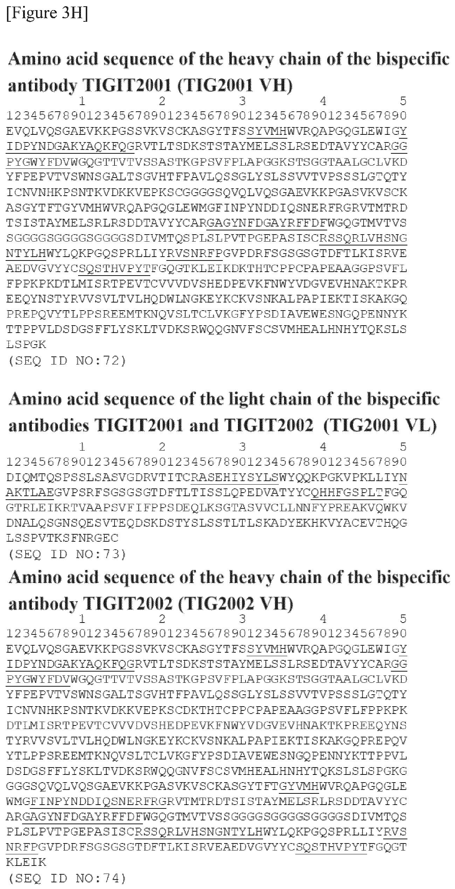

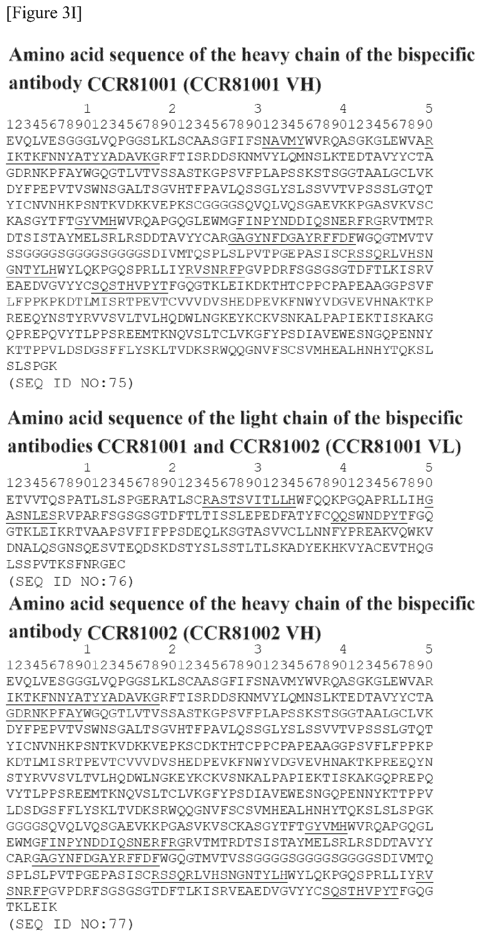

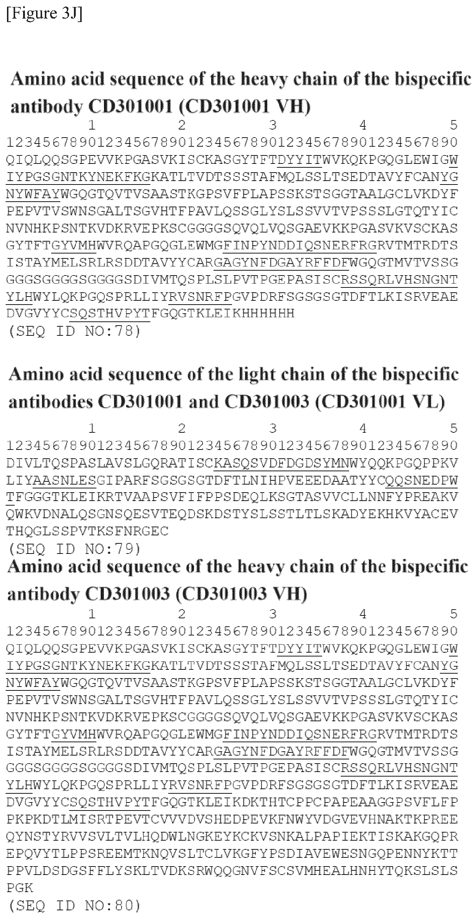

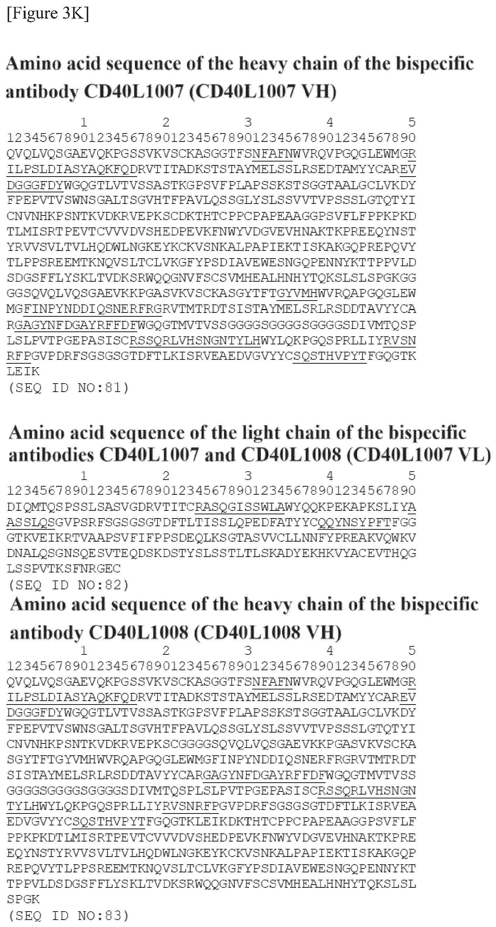

The bispecific antibodies listed in Table 4 were constructed by expressing the heavy and light chains listed in Table 4 with expression vectors, respectively. Details of the sequences of the heavy and light chains of each bispecific antibody are as described in Figs. 3A to 3K.

Specifically, bispecific antibody (heavy and light chains) expression vectors were constructed by using DNA encoding each bispecific antibody and an expression vector (pcDNA3.4, Thermo Fisher Scientific). The combinations of the heavy and light chains of the bispecific antibodies are as listed in Table 4. The above expression vectors were mixed at a ratio of 1:1 and transfected into CHO cells at a DNA amount of 0.8 µg per mL of culture medium by using ExpiFectamine CHO Reagent (Thermo Fisher Scientific). ExpiCHO Feed and ExpiFectamine CHO Enhancer were added on the next day and after 5 days of the transfection, and culture was performed at 32°C for 10 to 12 days. The cells were removed from the culture medium by centrifugation and filtration, and the culture supernatant was collected. Purification of the antibodies was performed by a combination of affinity chromatography using nickel-charged agarose and gel filtration chromatography or a combination of Protein A affinity chromatography and gel filtration chromatography.

### <Example 2> Preparation of cells

In this example, preparation of cells used in the experiments will be described.

The following reagents were used
RPMI 1640 medium (Nacalai Tesque, 30264-56)
FBS (fetal bovine serum, Nichirei Bioscience, 175012, proteins were deactivated by incubation on a water bath at 56°C for 30 minutes)
Penicillin-streptomycin mixed solution (Nacalai Tesque, 2625384)
MEM nonessential amino acid solution 100X (Gibco, 11140-050)
Sodium pyruvate solution 100 mM (Gibco, 11360-070)
Anti-TRBC1 antibody (prepared and purified by the methods described in Example 1, the heavy and light chain sequences of the anti-TRBC1 antibody used are the sequences of SEQ ID NOS: 4 and 5).
Imunace Injection 35 (IL-2 (interleukin 2), Shionogi Kyowa Pharmaceutical Industry, dissolved in PBS at 250 IU/µL and stored at -25°C)
PBS (phosphate buffered saline, Gibco, 10010-023)
Human PBMCs (peripheral blood mononuclear cells, CTL, CTL-UP1)

The various cells were prepared as follows.

T-LAK is a T cell population that expresses a lymphokine-activated killer (LAK) function. T-LAK was prepared with an experimental system optimized according to Ochoa AC et al. (Lymphokine-activated killer activity in long-term cultures with anti-CD3 plus interleukin 2: identification and isolation of effector subsets. Cancer Res. 1989 Feb 15;49(4):963-8., of which entire description is specifically incorporated herein by reference). Specifically, human PBMCs were lysed on a 37°C water bath and washed once with the culture medium. After 2 days of culture in the RPMI 1640 medium containing 10% FBS supplemented with 10 nM anti-TRBC1 antibody and 100 IU/mL IL-2, the cells were further cultured in the RPMI 1640 medium containing IL-2 and 10% FBS (up to 17 days) and thereby derived into T-LAK. The cells were subcultured every 2 or 3 days, and 100 IU/mL IL-2 was newly added at the time of culture medium change. The cell counts on the day of the start of the culture (day 0) and day 15 were measured by using an automated cell counter (BIO RAD, TC20).

The MT-2 cells used in Example 3 are cells of a human leukocyte cell strain established by co-culture with human ATL (adult T cell leukemia) cells, and they were purchased from the JCRB Cell Bank (National Institutes of Biomedical Innovation, Health and Nutrition, cell number JCRB1210). The MT-2 cells are CCR4-positive (Haematologica 2018 Volume 103(1):126-135, of which entire description is specifically incorporated herein by reference). The cells were cultured by using the RPMI medium containing 10% FBS, nonessential amino acids, and sodium pyruvate in a 5% CO₂, 37°C incubator, and subcultured 2 or 3 times per week.

The MOLT-4 cells used in Example 3 were purchased from the National Institutes of Biomedical Innovation, Health and Nutrition. The cells were cultured in the RPMI medium containing 10% FBS in a 5% CO₂, 37°C incubator, and subcultured 2 or 3 times per week.

The CCRF-CEM9 cells used in the following examples express CD1a and TRBC2. The CCRF-CEM9 cells were obtained from CCRF-CEM, a TALL-derived cell strain, by single-cell cloning using the limiting dilution method. Specifically, a CCRF-CEM cell suspension of 1 x 10⁵ cells/mL was repeatedly diluted to a density of 2.5 cells/ml. The diluted suspension of 2.5 cells/mL was added to two of 96-well plates in a volume of 100 µL per well, and culture was performed at 37°C. For each well in which cells (clone) had grown, expansion culture was performed, and TRBC1 and CD1a expression analysis was performed. As a result, CCRF-CEM9 cells for which expression of the both antigens was confirmed were obtained. The cells were cultured in the RPMI medium containing 10% FBS in a 5% CO₂, 37°C incubator and subcultured 2 or 3 times per week.

### <Reference Example 1> Verification of effector activity of anti-TRBC1 antibody and quantification of released cytokine

Reagents used
Flow Cytometry Staining Buffer (eBioscience, 00-4222-26)
Clear Back (MBL, MT-001)
Fixable Viability Dye eFluoror 780 (Invitrogen, 65-0865-14)
Transcription Factor Buffer Set (BD, 562574)
TF Fix/Perm Buffer (4x) (51-9008100), diluted 4 times with TF Diluent Buffer before use
TF Diluent Buffer (51-9008101)
TF Perm/Wash Buffer (5x) (51-9008102), diluted 5 times with distilled water before use MILLIPLEX Human Cytokine/Chemokine/Growth Factor Panel A (Millipore, HCYTA-60K)

The antibodies for immunostaining are shown in the following table.

**[Table 5]**

| Antibody | Dye-labeled | Manufacturer | Item number | Remarks |
|---|---|---|---|---|
| anti-TRBC1 antibody | HiLyte Fluor 647 | Prepared in-house | - | |
| anti-CD3 antibody | FITC | Invitrogen | 11-0037-42 | |
| anti-CD27 antibody | PE | BioLegend | 356406 | |
| anti-CD45RA antibody | BV421 | BioLegend | 304130 | |
| anti-CD69 antibody | PE | BioLegend | 310906 | |
| anti-CD25 antibody | BV421 | BioLegend | 302630 | |
| anti-Granzyme antibody | PE | BD | 561142 | |
| anti-Perforin antibody | BV421 | BioLegend | 308122 | |
| anti-PD-1 antibody | BV711 | BioLegend | 329928 | |
| human IgG antibody | HiLyte Fluor 647 | Jackson Immuno Research | 009-000-003 | Isotype control |
| mouse IgG2a antibody | FITC | BioLegend | 400209 | Isotype control |
| mouse IgG1 antibody | PE | BioLegend | 400114 | Isotype control |
| mouse IgG1 antibody | BV421 | BioLegend | 400158 | Isotype control |
| mouse IgG2a antibody | BV421 | BioLegend | 400259 | Isotype control |
| mouse IgG2b antibody | BV421 | BioLegend | 400342 | Isotype control |
| mouse IgG1 antibody | BV711 | BioLegend | 400168 | Isotype control |

| | | | | |
|---|---|---|---|---|
| *BV421, BV711: Abbreviations for Brilliant Violet 421 (registered trademark) and Brilliant Violet 711 (registered trademark) | | | | |

The anti-TRBC1 antibody was prepared and purified by the methods described in Example 1 and then dye-labeled by using HiLyte Fluor 647 Labeling Kit-NH2 (Dojindo, LK15). The heavy and light chain sequences of the anti-TRBC1 antibody are the sequences of SEQ ID NOS: 4 and 5.

Human IgG (ChromPure Human IgG, whole molecule, Jackson Immuno Research, 009-000-003) was similarly dye-labeled as an isotype control.

### Methods

The experiment was performed with reference to WO2021/173896 A1.

### Method for cell surface staining

Human PBMCs or the T-LAK cells were centrifuged (300 g x 5 minutes), then the supernatant was removed, and Clear Back was added. After incubation at room temperature for 5 minutes, various antibodies were added in Flow Cytometry Staining Buffer, and allowed to react at 4°C for 30 minutes. After the reaction, the cells were washed twice by adding Flow Cytometry Staining Buffer, centrifuging (500 g x 2 minutes) the suspension, and discarding the supernatant, and flow cytometric analysis was performed by using BD LSRFortessa X-20 (BD Biosciences) to quantify signals. Flow Jo_v10.6.1 was used as the analysis software.

### Method for intracellular staining

Human PBMCs or the T-LAK cells were centrifuged (300 g x 5 minutes), then the supernatant was removed, and Clear Back was added. After incubation at room temperature for 5 minutes, and anti-CD3 and anti-TRBC1 antibodies were added in Flow Cytometry Staining Buffer, and allowed to react at 4°C for 30 minutes. After the reaction, the cells were washed twice by adding Flow Cytometry Staining Buffer, centrifuging (500g x 2 minutes) the cell suspension, and discarding the supernatant. TF Fix/Perm Buffer contained in the Transcription Factor Buffer Set was added to the cells, and incubation was performed on ice for 30 minutes to fix and permeabilize the cells. After centrifugation (700g x 2 minutes), the cells were washed twice by adding TF Perm/Wash Buffer, centrifuging (700g x 2 minutes) the cell suspension, and discarding the supernatant. Anti-granzyme B or anti-perforin antibody was added in TF Perm/Wash Buffer, and the reaction was allowed for 30 minutes on ice. After the reaction, the cells were washed twice by adding TF Perm/Wash Buffer, centrifuging (700 g x 2 minutes) the cell suspension, and discarding the supernatant, and flow cytometric analysis was performed by using BD LSRFortessa X-20 (BD Biosciences) to quantify signals. Flow Jo_v10.6.1 was used as the analysis software.

### Method for quantifying released cytokines

After culturing the T-LAK cells for 24 hours with the anti-TRBC1 antibody, the amounts of various cytokines released were quantified by using MILLIPLEX Human Cytokine/Chemokine/Growth Factor Panel A. The T-LAK cells were washed once with the RPMI-1640 medium (culture medium) containing 10% FBS, and then the appropriate number of the cells was counted and added to a 384-well plate. The anti-TRBC1 antibody adjusted to a final concentration of 0, 0.1, 1.0, or 10 nM in the culture medium was added to the wells in which the T-LAK cells were prepared. The 384-well plate was incubated at 5% CO₂, 37°C for 24 hours, and centrifuged (300 g, 5 minutes), and then the supernatant was collected. IFN-γ, IL-2, IL-6, IL-10, and TNF-α were quantified by using MILLIPLEX Human Cytokine/Chemokine/Growth Factor Panel A. Quantified values were automatically calculated on the Luminex (registered trademark) 200xPONET (registered trademark) 3.1 system.

### Results

### TRBC1-positive cell ratio

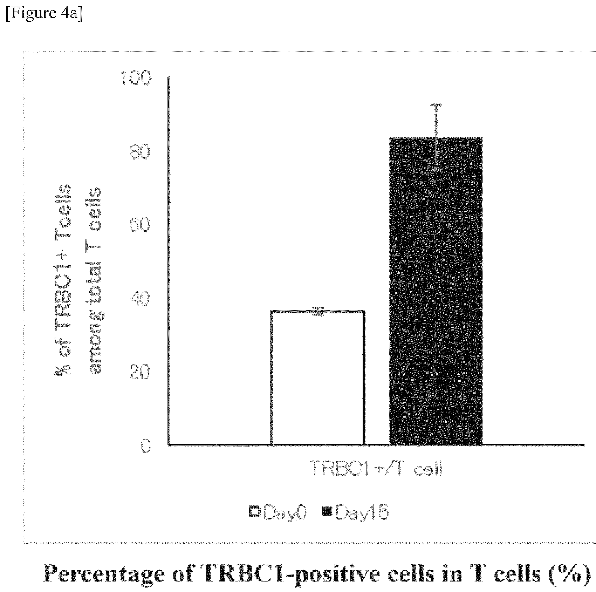

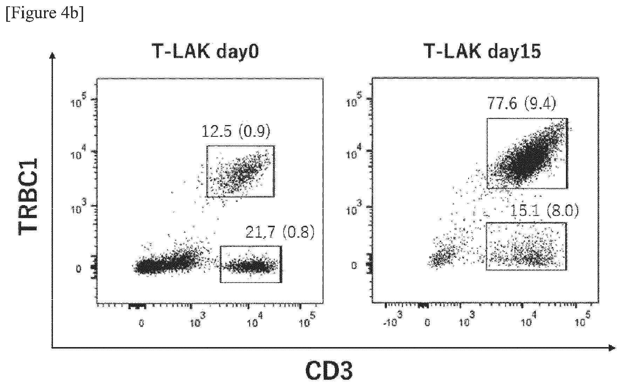

Figs. 4A and 4B show the increase in the ratio of TRBC1-positive cells after the addition of the anti-TRBC1 antibody. When 10 nM anti-TRBC1 antibody was added to human PBMCs and the cells were cultured in the presence of IL-2, the ratio of TRBC1-positive cells increased from 30% on day 0 to over 80% on day 15. It was suggested that the addition of anti-TRBC1 antibody selectively proliferated TRBC1-positive cells. It is considered that addition of a bispecific antibody having the same antigen-binding portion as the anti-TRBC1 antibody used in this experiment would similarly selectively proliferate TRBC1-positive cells.

### Increase in cytotoxic T cells

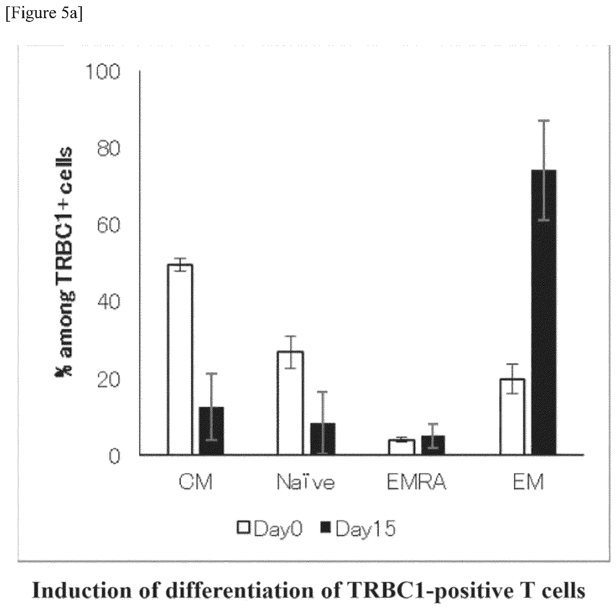

As shown in Figs. 5A and 5B, the T-LAK cells were stained with CD27 and CD45RA on day 0 and day 15, and the cell ratios of the effector memory, central memory, naive, and EMRA fractions were calculated. As a result, on day 15, the ratio of the effector memory cells significantly increased to about 70% from about 20%, while the ratios of the other fractions decreased. On the basis of these results, it was considered that the number of T cells that induce cytotoxicity can be increased by stimulation with anti-TRBC1 antibody. It is considered that stimulation with a bispecific antibody that has the same antigen-binding portion as the anti-TRBC1 antibody used in this experiment would similarly increase the number of T cells that induce cytotoxicity.

### Evaluation of T cell activation markers

CD69 is a cell surface glycoprotein that is expressed very early after T or B cell activation, but not expressed on quiescent lymphocytes. Cytotoxic T cells have intracellular granules containing cytotoxic proteins (granzyme B and perforin). Granzyme B is a serine protease that, once delivered into a target cell, cleaves various intracellular substrates including caspases to induce apoptosis of the target cell. Perforin disrupts the plasma membrane of the target cell to facilitate the transport of granzyme B into the target cell.

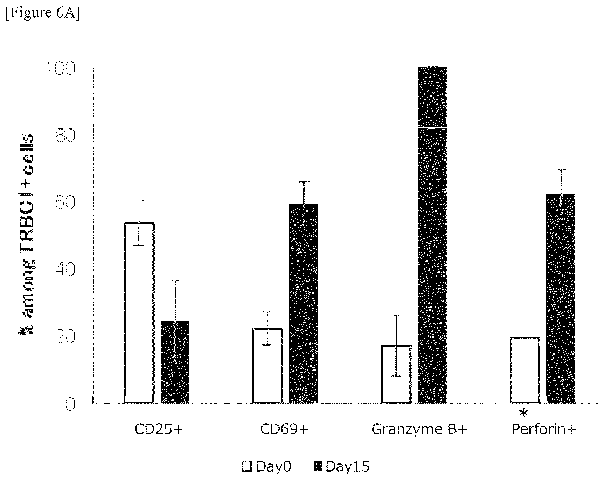

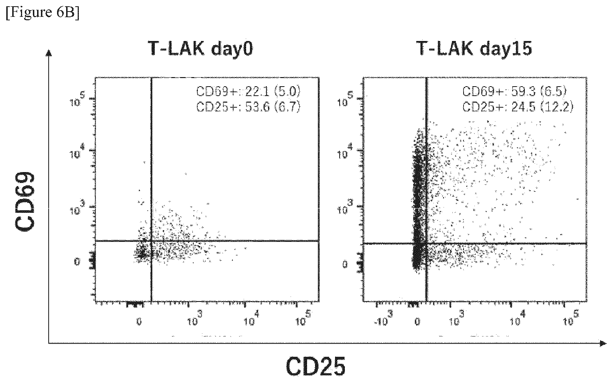

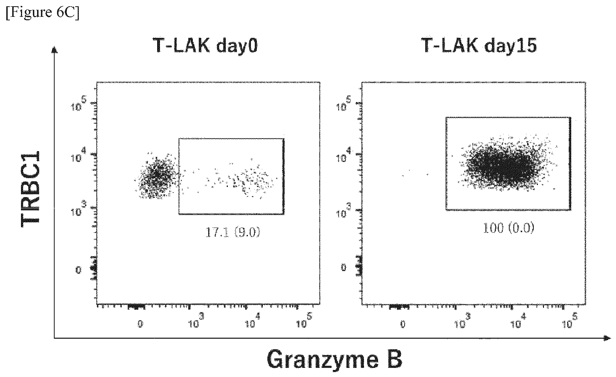

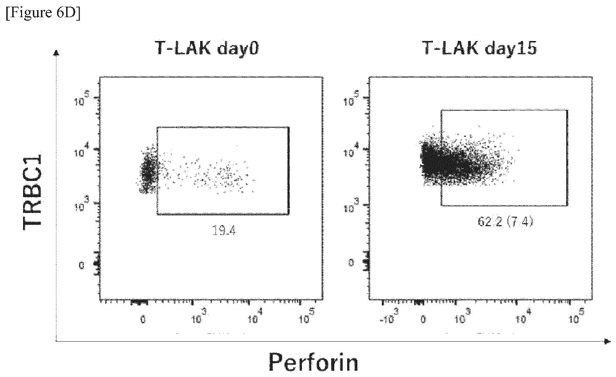

Fig. 6 shows the results of the analysis of the ratios of CD25, CD69, granzyme B, and perforin-positive cells in the T-LAK cells performed on day 0 and day 15.

In this experiment, the ratio of CD25-positive cells decreased, but the ratios of CD69, granzyme B, and perforin-positive cells increased, suggesting that TRBC1-positive cells were activated by the anti-TRBC1 antibody. The same effect is expected for a bispecific antibody having the same antigen-binding portion as the anti-TRBC1 antibody used in this experiment. It was estimated that, in this experiment, IL-2 was added during the culture, and therefore internalization or downregulation of CD25 occurred, resulting in decreased CD25 expression.

### Change in ratio of PD-1-positive cells

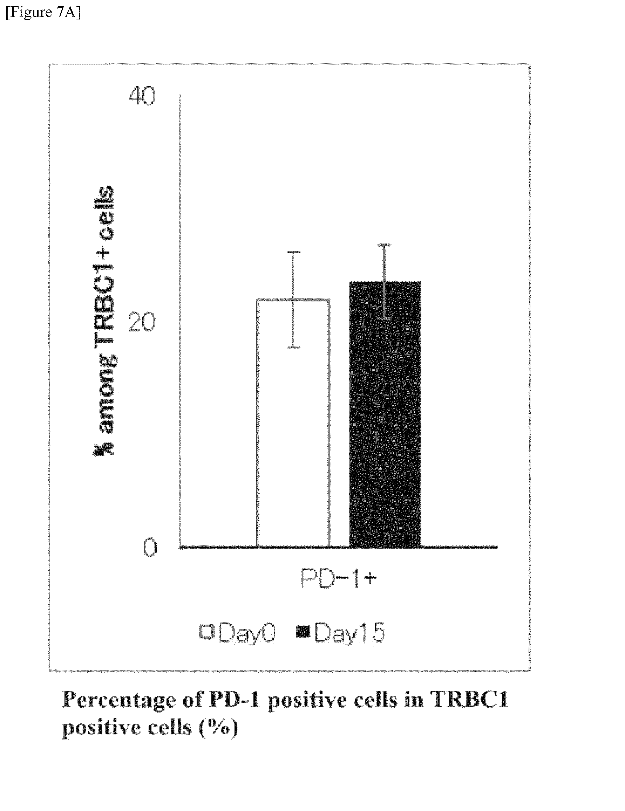

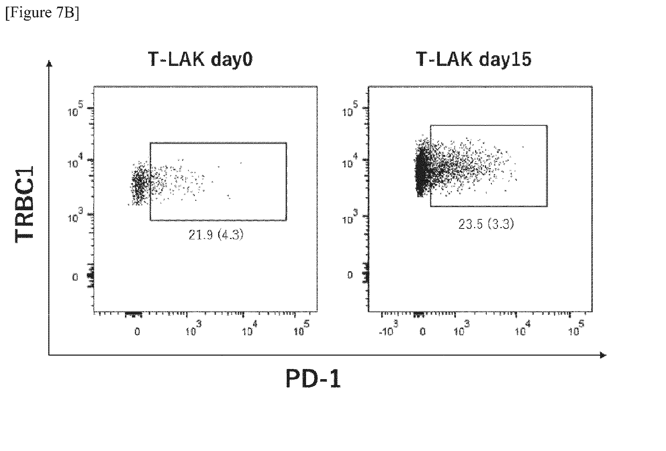

Fig. 7 shows the results of the analysis of the ratio of cells positive for PD-1, one of the immunosuppressive checkpoint molecules in T cells, in the T-LAK cells on day 0 and day 15. Expression of immunosuppressive checkpoint molecules such as PD-1 and TIGIT was scarcely observed in the effector memory (EM) cells of functional cytotoxic T cells, whereas high expression of such immunosuppressive checkpoint molecules as mentioned above is observed in non-functional exhausted T cells. As seen in the results shown in Fig. 7, the ratio of PD-1-positive cells did not change significantly. This suggested that T cells are less likely to be exhausted after T cell activation with anti-TRBC1 antibody. It is considered that T cells are similarly unlikely to be exhausted with a bispecific antibody having the same antigen-binding portion as the anti-TRBC1 antibody used in this experiment.

### Quantification of released cytokines

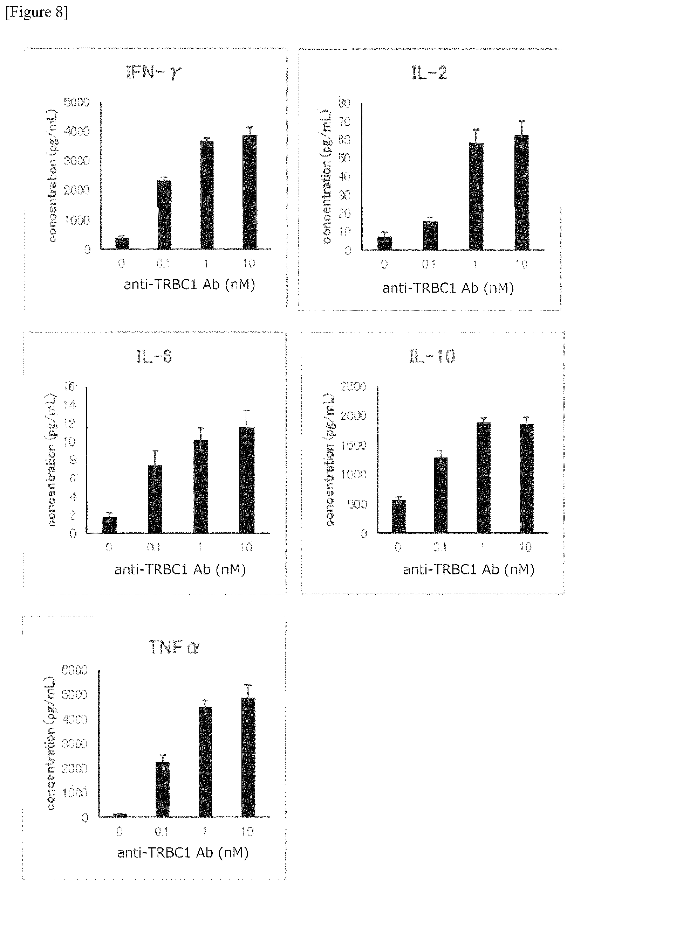

Fig. 8 shows the results of quantification of released amounts of IFN-γ, IL-2, IL-6, IL -10, and TNFα of the T-LAK cells on day 15 after stimulation with 0.1, 1.0, or 10 nM anti-TRBC1 antibody for 24 hours. As a result, antibody concentration-dependent increase in the released amount was observed for all the cytokines, and it was considered that the anti-TRBC1 antibody induced T cell activation and thus cytokines were released. In this experimental system, the released amount of IL-6, an inflammatory cytokine, was very small. These data indicate that the risk of cytokine release syndrome due to anti-TRBC1 antibody may be low. The same low risk of cytokine release syndrome is also suggested for a bispecific antibody having the same antigen-binding portion as the anti-TRBC1 antibody used in this example.

### <Reference Example 2> Evaluation of agonist activity of anti-TRBC2 antibody

An anti-TRBC2 antibody was prepared and purified as described in Example 1. The heavy and light chain sequences of the anti-TRBC2 antibody are shown as SEQ ID NOS: 6 and 7. The reagents and antibodies for immunostaining used in this reference example are the same as those described in Reference Example 1.

### Results

### Number of TRBC2-positive cells

Human PBMCs were lysed and washed on a 37°C water bath. The washed human PBMCs were allowed to react with JOVI-1 mIgG (Santa Cruz) at 4°C for 30 minutes. PBMCs reacted with the antibody were washed, suspended in a medium, and allowed to react with Dynabeads M-280 Anti-Mouse IgG (Invitrogen) at 4°C for 30 minutes. The beads binding unwanted cells were collected on the tube wall by using DynaMag (Invitrogen), and the supernatant containing TRBC2-positive T cells was collected. The collected cells were cultured for 2 days in the RPMI 1640 medium containing 10% FBS supplemented with 10 nM anti-TRBC2 antibody and 100 IU/mL IL-2, then further cultured in the RPMI 1640 medium containing IL-2 and 10% FBS (up to 17 days), and thereby derived into T-LAK. The cells were subcultured every 2 or 3 days, and 100 IU/mL of IL-2 was newly added at the time of culture medium change.

Cell counts were measured at the start of the culture (day 0) and on day 15 by using an automated cell counter (BIO RAD, TC20).

**[Table 6]**

| Culture# | Priming | Culture start date | Cell Count Day0 (×10⁶ cells) | Cell Count Day15 (×10⁶ cells) |
|---|---|---|---|---|
| 1 | hJovi-1-KFN | December 12, 2022 | 6.4 | 19.1 |
| 2 | hJovi-1-KFN | December 12, 2022 | 7.07 | 18.6 |
| 3 | hJovi-1-KFN | January 18, 2023 | 5.68 | 42.3 |

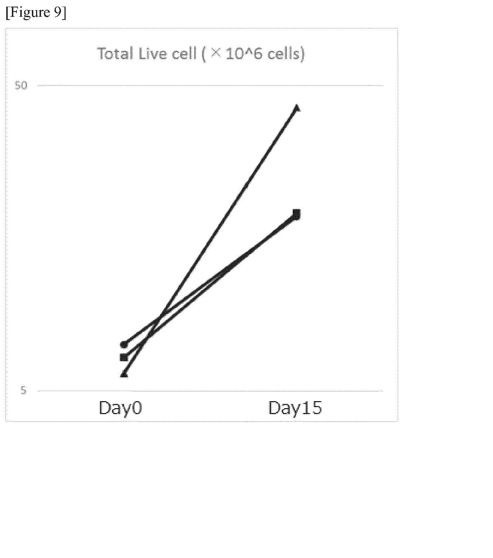

The cell count, about 5 x 10⁶ to 7 x 10⁶ cells, on day 0 increased to about 18 x 10⁶ to 42 x 10⁶ cells on day 15 (Fig. 9). It was suggested that the addition of the anti-TRBC2 antibody selectively proliferated TRBC2-positive cells. It is considered that addition of a bispecific antibody having the same antigen-binding portion as the anti-TRBC2 antibody used in this experiment would similarly selectively proliferate TRBC2-positive cells.

### Increase in cytotoxic T cells

As shown in Fig. 10, the anti-TRBC2 antibody-sensitized T-LAK cells were stained with CD27 and CD45RA on day 0 and day 15, and the cell ratios of the effector memory (EM), central memory (CM), naive, and EMRA fractions were calculated. As a result, on day 15, the ratio of the effector memory cells significantly increased to about 81% from about 27%, while the ratios of the other fractions decreased. These results suggest that stimulation with anti-TRBC2 antibody can increase the number of T cells that induce cytotoxicity. It is considered that stimulation with a bispecific antibody that has the same antigen-binding portion as the anti-TRBC2 antibody used in this experiment may similarly increase the number of T cells that induce cytotoxicity. The CD3-positive and TRBC1-negative cell population was defined as TRBC2-positive cells.

### Evaluation of T cell activation markers

Fig. 11 shows the results of analysis of the ratios of CD25, CD69, granzyme B, and perforin-positive cells in the anti-TRBC2 antibody-sensitized T-LAK cells on day 0 and day 15. In this experiment, the ratio of CD25-positive cells decreased, while the ratios of CD69, granzyme B, and perforin-positive cells increased, suggesting that TRBC2-positive cells were activated by the anti-TRBC2 antibody. The same effect is expected for a bispecific antibody having the same antigen-binding portion as the anti-TRBC2 antibody used in this experiment. It is estimated that, in this experiment, IL-2 was added during the culture, and therefore internalization or downregulation of CD25 occurred, resulting in decreased CD25 expression. The CD3-positive and TRBC1-negative cell population was defined as TRBC2-positive cells.

### Change in ratio of PD-1-positive cells

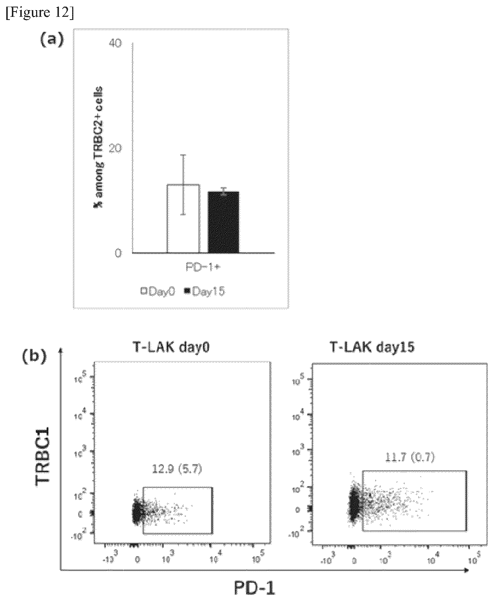

Fig. 12 shows the results of analysis of the ratio of cells positive for PD-1, one of the immunosuppressive checkpoint molecules in T cells, in the anti-TRBC2 antibody-sensitized T-LAK cells on day 0 and day 15. Expression of immunosuppressive checkpoint molecules such as PD-1 and TIGIT was scarcely observed in the effector memory (EM) cells of functional cytotoxic T cells, whereas high expression of such immunosuppressive checkpoint molecules as mentioned above is observed in non-functional exhausted T cells. As seen in the results shown in Fig. 12, the ratio of PD-1-positive cells did not change significantly. This suggests that T cells are less likely to be exhausted when activated with anti-TRBC2 antibody, as in the case of anti-TRBC1 antibody. It is considered that T cells are similarly unlikely to be exhausted by a bispecific antibody having the same antigen-binding portion as that of the anti-TRBC2 antibody used in this experiment.

### Quantification of released cytokines

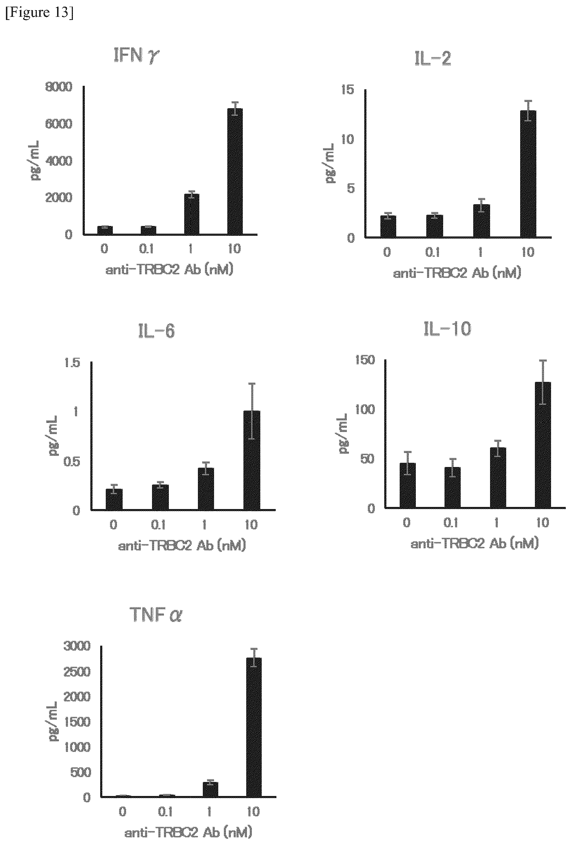

Fig. 13 shows the results of quantification of released amounts of IFN-γ, IL-2, IL-6, IL -10, and TNFα of the T-LAK cells on day 15 after stimulation with 0.1, 1.0, or 10 nM anti-TRBC2 antibody for 24 hours. As a result, antibody concentration-dependent increase in the released amount was observed for all the cytokines, and it was considered that the anti-TRBC2 antibody induced T cell activation and thus the cytokines were released. In this experimental system, the released amount of IL-6, an inflammatory cytokine, was very small. These data indicate that the risk of cytokine release syndrome due to anti-TRBC2 antibody may be low. The same low risk of cytokine release syndrome is also suggested for a bispecific antibody having the same antigen-binding portion as the anti-TRBC2 antibody used in this experiment.

### <Example 3> Cytotoxic activity of anti-CCR4/anti-TRBC1 bispecific antibody

In this example, to evaluate the *in vitro* efficacy of the bispecific antibodies prepared in Example 1, target cells were cultured with the T-LAK cells (effector cells) in the presence of the specific antibodies, and then cell viability of the target cells was measured by using a fluorescent dye (calcein activity) as an indicator to evaluate effector cell-dependent cytotoxicity. The experiment was performed with reference to Neri S et al. (Clin Diagn Lab Immunol. 2001 Nov;8(6):1131-5, of which entire description is specifically incorporated herein by reference).

The following reagents were used
HBSS (Hanks balanced salt solution), no phenol red (Gibco, 14025092)
RPMI 1640 Medium, no phenol red (Gibco, 11835-030)
Probenecid Water Soluble (Invitrogen, P36400)
Calcein-AM (Invitrogen, C1430)
Lysis Solution 10X (Promega, G1821)

### 3.1 Cytotoxic activity of bispecific antibody against MT-2 cells

### Methods

Bispecific antibodies adjusted to a final concentration of 100 nM to 100 fM with RPMI medium, no phenol red (assay medium) containing 5 mM Probenecid and 0.5% FBS were added to a round bottom 96-well culture plate. A well containing only the assay medium was set as a control.

For cytotoxicity assay using bispecific antibodies having a CCR4-binding portion, MT-2 cells (target cells) were collected from the culture medium in a required amount and washed twice with HBSS. The cells were incubated at 37°C for 30 minutes in 10 µM Calcein-AM supplemented with 5 mM Probenecid to label the target cells with calcein. After the incubation, the cells were washed three times with HBSS and suspended in the assay medium after the last washing. After counting the number of labeled target cells, the cells were added to the wells of the round-bottom 96-well culture plate in which bispecific antibodies were prepared. A required amount of the effector cells were collected from the culture medium, washed once with the assay medium, counted, and added to the wells of the round bottom 96-well culture plate in which the bispecific antibodies were prepared. The effector cells and the labeled target cells were added at a ratio of 10:1. The bispecific antibodies, labeled target cells, and effector cells on the plate were allowed to react at 5% CO₂ and 37°C for 2 hours. After the round bottom 96-well culture plate was centrifuged (300 g, 5 minutes), the supernatants were collected, and the fluorescence of calcein eluted from the labeled target cells in the supernatants was measured by using a plate reader (Bio Tek, SYNERGY H1). The cytotoxicity percentage of the target cells was calculated by using the fluorescence value of calcein as an index to evaluate the bispecific antibody-dependent cytotoxicity.

The cytotoxicity percentage was determined as follows.

The minimum eluted fluorescence value (lysis rate = 0%) of the labeled target cells was obtained from a well in which the labeled target cells and effector cells were incubated without any bispecific antibody. The maximum eluted fluorescence value (lysis rate = 100%) was obtained from a well containing the same content as that used for obtaining the minimum eluted fluorescence value and further containing 10x Lysis Solution.

The cytotoxicity percentage was calculated in accordance with the following equation. Cytotoxicity percentage = [(Eluted fluorescence value of target cells in the presence of bispecific antibody) - (Minimum eluted fluorescence value)]/[(Maximum eluted fluorescence value) - (Minimum eluted fluorescence value)]

S-shaped dose-response curves were calculated by using Prism Software (GraphPad Software Inc.), and EC50 values (50% effective concentration) and Emax values (maximum activity value) were calculated.

### Results

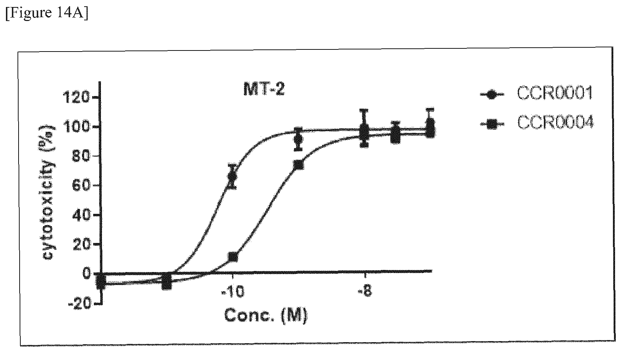

Fig. 14A and the following table show the results of the measurement of the cytotoxicities of the bispecific antibodies CCR0001 and CCR0004 performed by adding the T-LAK cells to the ATL-derived cell strain expressing CCR4, MT-2 cells.

The EC50 and Emax values (N=3) are shown in the following table.

**[Table 7]**

| Antibody name | EC50 (pM) | Standard deviation |
|---|---|---|
| CCR0001 | 44.4 | 22.8 |
| CCR0004 | 262.7 | 95.8 |

| Antibody name | Emax (%) | Standard deviation |
|---|---|---|
| CCR0001 | 106.9 | 12.4 |
| CCR0004 | 106.5 | 13.3 |

It is considered that the anti-CCR4 and anti-TRBC1 bispecific antibodies CCR0001 and CCR0004 exhibit extremely potent cytotoxicities against CCR4-expressing cells.

### 3.2 Cytotoxic activity of negative control antibody against MT-2 cells

Anti-CCR4 antibody, anti-TRBC1 antibody, and combination thereof (anti-CCR4 antibody + anti-TRBC1 antibody) were used as negative control antibodies, and cytotoxicity was measured by the method described above.

The anti-CCR4 antibody used in this example was mogamulizumab. The anti-TRBC1 antibody was prepared and purified by the method described in Example 1. The heavy and light chain sequences of the anti-TRBC1 antibody are shown as SEQ ID NOS: 4 and 5.

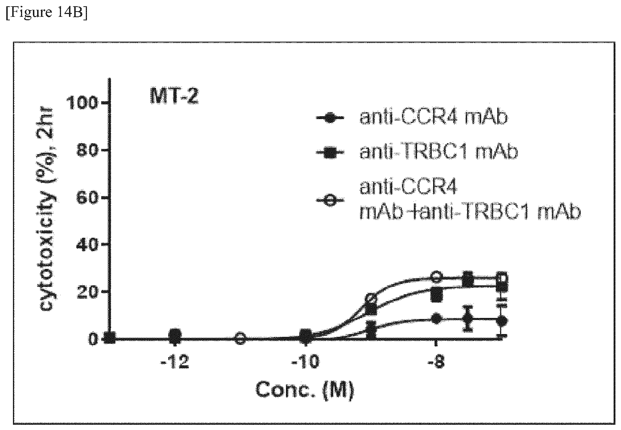

Fig. 14B and the following table show the results obtained by adding the T-LAK cells to the ATL-derived cell strain expressing CCR4, MT-2 cells, and measuring the cytotoxicity of the negative control antibodies.

The EC50 and Emax values (N=3) are shown in the following table.

**[Table 8]**

| Antibody name | EC50 (pM) | Standard deviation |
|---|---|---|
| anti-CCR4 antibody | 1137.8* | NC |
| anti-TRBC1 antibody | 980.4 | 156.5 |
| anti-CCR4 antibody + anti-TRBC1 antibody | 851.7* | NC |

| Antibody name | Emax (%) | Standard deviation |
|---|---|---|
| anti-CCR4 antibody | 8.5* | NC |
| anti-TRBC1 antibody | 23.4 | 3.4 |
| anti-CCR4 antibody + anti-TRBC1 antibody | 29.0 | 3.6 |

| | | |
|---|---|---|
| *For the anti-CCR4 antibody, the values could not be calculated in one time of the experiment performed three times, and therefore the average values for two times of the experiment are shown. | | |

To the ATL-derived cell strain expressing CCR4, MT-2 cells, the T-LAK cells were added, and the anti-CCR4 antibody alone, anti-TRBC1 antibody alone, or both the anti-CCR4 and anti-TRBC1 antibody were added, and cytotoxicity was measured. As a result, the EC50 and Emax values were significantly lower than those obtained with the addition of anti-CCR4/anti-TRBC1 bispecific antibodies CCR0001 and CCR0004. The anti-CCR4 antibody did not exhibit sufficient cytotoxicity in this experimental system. The anti-TRBC1 antibody did not exhibit sufficient cytotoxicity.

### 3.3 Cytotoxic activity of bispecific antibody against negative control cells

Instead of the MT-2 cells, the T cell tumor-derived cell strain that does not express CCR4, MOLT-4 cells, were used as negative control cells to measure the cytotoxicities of the bispecific antibodies CCR0001 and CCR0004. The MOLT-4 cells were labeled with Calcein-AM and suspended in the assay medium in the same manner as used for the MT-2 cells described above.

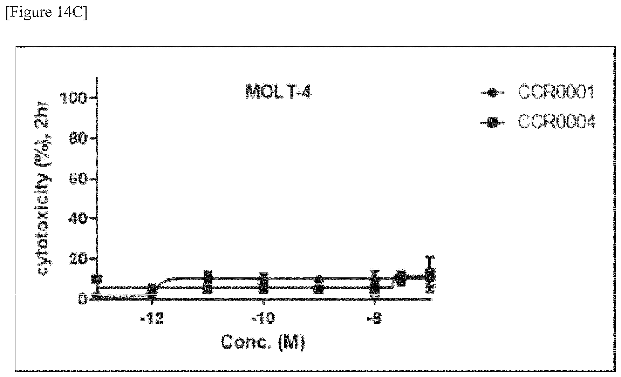

Fig. 14C and the following table show the results obtained by adding the T-LAK cells to the negative control MOLT-4 cells and measuring the cytotoxicities of CCR0001 and CCR0004.

The EC50 and Emax values (N=3) are shown in the following table.

**[Table 9]**

| Antibody name | EC50 (pM) | Standard deviation |
|---|---|---|
| CCR0001 | NC | NC |
| CCR0004 | 3348.4* | NC |

| Antibody name | Emax (%) | Standard deviation |
|---|---|---|
| CCR0001 | 17.2* | NC |
| CCR0004 | 12.5* | NC |

| | | |
|---|---|---|
| *The values could not be calculated in one time of the experiment performed three times, therefore the average values for two times of the experiment are shown, and the SD was not calculated. NC means not calculated. | | |

For the MOLT-4 cells, which are T cell tumor strain cells that do not express CCR4, cytotoxicity was significantly lower in terms of both the EC50 and Emax values compared with the CCR4-expressing cells. These results indicate that the bispecific antibodies CCR0001 and CCR0004 are antibodies that specifically act against CCR4-expressing cells.

### <Example 4> Cytotoxic activity of anti-CD1a/anti-TRBC1 bispecific antibody

In this example, to evaluate the *in vitro* efficacy of the anti-CD1a/anti-TRBC1 bispecific antibodies prepared in Example 1, target CCRF-CEM9 cells were cultured with anti-TRBC1 antibody-sensitized T-LAK cells (effector cells) in the presence of the specific antibodies, and then the cell viability of the target cells were measured by using a fluorescent dye (calcein activity) as an indicator to evaluate effector cell-dependent cytotoxicity.

The target CCRF-CEM9 cells express CD1a and TRBC2, and were obtained by single cell cloning of CCRF-CEM cells, which are TALL-derived cell strain, as described in Example 2.

The measurement of the cytotoxicity was performed by the same method as described in Example 3.

### Results

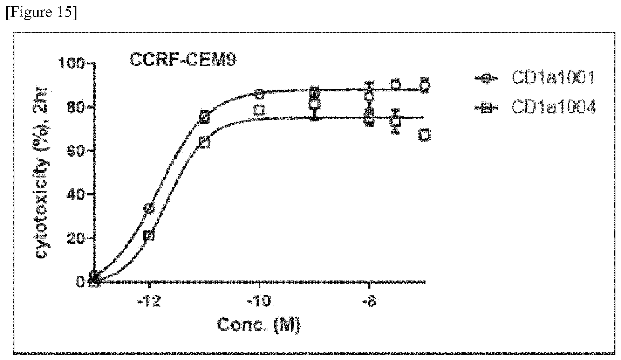

Fig. 15 and the following table show the results obtained by adding the T-LAK cells to the CCRF-CEM9 cells, which are TALL-derived cell strain expressing CD1a, and measuring the cytotoxicities of the bispecific antibodies CD1a1001 and CD1a1004.

The EC50 and Emax values (N=3) are shown in the following table.

**[Table 10]**

| Antibody name | EC50 (pM) | Standard deviation |
|---|---|---|
| CD1a1001 | 2.9 | 1.2 |
| CD1a1004 | 2.9 | 0.8 |

| Antibody name | Emax (%) | Standard deviation |
|---|---|---|
| CD1a1001 | 94.2 | 16.9 |
| CD1a1004 | 77.0 | 18.7 |

The T-LAK cells were added to the CCRF-CEM9 cells, and the cytotoxicities of CD1a1001 and CD1a1004 were measured. As a result, the EC50 values were 2.9 pM for the both, and the Emax values were 94.2% and 77.0%, respectively. On the basis of these results, it was considered that anti-CD1a and anti-TRBC1bispecific antibodies exhibit very potent cytotoxicity against CD1a- and TRBC2-expressing cells.

### <Example 5> Cytotoxic activity of anti-CCR4/anti-TRBC2 bispecific antibody

In this example, to evaluate the *in vitro* efficacy of the anti-CCR4/anti-TRBC2 bispecific antibody prepared in Example 1, target cells MT-2 were cultured with the anti-TRBC2 antibody-sensitized T-LAK cells (effector cells) in the presence of the bispecific antibody, and cell viability of the target cells was measured by using a fluorescent dye (calcein activity) as an indicator to evaluate the effector cell-dependent cytotoxicity.

### Results

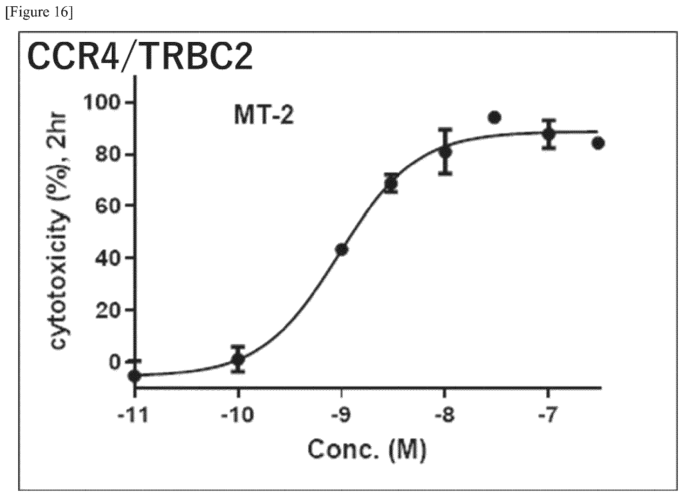

Fig. 16 shows the results obtained by adding the anti-TRBC2 antibody-sensitized T-LAK cells to the MT-2 cells, a ATL-derived cell strain expressing CCR4, and measuring the cytotoxicity of CCR0010. The EC50 and Emax values (N=3) are shown in the following table.

**[Table 11]**

| Antibody name | EC50 (pM) | Standard deviation |
|---|---|---|
| CCR0010 | 1677.0 | 1063.6 |

| Antibody name | Emax (%) | Standard deviation |
|---|---|---|
| CCR0010 | 81.4 | 13.4 |

To the MT-2 cells, the anti-TRBC2 antibody-sensitized T-LAK cells were added, and the cytotoxicity of CCR0010 was measured. As a result, CCR0010, a bispecific antibody recognizing CCR4 and TRBC2, showed the activity in terms of EC50 of 1677.0 pM and Emax of 81.4% against the CCR4-expressing cells. Therefore, it was considered that a bispecific antibody recognizing CCR4 and TRBC2 exhibits cytotoxicity against the CCR4-expressing ATL cell strain, like the bispecific antibody recognizing CCR4 and TRBC1.

### <Example 6> Cytotoxic activity of anti-CD1a/anti-TRBC2 bispecific antibody

In this example, to evaluate the *in vitro* efficacy of the anti-CD1a/anti-TRBC2 bispecific antibodies prepared in Example 1, target JM cells were cultured with the anti-TRBC2 antibody-sensitized T-LAK cells (effector cells, prepared by the method described in Reference Example 2) in the presence of the bispecific antibodies, and cell viability of the target cells was measured by using a fluorescent dye (calcein activity) as an indicator to evaluate effector cell-dependent cytotoxicity. The measurement of the cytotoxicity was performed in the same method as described in Example 3. The target JM cells were TALL-derived cell strain cells expressing CD1a and TRBC1. The JM cells were purchased from the RIKEN BioResource Research Center (RCB0537), cultured in the RPMI medium containing 10% FBS in a 5% CO₂, 37°C incubator, and subcultured two to three times per week.

### Results

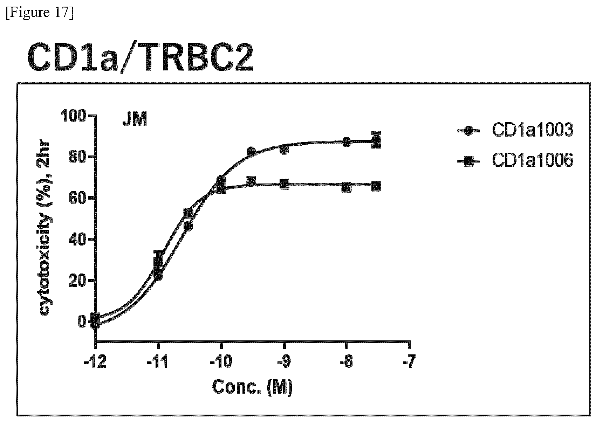

Fig. 17 shows the results obtained by adding the anti-TRBC2 antibody-sensitized T-LAK cells to the JM cells, TALL-derived cell strain expressing CD1a, and measuring the cytotoxicities of the bispecific antibodies CD1a1003 and CD1a1006.

The EC50 and Emax values (N=3) are shown in the following table.

**[Table 12]**

| Antibody name | EC50 (pM) | Standard deviation |
|---|---|---|
| CD1a1003 | 22.5 | 12.2 |
| CD1a1006 | 12.9 | 7.6 |

| Antibody name | Emax (%) | Standard deviation |
|---|---|---|
| CD1a1003 | 90.1 | 9.6 |
| CD1a1006 | 70.1 | 6.5 |

The T-LAK cells were added to the JM cells, and the cytotoxicities of CD1a1003 and CD1a1006 were measured. As a result, EC50 values were 22.5 pM and 12.9 pM, respectively, and Emax values were 90.1% and 70.1%, respectively. On the basis of these results, it was considered that anti-CD1a and anti-TRBC2 bispecific antibodies exhibit very potent cytotoxicity against CD1a- and TRBC1-expressing cells.

### <Example 7> Cytotoxic activity of anti-CCR9/anti-TRBC1 bispecific antibody

In this example, to evaluate the *in vitro* efficacy of the anti-CCR9/anti-TRBC1 bispecific antibodies prepared in Example 1, target CCRF-CEM9 cells were cultured with the T-LAK cells (effector cells) in the presence of the specific antibodies, and then the cell viability of the target cells was measured by using a fluorescent dye (calcein activity) as an indicator to evaluate effector cell-dependent cytotoxicity. The target CCRF-CEM9 cell express CCR9 and TRBC2, which were obtained by single cell cloning of the CCRF-CEM cells, a TALL-derived cell strain, as described in Example 2. The measurement of the cytotoxicity was performed by the same method as described in Example 3.

### Results

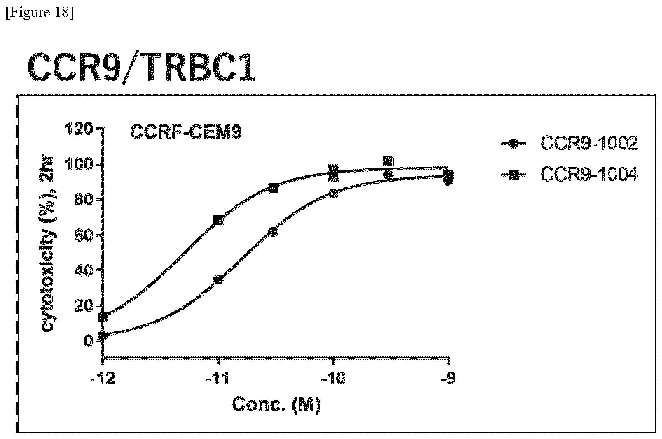

Fig. 18 shows the results obtained by adding the T-LAK cells to the CCRF-CEM9 cells, a TALL-derived cell strain expressing CCR9, and measuring the cytotoxicities of the bispecific antibodies CCR9-1002 and CCR9-1004.

The EC50 and Emax values (N=3) are shown in the following table.

**[Table 13]**

| Antibody name | EC50 (pM) | Standard deviation |
|---|---|---|
| CCR9-1002 | 29.1 | 9.3 |
| CCR9-1004 | 6.6 | 3.1 |

| Antibody name | Emax (%) | Standard deviation |
|---|---|---|
| CCR9-1002 | 85.9 | 7.4 |
| CCR9-1004 | 94.5 | 6.7 |

The T-LAK cells were added to the CCRF-CEM9 cells and the cytotoxicities of CCR9-1002 and CCR9-1004 were measured. As a result, EC50 values were 29.1 pM and 6.6 pM, respectively, and Emax values were 85.9% and 94.5%, respectively. On the basis of these results, it was considered that anti-CCR9 and anti-TRBC1 bispecific antibodies exhibit very potent cytotoxicity against CCR9- and TRBC2-expressing cells.

### <Example 8> Cytotoxic activity of anti-CXCR4/anti-TRBC1 bispecific antibody

In this example, to evaluate the *in vitro* efficacy of the anti-CXCR4/anti-TRBC1 bispecific antibodies prepared in Example 1, target CCRF-CEM9 cells were cultured with the T-LAK cells (effector cells) in the presence of the specific antibodies, and then the cell viability of the target cells was measured by using a fluorescent dye (calcein activity) as an indicator to evaluate effector cell-dependent cytotoxicity. The target CCRF-CEM9 cell express CXCR4 and TRBC2 and were obtained by single cell cloning of the CCRF-CEM cells, a TALL-derived cell strain, as described in Example 2. The measurement of the cytotoxicity was performed by the same method as described in Example 3.

### Results

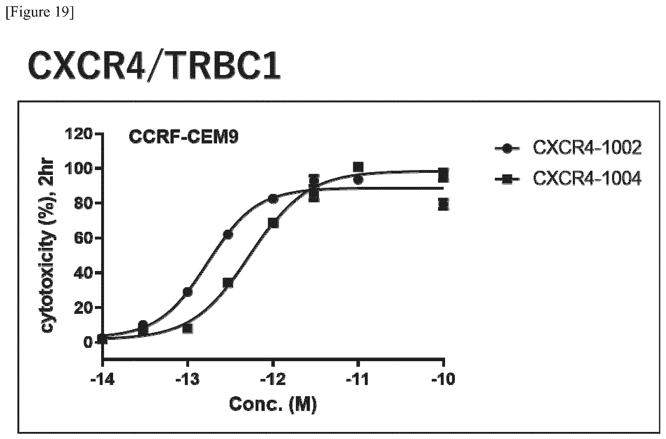

Fig. 19 shows the results obtained by adding the T-LAK cells to the CCRF-CEM9 cells, a TALL-derived cell strain expressing CXCR4, and measuring the cytotoxicities of the bispecific antibodies CXCR4-1002 and CXCR4-1004.

The EC50 and Emax values (N=3) are shown in the following table.

**[Table 14]**

| Antibody name | EC50 (pM) | Standard deviation |
|---|---|---|
| CXCR4-1002 | 0.31 | 0.28 |
| CXCR4-1004 | 0.73 | 0.46 |

| Antibody name | Emax (%) | Standard deviation |
|---|---|---|
| CXCR4-1002 | 88.3 | 7.8 |
| CXCR4-1004 | 95.6 | 8.4 |

The T-LAK cells were added to the CCRF-CEM9 cells, and the cytotoxicities of CXCR4-1002 and CXCR4-1004 were measured. As a result, EC50 values were 0.31 pM and 0.73 pM, respectively, and Emax values were 88.3% and 95.6%, respectively. On the basis of these results, it was considered that anti-CXCR4/anti-TRBC1 bispecific antibodies show very potent cytotoxicity against CXCR4 and TRBC2-expressing cells.

### <Example 9> Cytotoxic activity of anti-CCR8/anti-TRBC1 bispecific antibody

In this example, to evaluate the *in vitro* efficacy of the anti-CCR8/anti-TRBC1 bispecific antibodies prepared in Example 1, target cells were cultured with the T-LAK cells (effector cells) in the presence of the specific antibodies, and then cell viability of the target cells was measured by using a fluorescent dye (calcein activity) as an indicator to evaluate effector cell-dependent cytotoxicity. The target MT-1 cells are an ATL-derived cell strain expressing CCR8. The measurement of the cytotoxicity was performed by the same method as described in Example 3. The MT-1 cells were purchased from the JCRB Cell Bank (National Institute of Biomedical Innovation, Health and Nutrition, JCRB1209). The MT-1 cells were cultured by using the RPMI medium containing 10% FBS in a 5% CO₂, 37°C incubator, and subcultured 2 or 3 times per week.

### Results

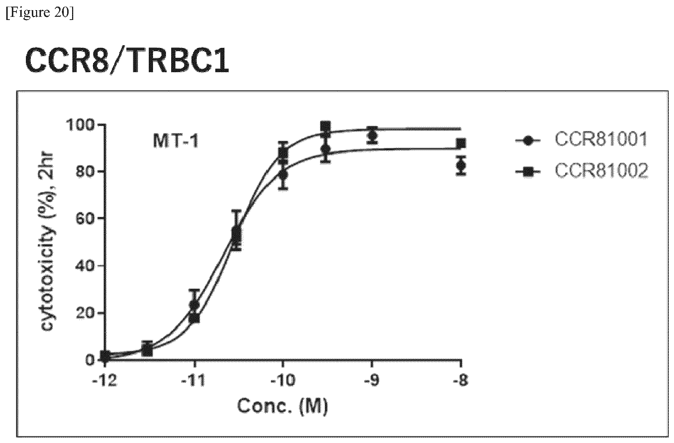

Fig. 20 shows the results obtained by adding the T-LAK cells to the MT-1 cells, an ATL-derived cell strain expressing CCR8, and measuring the cytotoxicities of CCR81001 and CCR81002. The EC50 and Emax values (N=3) are shown in the following table.

**[Table 15]**

| Antibody name | EC50 (pM) | Standard deviation |
|---|---|---|
| CCR81001 | 23.5 | 2.2 |
| CCR81002 | 31.4 | 5.0 |

| Antibody name | Emax (%) | Standard deviation |
|---|---|---|
| CCR81001 | 89.9 | 0.6 |
| CCR81002 | 97.1 | 1.7 |

The T-LAK cells were added to the MT-1 cells and the cytotoxicities of CCR81001 and CCR81002 were measured. As a result, EC50 values were 23.5 pM and 31.4 pM, respectively, and Emax values were 89.9% and 97.1%, respectively. On the basis of these results, it was considered that the bispecific antibodies CCR81001 and CCR81002, which recognize CCR8 and TRBC1, show very potent cytotoxicity against CCR8-expressing cells.

### <Example 10> Cytotoxic activity of anti-TIGIT/anti-TRBC1 bispecific antibody

In this example, to evaluate the *in vitro* efficacy of the anti-TIGIT/anti-TRBC1 bispecific antibodies prepared in Example 1, target cells were cultured with the T-LAK cells (effector cells) in the presence of the specific antibodies, and then cell viability of the target cells was measured by using a fluorescent dye (calcein activity) as an indicator to evaluate effector cell-dependent cytotoxicity. The target ILT-Mat cells are an ATL-derived strain expressing TIGIT. The cytotoxicity was measured in the same manner as described in Example 3. The ILT-Mat cells were purchased from the RIKEN BioResource Research Center (RCB0475). The ILT-Mat cells were cultured by using the RPMI medium containing 320 IU/mL of IL-2 and 10% FBS in a 5% CO₂, 37°C incubator and subcultured 2 or 3 times per week.

### Results

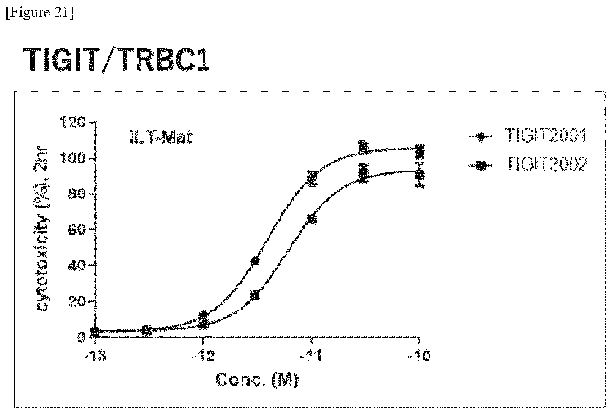

Fig. 21 shows the results obtained by adding the T-LAK cells to the ILT-Mat cells, an ATL-derived cell strain expressing TIGIT, and measuring the cytotoxicities of TIGIT2001 and TIGIT2002. The EC50 and Emax values (N=3) are shown in the following table.

**[Table 16]**

| Antibody name | EC50 (pM) | Standard deviation |
|---|---|---|
| TIGIT2001 | 3.4 | 0.5 |
| TIGIT2002 | 5.6 | 0.3 |

| Antibody name | Emax (%) | Standard deviation |
|---|---|---|
| TIGIT2001 | 105.1 | 6.8 |
| TIGIT2002 | 91.9 | 6.4 |

The T-LAK cells were added to the ILT-Mat cells and the cytotoxicities of TIGIT 2001 and TIGIT 2002 were measured. As a result, EC50 values were 3.4 pM and 5.6 pM, respectively, and Emax values were 105.1% and 91.9%, respectively. On the basis of these results, it was considered that TIGIT2001 and TIGIT2002, bispecific antibodies recognizing TIGIT and TRBC1, exhibit very potent cytotoxicity against TIGIT-expressing cells.

### <Example 11> Cytotoxic activity of anti-CD30/anti-TRBC1 bispecific antibody

In this example, to evaluate the *in vitro* efficacy of the anti-CD30/anti-TRBC1 bispecific antibodies prepared in Example 1, target cells were cultured with the T-LAK cells (effector cells) in the presence of the specific antibodies, and cell viability of the target cells was measured by using a fluorescent dye (calcein activity) as an indicator to evaluate effector cell-dependent cytotoxicity. The target DL40 cells are a PTCL-derived cell strain expressing CD30. The cytotoxicity was measured by the same method as described in Example 3. The DL40 cells were purchased from the JCRB Cell Bank (National Institute of Biomedical Innovation, National Institute of Health and Nutrition, JCRB1337). The DL40 cells were cultured in the RPMI medium containing 10% FBS in a 5% CO₂, 37°C incubator, and subcultured 2 or 3 times per week.

### Results

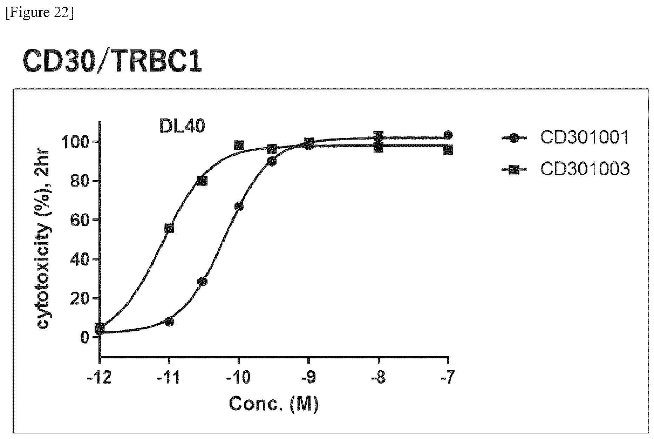

Fig. 22 shows the results obtained by adding the T-LAK cells to the DL40 cells, a PTCL-derived cell strain expressing CD30, and measuring the cytotoxicities of CD301001 and CD301003. The EC50 and Emax values (N=3) are shown in the following table.

**[Table 17]**

| Antibody name | EC50 (pM) | Standard deviation |
|---|---|---|
| CD301001 | 151.1 | 95.4 |
| CD301003 | 14.5 | 5.8 |

| Antibody name | Emax (%) | Standard deviation |
|---|---|---|
| CD301001 | 89.1 | 12.6 |
| CD301003 | 95.7 | 2.2 |

The T-LAK cells were added to the DL40 cells and the cytotoxicities of CD301001 and CD301003 were measured. As a result, the EC50 values were 151.1 pM and 14.5 pM, respectively, and Emax values were 89.1% and 95.7%, respectively. On the basis of these results, it was considered that CD301001 and CD301003, bispecific anti-CD30 and anti-TRBC1 antibodies, exhibit very potent cytotoxicity against CD30-expressing cells.

### <Example 12> Cytotoxic activity of anti-CD40L/anti-TRBC1 bispecific antibody

In this example, to evaluate the *in vitro* efficacy of the anti-CD40L/anti-TRBC1 bispecific antibodies prepared in Example 1, target cells were cultured with the T-LAK cells (effector cells) in the presence of the specific antibodies, and cell viability of the target cells was measured by using a fluorescent dye (calcein activity) as an indicator to evaluate effector cell-dependent cytotoxicity. The target MOLT3 cells are a TALL-derived cell strain expressing CD40L (CD154). The cytotoxicity was measured by the same method as described in Example 3. The MOLT3 cells were purchased from RIKEN BioResource Research Center (RCB9048). The MOLT3 cells were cultured in the RPMI medium containing 10% FBS in a 5% CO₂, 37°C incubator and subcultured two to three times per week.

### Results

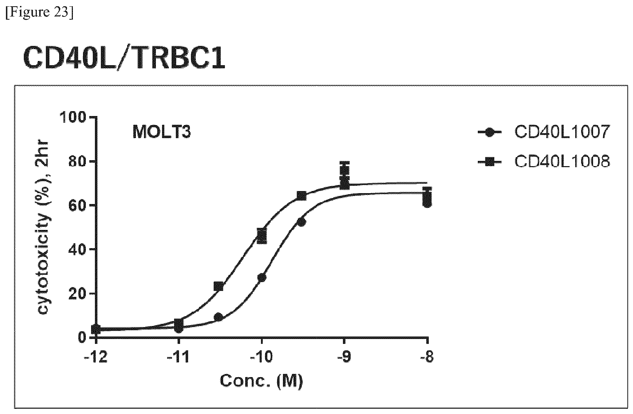

Fig. 23 shows the results obtained by adding the T-LAK cells to the MOLT3 cells, a TALL-derived cell strain expressing CD40L (CD154), and measuring the cytotoxicities of CD40L1007 and CD40L1008. The EC50 and Emax values (N=3) are shown in the following table.

**[Table 18]**

| Antibody name | EC50 (pM) | Standard deviation |
|---|---|---|
| CD40L1007 | 125.0 | 8.2 |
| CD40L1008 | 49.1 | 7.7 |

| Antibody name | Emax (%) | Standard deviation |
|---|---|---|
| CD40L1007 | 67.7 | 1.7 |
| CD40L1008 | 72.7 | 2.2 |

The T-LAK cells were added to the MOLT3 cells and the cytotoxicities of CD40L1007 and CD40L1008 were measured. As a result, EC50 values were 125.0 pM and 49.1 pM, respectively, and Emax values were 67.7% and 72.7%, respectively. On the basis of these results, it was considered that CD40L1007 and CD40L1008, anti-CD40L and anti-TRBC1 bispecific antibodies, exhibit very potent cytotoxicity against CD40L (CD154)-expressing cells.

### <Example 13> Comparison of anti-CCR4/anti-TRBC1 bispecific antibody and mogamulizumab

In this example, the *in vitro* efficacies of the anti-CCR4/anti-TRBC1 bispecific antibody CCR0001 prepared in Example 1 and the anti-CCR4 monoclonal antibody, mogamulizumab, were evaluated in MOLT4 cells forced to express CCR4. The MOLT4 cells were purchased from the JCRB Cell Bank (National Institutes of Biomedical Innovation, Health and Nutrition, JCRB9031). The cells were cultured in the RPMI 1640 medium containing 10% FBS in a 5% CO₂, 37°C incubator and subcultured two to three times per week. The target CR4-expressing MOLT4 cells were prepared by transfection of a CCR4-expressing plasmid into the MOLT4 cells that did not express CCR4 using electroporation. Four types of cells showing different expression levels could be established and these were used for the evaluation.

### Evaluation of anti-CCR4/anti-TRBC1 bispecific antibody

The target cells, CCR4-expressing MOLT4 cells, were cultured with the T-LAK cells (effector cells) in the presence of the bispecific antibody CCR0001, and then cell viability of the target cells was measured by using a fluorescent dye (calcein activity) as an indicator to evaluate effector cell-dependent cytotoxicity.

### Evaluation of anti-CCR4 monoclonal antibody, mogamulizumab

The target cells, CCR4-expressing MOLT4 cells, were cultured with NK cells (effector cells) isolated from peripheral blood of healthy subjects in the presence of mogamulizumab, and cell viability of the target cells was measured by using a fluorescent dye (calcein activity) as an indicator to evaluate effector cell-dependent cytotoxicity.

### Results

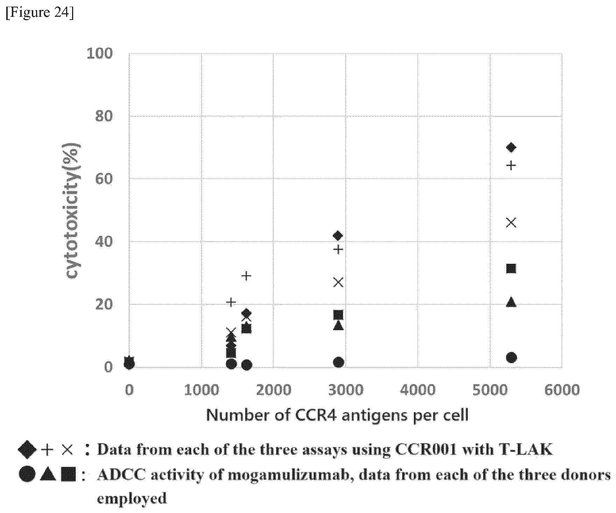

Fig. 24 shows the results obtained by adding the T-LAK or NK cells to the CCR4-expressing MOLT4 cells, and measuring the cytotoxicities of the anti-TRBC1 bispecific antibody CCR0001 and mogamulizumab. For all of the four types of CCR4-expressing MOLT4 cells, CCR0001 showed higher cytotoxicity than mogamulizumab, suggesting that the anti-CCR4 and anti-TRBC1 bispecific antibody is more effective than the existing drug, mogamulizumab.

### <Example 14> Evaluation of cytotoxicity of bispecific antibody employing anti-CD3 antibody in T cell tumor

CD3 is similarly expressed in normal T cells and tumor T cells. Bispecific antibodies targeting CD3 and tumor antigen simultaneously bind to the CD3 antigens expressed in T cell malignant tumor and the target antigens expressed on T cell malignant tumor to link the malignant tumor cells with one another (Fig. 1A), making the therapeutic effect of the bispecific antibody highly likely to be insufficient.

In this example, cytotoxicity of a bispecific antibody employing anti-CD3 antibody against CD3-expressing tumor cell strain and CD3-knockout tumor cell strain was evaluated. Further, a similar experiment was performed by using a bispecific antibody employing anti-TRBC1 antibody.

Anti-CD1a/anti-CD3 bispecific antibody CD1a1016 and anti-CD1a/anti-TRBC1 bispecific antibody CD1a1005 were prepared by the method described in Example 1. The CD1a-binding portions of CD1a1016 and CD1a1005 contain the same amino acid sequence. The CD3-binding portion of CD1a1016 was designed on the basis of the anti-CD3 antibody described in the Unites States Patent No. 5,821,337, of which entire description is specifically incorporated herein by reference.

**[Table 19]**

| Antibody name | Antibody format | Tumor antigen | T cell antigen | | Amino acid sequence of heavy chain | | Amino acid sequence of light chain |
|---|---|---|---|---|---|---|---|
| CD1a1005 | Fab-scFv-Fc | CD1a | TRBC1 | SEQ ID NO: 87 | CD1a1005 VH | SEQ ID NO: 29 | CD1a1003 VL |
| | | | | | | | (CD1a1001 VL) |
| CD1a1016 | Fab-scFv-Fc | CD1a | CD3 | SEQ ID NO: 88 | CD1a1016 VH | SEQ ID NO: 29 | CD1a1003 VL |
| | | | | | | | (CD1a1001 VL) |

Target HPB-ALL and HPB-ALL CD3 KO cells were cultured with the T-LAK cells (effector cells) in the presence of each bispecific antibody, and then cell viability of the target cells was measured by using a fluorescent dye (calcein activity) as an indicator to evaluate effector cell-dependent cytotoxicity. The target HPB-ALL cells express CD1a, CD3 and TRBC2. The target HPB-ALL CD3 KO cells are a cell strain obtained by knocking out the CD3 gene of the HPB-ALL cells, a TALL-derived cell strain, using the CRSPR-Cas9 method. The measurement of the cytotoxicity was performed by the same method as described in Example 3. The target HPB-ALL cells were purchased from RIKEN BioResource Research Center (RCB1935). The cells were cultured in the RPMI 1640 medium containing 10% FBS in a 5% CO₂, 37°C incubator and subcultured two to three times per week.

### Results

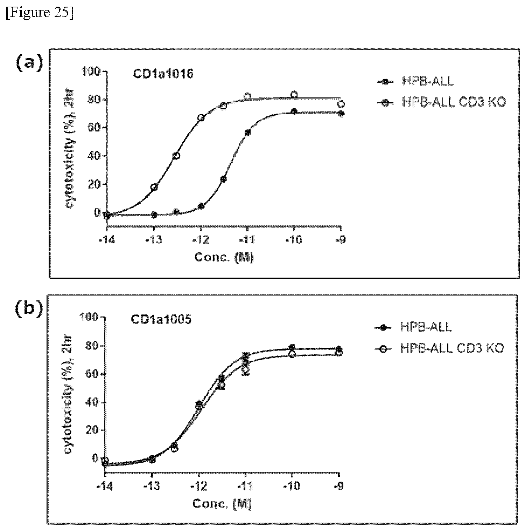

Fig. 25 shows the results obtained by adding the T-LAK cells to the HPB-ALL cells, a TALL-derived cell strain expressing CD3, and HPB-ALL CD3 KO cells, in which CD3 was knocked out, and measuring the cytotoxicities of the anti-CD1a/anti-CD3 bispecific antibody CD1a1016 and anti-CD1a/anti-TRBC1 bispecific antibody CD1a1005.

The EC50 values and Emax values (N=3) are shown in the following table.

**[Table 20]**

| CD1a1016 | | |
|---|---|---|
| Target cell name | EC50 (pM) | Standard deviation |
| HPB-ALL | 9.7 | 3.9 |
| HPB-ALL CD3 KO | 1.0 | 0.5 |

| Target cell name | Emax (%) | Standard deviation |
|---|---|---|
| HPB-ALL | 71.9 | 13.3 |
| HPB-ALL CD3 KO | 85.3 | 13.1 |

### CD1a1005

| Target cell name | EC50 (pM) | Standard deviation |
|---|---|---|
| HPB-ALL | 3.6 | 1.9 |
| HPB-ALL CD3 KO | 5.3 | 3.0 |

| Target cell name | Emax (%) | Standard deviation |
|---|---|---|
| HPB-ALL | 76.5 | 11.9 |
| HPB-ALL CD3 KO | 74.5 | 13.1 |

The EC50 of the anti-CD1a/anti-CD3 bispecific antibody CD1a1016 was about 10-fold higher and the Emax was lower when CD3 was expressed in the target cells compared with those observed when CD3 was not expressed. On the basis of these results, it was considered that when CD3 is expressed on the target cell side, the cytotoxicity of bispecific antibody employing anti-CD3 antibody on the effector cell side is attenuated.

The EC50 and Emax values of the anti-CD1a/anti-TRBC1 bispecific antibody CD1a1005 did not significantly change depending on the target cells.

### <Example 15> Cytotoxic activity of bispecific antibody employing anti-TRBC2 antibody against TRBC1-expressing tumor cell and TRBC2-expressing tumor cell

In this example, the cytotoxicities of the anti-CD1a/anti-TRBC2 bispecific antibody CD1a1006, prepared in Example 1, against TRBC1-positive tumor cell strain and TRBC2-positive tumor cell strain were compared.

The target CCRF-CEM9 cells and JM cells were cultured with the anti-TRBC2 antibody-sensitized T-LAK cells (effector cells) in the presence of the bispecific antibody, and cell viability of the target cells was measured by using a fluorescent dye (calcein activity) as an indicator to evaluate effector cell-dependent cytotoxicity. The target CCRF-CEM9 cells are a TALL-derived cell strain expressing CD1a and TRBC2. The target JM cells are a TALL-derived cell strain expressing CD1a and TRBC1. The measurement of the cytotoxicity was performed by the same method as described in Example 3.

The cytotoxicity of the anti-CD1a/anti-TRBC1 bispecific antibody against the TRBC2-positive tumor cell strain CCRF-CEM9 was evaluated in Example 4. The cytotoxicity of the anti-CD1a/anti-TRBC2 bispecific antibody CD1a1006 against the TRBC1-positive tumor cell strain JM was also measured in Example 6.

### Results

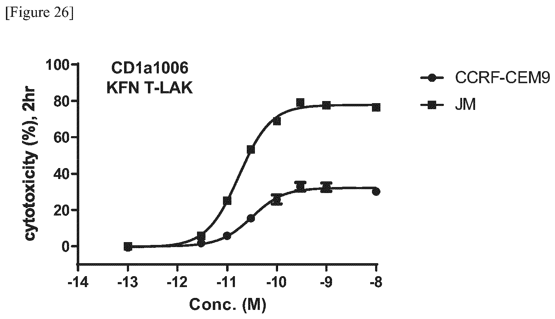

Fig. 26 shows the results obtained by adding the anti-TRBC2 antibody-sensitized T-LAK cells to the CCRF-CEM9 cells, a TALL-derived cell strain expressing TRBC2, and JM cells, a TALL-derived cell strain expressing TRBC1, and measuring cytotoxicity of the anti-CD1a/anti-TRBC2 bispecific antibody CD1a1006. The EC50 and Emax values (N=3) are shown in the following table.

**[Table 21]**

| Target cell name | EC50 (pM) | Standard deviation |
|---|---|---|
| CCRF-CEM9 | 28.4 | 2.1 |
| JM | 18.0 | 1.9 |

| Target cell name | Emax (%) | Standard deviation |
|---|---|---|
| CCRF-CEM9 | 33.3 | 4.1 |
| JM | 72.8 | 3.6 |

The EC50 values were 28.4 pM and 18.0 pM, and the Emax were 33.0 and 72.8% for the target CCRF-CEM9 and JM cells, respectively. These results indicate that the anti-CD1a/anti-TRBC2 bispecific antibody exhibits high cytotoxicity against CD1a and TRBC1-expressing cells and low cytotoxicity against CD1a and TRBC2-expressing cells. In addition, as shown in Example 4, when the effector-side antibody is TRBC1, the antibody shows extremely potent cytotoxicity against the target CCRF-CEM9 cells. These results indicate that it is important for the effector side antibody to be an antibody that recognizes a subtype not expressed on tumor cells.

### <Example 16> In vivo evaluation of anti-tumor activity

In this example, *in vivo* antitumor effect of the anti-CD1a/anti-TRBC2 bispecific antibody, CD1a1006, was verified.

### Preparation of cells

The JM cells are a TALL-derived cell strain expressing CD1a and TRBC1, and were purchased from the RIKEN BioResource Research Center, Cell Engineering Division (Cell Bank, cell number RCB1164, product number RCB0537). JM-Luc cells obtained by making JM cells stably express the luciferase gene were used. The JM-Luc cells cultured in the RPMI 1640 medium containing 10% FBS in a 5% CO₂, 37°C incubator were used for *in vivo* antitumor efficacy studies.

### Test animal

Animal species: Mouse
Type: NOD/Shi-scid, IL-2RγKO JiC (NOG)
Supplier: Clea Japan, Inc.
Age in weeks: 6 Weeks old (at the time of receipt)

### Preparation of PBMC

PBMCs were purchased from Hemacare (product number PB009C-3, Lot No. 22074722). Frozen PBMCs (1 x 10⁸ cells/vial) were thawed in a thermostatic bath at approximately 37°C, and the cell suspension was collected by using a pipette and slowly dropped into a 50-mL centrifuge tube. The empty vial was rinsed with Thawing Media (Hanks' Balanced Salt Solution (-) containing 10% FBS), and the medium was then slowly dropped into the above centrifuge tube and mixed with the cell suspension. After centrifugation (room temperature, 400 x g, 5 minutes) and removal of the supernatant by aspiration, Washing Media (25 mL of D-PBS containing 0.005% FBS + 100 µL of 0.5 M EDTA) was added to re-suspend the cells. The cell suspension was diluted 2-fold with trypan blue, and then the number of live cells was calculated. The cell suspension was centrifuged again (room temperature, 400 to g, 5 minutes), the supernatant was removed by aspiration, and then a cell suspension of 1 x 10⁸ cells/mL was prepared in the RPMI 1640 medium.

### Transplantation of PBMC

The prepared PBMC suspension was brought into the breeding room while being preserved on ice, and within 2 hours after the preparation, the cells were intravenously transplanted into NOG mice, i.e., the tails of the mice were disinfected with alcohol cotton, and the cell suspension was injected into the tail vein in a volume of 0.1 mL (1 to 10⁷ cells/mouse) using a 1-mL disposable syringe and a 27G needle (Terumo Corporation). This transplantation day was defined as "day 0".

### Preparation of JM-Luc cells

JM-Luc cells in culture were collected on day 0. The culture was centrifuged (room temperature, 400 to g, 5 minutes), and the supernatant was removed by aspiration. Then, the cells was washed with PBS and centrifuged again (room temperature, 400 to g, 5 minutes). The supernatant was removed by aspiration, the cells were diluted with PBS as appropriate, stained with trypan blue, and counted, and a suspension of 1 x 10⁷ cells/mL was prepared. The prepared cell suspension was kept on ice until transplantation.

### Transplantation of JM-Luc cells

The prepared JM-Luc cell suspension was brought into the breeding room while being preserved on ice, and within 2 hours after the preparation, the cells were intravenously transplanted into NOG mice, i.e., the tails of the mice were disinfected with alcohol cotton, and the cell suspension was injected into the tail vein in a volume of 0.1 mL (1 to 10⁶ cells/mouse) using a 1-mL disposable syringe and a 27G needle (Terumo Corporation). This transplantation day was defined as "day 0".

### Preparation of antibody

CD1a1006 was prepared in PBS at antibody doses of 0.05 mg/kg (0.01 mg/mL), 0.15 mg/kg (0.03 mg/mL), 0.5 mg/kg (0.1 mg/mL), 1.5 mg/kg (0.3 mg/mL) and 5 mg/kg (1.0 mg/mL).

### Administration of antibody

On days 1, 7, 10, 14, 17, 21, and 25, the tails were disinfected with alcohol cotton and 0.1 mL of each of the prepared antibody solutions was intravenously implanted via the tail vein by using a 1-mL disposable syringe and a 27G needle (Terumo Corporation).

### Method for analyzing anti-tumor effect

On days 14 and 28, luminescence levels of the JM-Luc cells were measured in all surviving individuals by using IVIS Imaging System (Revvity). On each IVIS measurement day, 150 mg/kg (10 mL/kg) of luciferin was intraperitoneally administered to the animals, the animals were placed supine in the IVIS chamber under isoflurane inhalation anesthesia, and luminescence (total flux) was measured for the whole body as the region of interests (ROI) about every 1 minute for 20 minutes starting approximately 5 minutes after the luciferin administration. The maximum value of the luminescence measured about every 1 minute was taken as the measurement value for each individual on each measurement day.

### Results

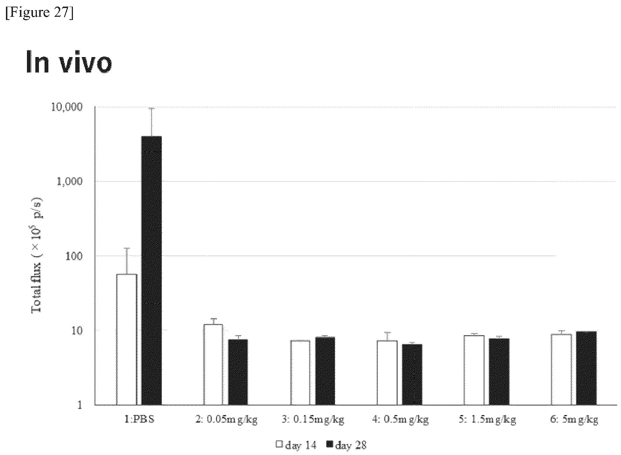

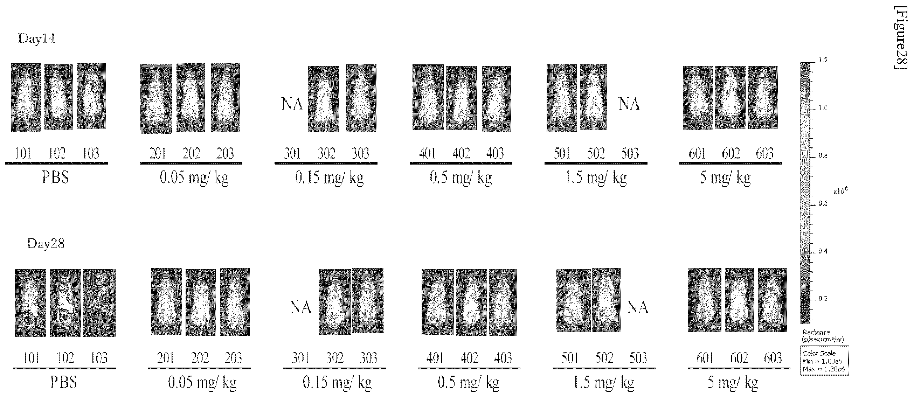

A graph of the IVIS measurement values for each group on days 14 and 28 is shown in Fig. 27, and tumor distribution images are shown in Fig. 28. The IVIS measurement values of the first group on day 14 were 56.34 ± 68.05 x 10⁵ p/s, whereas those of the second to sixth groups administered with CD1a1006 on the same day were 11.74 ± 2.59 x 10⁵ p/s, 7.17 ± 0.14 x 10⁵ p/s, 7.10 ± 2.01 x 10⁵ p/s, 8.54 ± 0.32 ×10⁵ p/s, and 8.64 ± 1.05 × 10⁵ p/s, respectively.

The IVIS measurement values of the first group on day 28 were 3951.23 ± 5229.34 x 10⁵ p/s, whereas the IVIS measurement values of the second to sixth groups administered with CD1a1006 on the same day were 7.43 ± 0.99 x 10⁵ p/s, 7.81 ± 0.66 x 10⁵ p/s, 6.43 ± 0.29 x 10⁵ p/s, 7.66 ± 0.59 x 10⁵ p/s, and 9.40 ± 0.20 x 10⁵ p/s, respectively.

These results revealed that CD1a1006 has an efficacy to inhibit engraftment and subsequent proliferation of the JM-Luc cells.

### <Example 17> Data on cytotoxicity in CD1a KO cells 1

In this example, to evaluate antigen-specific *in vitro* efficacy of the anti-CD1a/anti-TRBC1 bispecific antibodies prepared in Example 1, target CCRF-CEM9 and CCRF-CEM CD1a KO cells were cultured with the T -LAK cells (effector cells) in the presence of the bispecific antibodies, and then cell viability of the target cells was measured by using a fluorescent dye (calcein activity) as an indicator to evaluate effector cell-dependent cytotoxicity.

The target CCRF-CEM9 cells were obtained by single cell cloning of CCRF-CEM, a T-ALL-derived cell strain expressing CD1a and TRBC2. The CCRF-CEM CD1a KO cells are a cell strain obtained from CCRF-CEM by knocking out the CD1a gene using the CRSPR-Cas9 method. The measurement of the cytotoxicity was performed by the same method as described in Example 3.

### Results

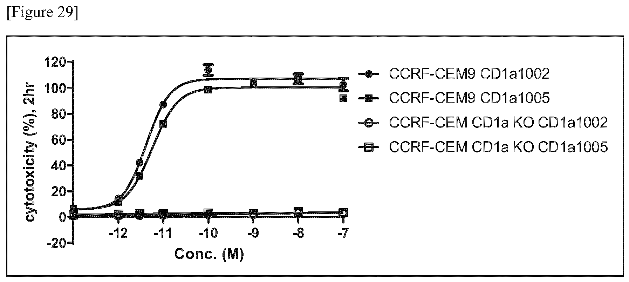

Fig. 29 shows the results obtained by adding the T-LAK cells to the CCRF-CEM9 cells, a T-ALL-derived cell strain expressing CD1a, and CCRF-CEM CD1a KO cells, and measuring the cytotoxicities of the bispecific antibodies CD1a1002 and CD1a1005.

The EC50 values and Emax values (N=3) are shown in the following table.

**[Table 22]**

| Target cell name | Antibody | EC50 (pM) | Standard deviation |
|---|---|---|---|
| CCRF-CEM9 | CD1a1002 | 3.7 | 0.5 |
| CCRF-CEM9 | CD1a1005 | 4.8 | 1.2 |
| CCRF-CEM CD1a KO | CD1a1002 | 208.2 | NC |
| CCRF-CEM CD1a KO | CD1a1005 | 3737.0 | NC |
| CCRF-CEM9 | CD1a1002 | 103.4 | 8.5 |
| CCRF-CEM9 | CD1a1005 | 95.8 | 8.4 |
| CCRF-CEM CD1a KO | CD1a1002 | 2.7 | 1.1 |
| CCRF-CEM CD1a KO | CD1a1005 | 2.9 | NC |

For EC50 of CCRF-CEM CD1a KO/CD1a1002, since the value could not be calculated in one time of the experiment that was repeated three times, the average value for two times of the experiment is shown.
For EC50 of CCRF-CEM CD1a KO/CD1a1005, since the value could not be calculated in two times of the experiment that was repeated three times, the value for one time of the experiment is shown.
For Emax of CCRF-CEM CD1a KO/CD1a1005, since the value could not be calculated in one time of the experiment that was repeated three times, the average value for two times of the experiment is shown.

The T-LAK cells were added to the CCRF-CEM9 cells and CCRF-CEM CD1a KO cells, and the cytotoxicity of CD1a1002 and CD1a1005 was measured. As a result, for the CCRF-CEM9 cells, Emax was 103.4% or 95.8%, and for the CCRF-CEM CD1a KO cells, Emax was 2.7% or 2.9%, respectively, and no antibody concentration-dependent cytotoxicity was observed. These results indicate that the CD1a/anti-TRBC1 bispecific antibodies act on cells expressing the antigen in a cell-specific manner.

### <Example 18> Data on cytotoxicity in CD1a KO cells 2

In this example, to evaluate the antigen-specific *in vitro* efficacy of the anti-CD1a/anti-TRBC2 bispecific antibodies CD1a1003 and CD1a1006 prepared in Example 1, target JM and JM CD1a KO cells were cultured with the anti-TRBC2 antibody-sensitized T-LAK cells (effector cells) in the presence of the bispecific antibodies, and then cell viability of the target cells was measured by using a fluorescent dye (calcein activity) as an indicator to evaluate effector cell-dependent cytotoxicity.

The target JM cells are a TALL-derived cell strain expressing CD1a and TRBC1; The JM CD1a KO cells are a cell strain obtained from the JM cells by knocking out the CD1a gene using the CRSPR-Cas9 method. The measurement of the cytotoxicity was performed by the same method as described in Example 3.

### Results

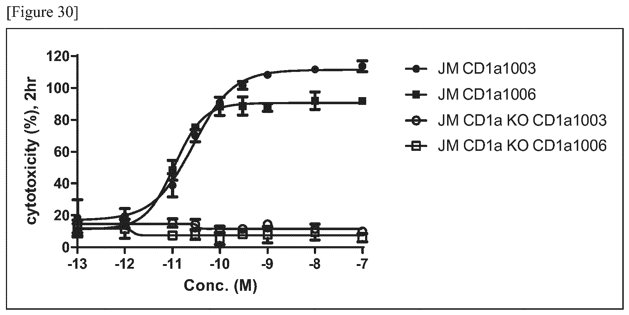

Fig. 30 shows the results obtained by adding the anti-TRBC2 antibody-sensitized T-LAK cells to the JM cells, a TALL-derived cell strain expressing CD1a, and JM CD1a KO cells, and measuring the cytotoxicities of the bispecific antibodies CD1a1003 and CD1a1006.

The EC50 values and Emax values (N=3) are shown in the following table.

**[Table 23]**

| Target cell name | Antibody | EC50 (pM) | Standard deviation |
|---|---|---|---|
| JM (control) | CD1a1003 | 15.7 | 8.2 |
| JM (control) | CD1a1006 | 7.4 | NC |
| JM CD1a KO | CD1a1003 | 92.3 | NC |
| JM CD1a KO | CD1a1006 | NC | NC |

| Target cell name | Antibody | Emax (%) | Standard deviation |
|---|---|---|---|
| JM (control) | CD1a1003 | 91.2 | 25.3 |
| JM (control) | CD1a1006 | 66.1 | NC |
| JM CD1a KO | CD1a1003 | 12.2 | NC |
| JM CD1a KO | CD1a1006 | 6.9 | NC |

For EC50 of JM CD1a KO/CD1a1003, since the value could not be calculated in two times of the experiment that was repeated three times, the value for one time of the experiment is shown.
For EC50 of JM CD1a KO/CD1a1006, the value could not be calculated in two times of the experiment that was repeated two times.
For Emax of JM CD1a KO/CD1a1003, the value could not be calculated in one time of the experiment that was repeated three times, the average value for two times of the experiment is shown.

The anti-TRBC2 antibody-sensitized T-LAK cells were added to the JM cells and JM CD1a KO cells, and the cytotoxicities of CD1a1003 and CD1a1006 were measured. As a result, for the JM cells, Emax was 91.2% or 66.1%, whereas for the JM CD1a KO cells, Emax was 12.2% or 6.9%, and no antibody concentration-dependent cytotoxicity was observed. These results indicate that the anti-CD1a/anti-TRBC2 bispecific antibodies act on cells expressing the antigen in a cell-specific manner.

### <Example 19> Comparison with bispecific antibody employing anti-TRBV5-5 antibody 1

The following reagents were used.
PBS (Nacalai Tesque, 14249-24)
Ficoll-Paque^{™} PLUS (GE Healthcare, 17-1440-02)
Leucosep (Grener Bio-One, 227290)
FBS (NICHIREI, 175012, Lot. 19J00C)
2% FBS-PBS (PBS 49 mL + FBS 1 mL)
Red Blood Cell Lysis Buffer (Roche, 11814389001)
CELLBUNKER2 (Takara, 11914)
Pan T Cell Isolation Kit human (Miltenyi Biotec, 130-096-535)
10% BSA (Thermo, 37525)
0.5 mmol/L EDTA (Nacalai, 06894-14)
Steady-Glo (Promega, E2520)
RPMI 1640 (Nacalai Tesque, 30264-85)
1 M Hepes (Nacalai, 17557-94)
100x Sodium pyruvate (Gibco, 11360-070)
100x 2-ME (Wako, 198-15781)

In this example, cytotoxicities of the anti-CD1a/anti-TRBC1 bispecific antibody of the present invention and an anti-CD1a/anti-TRBV5-5 bispecific antibody produced on the basis of the description of WO2022/119955 were verified. WO2022/119955 discloses bispecific antibodies targeting CD3 and a TRBV polypeptide for use in the treatment of T-cell tumors (claims). The TRBV polypeptide described in WO2022/119955 is an example of antigen having 30 subtypes.

Anti-CD1a/anti-TRBV5-5 bispecific antibodies were produced as described in Example 1.

**[Table 24]**

| Antibody name | Antibody format | Tumor antigen | T cell antigen | Amino acid sequence of heavy chain | | Amino acid sequence of light chain | |
|---|---|---|---|---|---|---|---|
| TRBV50001 | IgG | | TRBV5-5 | SEQ ID NO: 84 | TRBV50001 VH | SEQ ID NO: 85 | TRBV50001 VL |
| TRBV50003 | Fab-scFv | CD1a | TRBV5-5 | SEQ ID NO: 143 | TRBV50003 VH | SEQ ID NO: 29 | CD1a1003 VL |
| | | | | | | | (CD1a1001 VL) |
| TRBV50004 | Fab-scFv-Fc | CD1a | TRBV5-5 | SEQ ID NO: 86 | TRBV50004 VH | SEQ ID NO: 29 | CD1a1003 VL |
| | | | | | | | (CD1a1001 VL) |

The anti-CD1a/anti-TRBC1 bispecific antibody CD1a1005 prepared in Example 14 was used. The target CCRF-CEM-Luc cells were cultured with healthy human-derived T cells (effector cells) in the presence of the bispecific antibodies, and then cell viability of the target cells was measured by using a luminescent enzyme activity (luciferase activity) as an indicator to evaluate effector cell-dependent cytotoxicity.

The target CCRF-CEM-Luc cells are a cell strain obtained by introducing the luciferase gene into the TALL-derived cell strain, CCRF-CEM cells, using a lentivirus vector.

### Methods

The bispecific antibodies were adjusted to a final concentration of 10 nM to 100 fM by using the RPMI 1640 medium containing 10% FBS, 10 mM HEPES, 1 mM sodium pyruvate and 0.1 mM 2-ME (TDCC medium), and added to a flat bottom 384-well culture plate. As a control, a well containing only the TDCC medium was prepared.

For cytotoxicity assay using bispecific antibodies having antigen-binding sites for CD1a and TRBC1 or TRBV5-5, CCRF-CEM-Luc cells (target cells) were collected from the culture medium in a necessary amount, and suspended in the TDCC medium, and the cell number was counted. The cells were then added to wells of the flat bottom 384-well culture plate in which the bispecific antibodies were prepared. A required amount of the effector cells were collected, and suspended in the TDCC medium, and the cell number was counted. The cells were then added to the wells of the flat bottom 384-well culture plate in which the bispecific antibodies were prepared. The effector cells and the labeled target cells were added at a ratio of 10:1. The plate containing the bispecific antibodies, labeled target cells, and effector cells was incubated at 5% CO₂ and 37°C. After 72 hours, 25 µL of Steady-Glo was added to each well, and after 5 minutes, luminescence produced by luciferase in live cells was measured with a plate reader (Bio Tek, Cytation5). Injured target cell ratio was calculated by using the value of luminescence produced by luciferase as an index to evaluate bispecific antibody-dependent cytotoxicity.

The cytotoxicity percentage was determined as follows.

The maximum luminescence value of the labeled target cells (injury ratio = 0%) was obtained from a well in which the labeled target cells and effector cells were incubated without any bispecific antibody.

The cytotoxicity percentage was calculates in accordance with the following equation. Cytotoxicity percentage = 100 - [100 x (Luminescence value of target cells in the presence of bispecific antibody)/(Maximum luminescence value)]

S-shaped dose-response curves were obtained by calculation using Prism Software (GraphPad Software Inc.), and EC50 value (50% effective concentration) and Emax value (maximum activity value) were calculated.

### Preparation of healthy human-derived T cells

T cells were prepared from blood collected from healthy subjects as follows. Blood heparinized at the time of blood collection was diluted 3-fold with PBS, added to a Leucosep tube filled with Ficoll and diluted with PBS to 50 mL. The tube was then centrifuged (1000 x g, 10 minutes, 20°C), the upper layer was removed, and the PBMC layer was transferred to a separate tube. The cells were washed twice with PBS, and suspended in 3 mL of 2% FBS-PBS, 6 mL of RBC Buffer was added to the suspension, and the mixture was allowed to stand for 10 minutes. The cells were washed with 10 mL of 2% FBS/PBS, suspended in CELLBUNKER2, and stored in liquid nitrogen until use. The stored human PBMCs were thawed in a 37°C water bath and suspended in MACS buffer (PBS containing 0.5% BSA and 2 mM EDTA) at a density of 1 x 10⁷ cells/40 µL. T cells were fractionated from PBMCs by using Pan T cell Isolation Kit human, and used in the assay.

### Results

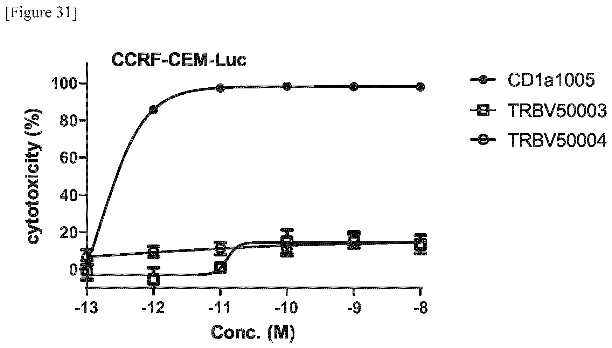

Fig. 31 shows the results obtained by adding healthy human-derived T cells to the CCRF-CEM-Luc cells, obtained by making CCRF-CEM cells, a TALL-derived cell strain, express luciferase, and measuring the cytotoxicities of the anti-CD1a/anti-TRBC1 bispecific antibody (CD1a1 005) and anti-CD1a/anti-TRBV5-5 bispecific antibodies (TRBV50003 and TRBV50004).

The EC50 values and Emax values (N=3) are shown in the following table.

**[Table 25]**

| Target cell name | Antibody | EC50 (pM) | Standard deviation |
|---|---|---|---|
| CCRF-CEM-Luc | CD1a1005 | 1.2 | 0.8 |
| CCRF-CEM-Luc | TRBV50003 | 14.0 | NC |
| CCRF-CEM-Luc | TRBV50004 | 0.5 | NC |

For EC50 of CCRF-CEM-Luc/TRBV50003, since the value could not be calculated in one time of the experiment that was repeated three times, the average value for two times of the experiment is shown.
For EC50 of CCRF-CEM-Luc/TRBV50004, since the value could not be calculated in two times of the experiment that was repeated three times, the value for one time of the experiment is shown.

| Target cell name | Antibody | Emax (%) | Standard deviation |
|---|---|---|---|
| CCRF-CEM-Luc | CD1a1005 | 79.6 | 13.3 |
| CCRF-CEM-Luc | TRBV50003 | 23.6 | 7.8 |
| CCRF-CEM-Luc | TRBV50004 | 15.0 | 6.1 |

The Emax of the bispecific antibody of anti-CD1a and anti-TRBC1 antibodies was 79.6%, showing good activity. On the other hand, the Emax of the anti-CD1a/anti-TRBV5-5 bispecific antibodies was 23.6% or 15.4%, showing significantly weaker activity compared with the bispecific antibody employing the anti-TRBC1 antibody.

TRBV is known to have 30 subtypes, and a single T cell is known to randomly express only one of these isotypes. Therefore, it is estimated that only about 3% of all T cells expresses TRBV5-5. On the other hand, there are two isotypes of TRBC, and one T cell expresses only one isotype, either TRBC1 or TRBC2. Therefore, it is estimated that TRBC1-expressing T cells account for about 50% of all T cells, and it is thought that the difference in the number of T cells (effector cells) that can bind to the bispecific antibody be responsible for the difference in cytotoxicity in the data mentioned above. From these results, it was considered that the bispecific antibody employing anti-TRBC1 antibody, which can make a larger number of T cells function *in vivo,* is expected to provide higher efficacy.

As shown in Reference Example 3, when there were enough T cells expressing TRBV5-5, both TRBV50003 and TRBV50004 showed activity represented by Emax of about 60%. These results support that the difference in the number of T cells that can bind to bispecific antibodies has a significant impact on cytotoxicity as described above.

### <Example 20> Comparison with bispecific antibody employing anti-TRBV5-5 antibody 2

The same experiment as in Example 19 was performed on target JM-Luc-BFP cells. The target JM-Luc-BFP cells are a cell strain obtained by introducing the luciferase gene into the TALL-derived cell strain, JM cells, using a lentivirus vector.

In this example, the anti-CD1a/anti-TRBC2 bispecific antibody CD1a1006 prepared in Example 1, anti-CD1a/anti-TRBV5-5 bispecific antibodies TRBV50003 and TRBV50004 prepared in Example 19 were used. The target JM-Luc-BFP cells were cultured with healthy human-derived T cells (effector cells) in the presence of the bispecific antibodies, and cell viability of the target cells was measured by using luminescent enzyme activity (luciferase activity) as an indicator to evaluate effector cell-dependent cytotoxicity.

### Results

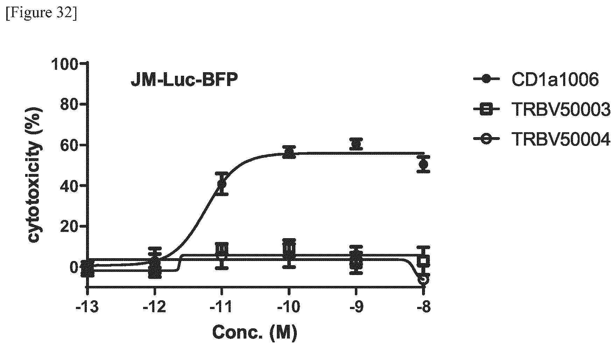

Fig. 32 shows the results obtained by adding healthy human-derived T cells to the JM-Luc-BFP cells, which are JM cells, a TALL-derived cell strain, made to express luciferase, and measuring the cytotoxicities of the anti-CD1a/anti-TRBC2 bispecific antibody (CD1a1006) and anti-CD1a/anti-TRBV5 -5 bispecific antibodies (TRBV50003 and TRBV50004).

The EC50 values and Emax values (N=2) are shown in the following table.

**[Table 26]**

| Target cell name | Antibody | EC50 (pM) | Standard deviation |
|---|---|---|---|
| JM-Luc-BFP | CD1a1006 | 6.1 | NC |
| JM-Luc-BFP | TRBV50003 | NC | NC |
| JM-Luc-BFP | TRBV50004 | 660.7 | NC |

| Target cell name | Antibody | Emax (%) | Standard deviation |
|---|---|---|---|
| JM-Luc-BFP | CD1a1006 | 51.1 | NC |
| JM-Luc-BFP | TRBV50003 | 3.9 | NC |
| JM-Luc-BFP | TRBV50004 | 3.8 | NC |

For EC50 of JM-Luc-BFP/TRBV50003, since the value could not be calculated in two times of the experiment that was repeated two times, the result is shown as NC. The result for EC50 of JM-Luc-BFP/TRBV50004 is the result of the experiment performed once.

The Emax of the bispecific antibody of the anti-CD1a antibody and anti-TRBC2 antibody was 51.1%, showing good activity. On the other hand, the Emax of the anti-CD1a/anti-TRBV5-5 bispecific antibody was 3.9% or 3.8%, which are results indicating significantly weaker activity than the bispecific antibody employing the anti-TRBC2 antibody. On the basis of these results, it was considered that bispecific antibodies employing anti-TRBC2 antibody, which can activate a larger number of T cells *in vivo,* are expected to provide higher efficacy.

### <Reference Example 3> Activity of bispecific antibody employing anti-TRBV5-5 antibody

This reference example was performed to confirm that the bispecific antibodies TRBV50003 and TRBV5004 prepared in Examples 19 and 20 are antibodies having cytotoxicity.

The target CCRF-CEM9 cells were cultured with the T-LAK cells (effector cells) stimulated with the anti-TRBV5-5 antibody (TRBV50001) in the presence of the bispecific antibodies, and then the cell viability of the target cells was measured by using a fluorescent dye (calcein activity) as an indicator to evaluate effector cell-dependent cytotoxicity.

### Preparation of T-LAK cells stimulated with anti-TRBV5-5 antibody

Human PBMCs were thawed in a 37°C water bath and washed once with the culture medium. After 2 days of the culture in the RPMI 1640 medium containing 10% FBS supplemented with 10 nM anti-TRBV5-5 antibody (TRBV50001) and 100 IU/mL IL-2, the cells were further cultured in the RPMI 1640 medium containing IL-2 and 10% FBS (up to 17 days) and thereby derived into T-LAK. The cells were subcultured every 2 to 3 days, and 100 IU/mL IL-2 was newly added at the time of culture medium change.

### Results

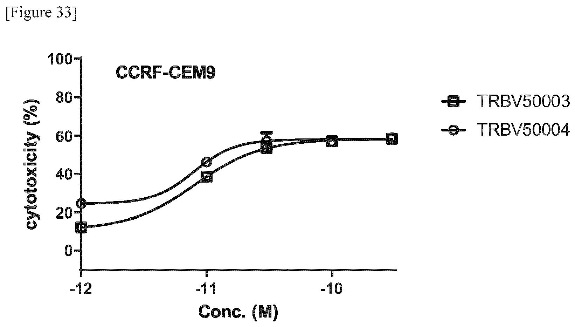

Fig. 33 shows the results obtained by adding the T-LAK cells stimulated with the anti-TRBV5-5 antibody (TRBV50001) to the CCRF-CEM9 cells, a TALL-derived cell strain, and measuring cytotoxicities of the anti-CD1a/anti-TRBV5-5 bispecific antibodies (TRBV50003 and TRBV50004).

The EC50 values and Emax values are shown in the following table.

**[Table 27]**

| Target cell name | Antibody | EC50 (pM) |
|---|---|---|
| CCRF-CEM9 | TRBV50003 | 8.2 |
| CCRF-CEM9 | TRBV50004 | 8.0 |

| Target cell name | Antibody | Emax (%) |
|---|---|---|
| CCRF-CEM9 | TRBV50003 | 57.5 |
| CCRF-CEM9 | TRBV50004 | 57.4 |

The Emax vales of the anti-CD1a/anti-TRBV5-5 bispecific antibodies were 57.5% and 57.4%, respectively, indicating antibody concentration-dependent cytotoxicity. It could be confirmed that even bispecific antibodies employing anti-TRBV5-5 antibody on the effector side show cytotoxicity under the condition that activated T cells stimulated with anti-TRBV5-5 antibody and expressing TRBV5-5 are abundantly present.

## Claims

1. A therapeutic agent for a T cell malignancy comprising a bispecific antigen-binding molecule, wherein the bispecific antigen-binding molecule comprises
(1) at least one portion that specifically binds to a target tumor antigen expressed on T cell tumor cells, and
(2) at least one portion that specifically binds to a normal T cell-side target antigen having subtypes,
provided that
the target tumor antigen expressed on the T cell tumor cells is not present on normal T cells, or even if it is present, the normal T cells are not substantially activated when the bispecific antigen-binding molecule binds to the same antigen as the target tumor antigen present on the normal T cells,
binding of the bispecific antigen-binding molecule to the normal T cell-side target antigen activates the normal T cells, and
a sufficient proportion of a subtype of the normal T cell-side target antigen is present to provide a sufficient number of activated T cells for the treatment of the T cell tumor.

2. The therapeutic agent according to claim 1, wherein the subtype of the normal T cell-side target antigen is a subtype selected from the subtypes of the antigen that are not a subtype of the antigen expressed on the T cell tumor cells.

3. The therapeutic agent according to claim 1 or 2, wherein the normal T cell-side target antigen having subtypes is a TRBC (T cell receptor beta-chain constant region),
the subtype expressed on the T cell tumor cells is TRBC1, and
the subtype of the normal T cell-side target antigen is TRBC2.

4. The therapeutic agent according to claim 1 or 2, wherein the normal T cell-side target antigen having subtypes is a TRBC,
the subtype expressed on the T cell tumor cells is TRBC2, and
the subtype of the normal T cell-side target antigen is TRBC1.

5. The therapeutic agent according to claim 1 or 2, wherein the normal T cell-side target antigen having subtypes is a TRBC,
the T cell tumor cells are TRBC1-negative and TRBC2-negative, and
the subtype of the normal T cell-side target antigen is TRBC1 or TRBC2.

6. The therapeutic agent according to claim 1 or 2, wherein the subtype of the normal T cell-side target antigen is TRBC1,
the bispecific antigen-binding molecule comprises at least one portion that specifically binds to TRBC1 of the normal T cells, and the at least one portion comprises
a VH domain comprising
a heavy chain CDR1 comprising the amino acid sequence of SEQ ID NO: 10;
a heavy chain CDR2 comprising the amino acid sequence of SEQ ID NO: 11; and
a heavy chain CDR3 comprising the amino acid sequence of SEQ ID NO: 12; and
a VL domain comprising
a light chain CDR1 comprising the amino acid sequence of SEQ ID NO: 13;
a light chain CDR2 comprising the amino acid sequence of SEQ ID NO: 14; and
a light chain CDR3 comprising the amino acid sequence of SEQ ID NO: 15.

7. The therapeutic agent according to claim 6, wherein the at least one portion that specifically binds to TRBC1 of the normal T cells comprises
a heavy chain variable region VH that is at least 90% identical to the amino acid sequence of SEQ ID NO: 4, and
a light chain variable region VL that is at least 90% identical to the amino acid sequence of SEQ ID NO: 5.

8. The therapeutic agent according to claim 1 or 2, wherein the subtype of the normal T cell-side target antigen is TRBC2,
the bispecific antigen-binding molecule comprises at least one portion that specifically binds to TRBC2 of the normal T cells, and the at least one portion comprises
a VH domain comprising
a heavy chain CDR1 comprising the amino acid sequence of SEQ ID NO: 16;
a heavy chain CDR2 comprising the amino acid sequence of SEQ ID NO: 17; and
a heavy chain CDR3 comprising the amino acid sequence of SEQ ID NO: 18; and
a VL domain comprising
a light chain CDR1 comprising the amino acid sequence of SEQ ID NO: 19;
a light chain CDR2 comprising the amino acid sequence of SEQ ID NO: 20; and
a light chain CDR3 comprising the amino acid sequence of SEQ ID NO: 21.

9. The therapeutic agent according to claim 8, wherein the at least one portion that specifically binds to TRBC2 of the normal T cells comprises
a heavy chain variable region VH that is at least 90% identical to the amino acid sequence of SEQ ID NO: 6, and
a light chain variable region VL that is at least 90% identical to the amino acid sequence of SEQ ID NO: 7.

10. The therapeutic agent according to claim 1 or 2, wherein the T cell tumor is T cell acute lymphoblastic leukemia/lymphoblastic lymphoma or mature T cell tumor.

11. The therapeutic agent according to claim 1 or 2, which is for use in a method for treating a T cell malignancy of a subject, and wherein the treatment method comprises determining subtype expressed on T cell tumor cells of the subject and administering the therapeutic agent to the subject, and the therapeutic agent comprises a bispecific antigen-binding molecule comprising at least one portion that specifically binds to a normal T cell-side target antigen of a subtype different from the subtype determined to be expressed on the T cell tumor cells of the subject.

12. A bispecific antigen-binding molecule comprising
(1) at least one portion that specifically binds to a target tumor antigen expressed on T cell tumor cells, and
(2) at least one portion that specifically binds to a normal T cell-side target antigen having subtypes, wherein
the at least one portion that specifically binds to a normal T cell-side target antigen having subtypes is at least one portion that specifically binds to TRBC1 or TRBC2 (T cell receptor beta constant region 1 or 2) of normal T cells.

13. The bispecific antigen-binding molecule according to claim 12, wherein the at least one portion that specifically binds to TRBC1 of normal T cells comprises
a VH domain comprising
a heavy chain CDR1 comprising the amino acid sequence of SEQ ID NO: 10,
a heavy chain CDR2 comprising the amino acid sequence of SEQ ID NO: 11, and
a heavy chain CDR3 comprising the amino acid sequence of SEQ ID NO: 12, and
a VL domain comprising
a light chain CDR1 comprising the amino acid sequence of SEQ ID NO: 13,
a light chain CDR2 comprising the amino acid sequence of SEQ ID NO: 14, and
a light chain CDR3 comprising the amino acid sequence of SEQ ID NO: 15.

14. The bispecific antigen-binding molecule according to claim 14, wherein the at least one portion that specifically binds to TRBC1 of normal T cells comprises
a heavy chain variable region VH that is at least 90% identical to the amino acid sequence of SEQ ID NO: 4, and
a light chain variable region VL that is at least 90% identical to the amino acid sequence of SEQ ID NO: 5.

15. The bispecific antigen-binding molecule according to claim 12, wherein the at least one portion that specifically binds to TRBC2 of normal T cells comprises
a VH domain comprising
a heavy chain CDR1 comprising the amino acid sequence of SEQ ID NO: 16,
a heavy chain CDR2 comprising the amino acid sequence of SEQ ID NO: 17, and
a heavy chain CDR3 comprising the amino acid sequence of SEQ ID NO: 18, and
a VL domain comprising
a light chain CDR1 comprising the amino acid sequence of SEQ ID NO: 19,
a light chain CDR2 comprising the amino acid sequence of SEQ ID NO: 20, and
a light chain CDR3 comprising the amino acid sequence of SEQ ID NO: 21.

16. The bispecific antigen-binding molecule according to claim 15, wherein the at least one portion that specifically binds to TRBC2 of normal T cells comprises
a heavy chain variable region VH that is at least 90% identical to the amino acid sequence of SEQ ID NO: 6, and
a light chain variable region VL that is at least 90% identical to the amino acid sequence of SEQ ID NO: 7.
